# EUROPEAN PATENT APPLICATION

(11) **EP 4 524 254 A1**
(43) Date of publication of application: **19.03.2025**
(21) Application number: 23803610.7
(22) Date of filing: 11.05.2023
(51) Int. Cl.: C12Q 1/34, C07D 493/10, C07F 7/10, C09B 11/28, C09B 57/00, C09K 11/06, C12Q 1/37, G01N 21/64, G01N 21/78, G01N 37/00

(54) **FLUORESCENT PROBE FOR DETECTING PEPTIDE-LINKED HYDROLASE**

(30) Priority: 12.05.2022 JP 2022079082
(71) Applicant: The University of Tokyo, Bunkyo-ku, Tokyo 113-8654 (JP); RIKEN, Wako-shi, Saitama 351-0198 (JP)
(72) Inventor: URANO Yasuteru, Tokyo 113-8654 (JP); KOMATSU Toru, Tokyo 113-8654 (JP); KAGAMI Yu, Tokyo 113-8654 (JP); SAKAMOTO Shingo, Tokyo 113-8654 (JP); KASAI Takafumi, Tokyo 113-8654 (JP); WATANABE Rikiya, Wako-shi, Saitama 351-0198 (JP)
(74) Representative: ABG Intellectual Property Law, S.L.
(86) International application number: PCT/JP2023/017768
(87) International publication number: WO 2023/219136

(57) **Abstract**

An object of the present invention is to provide a fluorescent probe that can be used in an enzyme activity detection method. According to the present invention, there is provided a fluorescent probe for detecting a hydrolase, the fluorescent probe containing a compound having at least one water-soluble substituent selected from the group consisting of -CO₂H, -PO₃H₂, and -SO₃H and represented by General Formula (II), or a salt thereof. The fluorescent probe of the present invention can be used in an enzyme activity detection method using a microdevice.

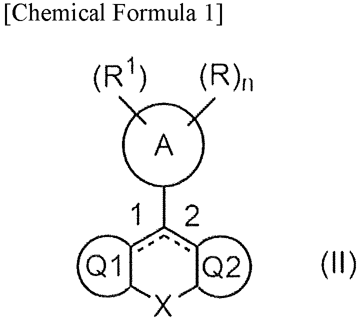

## Description

### CROSS-REFERENCE TO RELATED APPLICATION

The present application enjoys the benefit of priority from the prior Japanese Patent Application No. 2022-79082 (filing date: May 12, 2022), the entire disclosures of which are incorporated herein by reference.

### TECHNICAL FIELD

The present invention relates to a fluorescent probe for detecting a peptide bond hydrolase.

### BACKGROUND ART

An enzyme activity detection method at a single enzyme level using a microdevice has been widely used for the purpose of clarifying individual biochemical parameters of an enzyme, detecting the presence of a specific protein with high sensitivity using the individual biochemical parameters as a reporter protein, and the like.

The research group of the present inventors has hitherto performed profiling based on detection and activity of an enzyme in blood at a single molecule level using a group of microdevice-compatible enzyme fluorescent probes for activity detection, and found new relevance to a disease.

On the other hand, in the enzyme activity detection method at a single enzyme level using a microdevice, by sufficiently diluting a biological sample and adding the biological sample to the microdevice, a state in which one molecule or less of enzyme is present in one well can be created, and so-called multicolor analysis in which fluorescent probes of a plurality of colors having different reactivity between enzymes are simultaneously reacted, fluorescence increases at a plurality of wavelengths in each well are measured, and the enzyme in the well is specified from a combination thereof is theoretically possible.

However, as a fluorescent probe that can be used in the enzyme activity detection method using a microdevice, a probe emitting red fluorescence (red fluorescent probe) or a probe emitting green fluorescence (green fluorescent probe) that can be practically used has not been found yet.

### SUMMARY OF THE INVENTION

### Technical Problem

An object of the present invention is to provide a fluorescent probe that can be used in an enzyme activity detection method.

### Solution to Problem

The present inventors have conducted intensive studies to solve the above problems, and as a result, have found that a fluorescent probe that can also be used in an enzyme activity detection method using a microdevice can be provided by introducing a specific water-soluble substituent into a compound having a rhodamine skeleton and further setting a LogP value of the compound to a specific range, thereby completing the present invention.

According to the present invention, the following inventions are provided.
[1] A fluorescent probe for detecting a hydrolase, the fluorescent probe comprising a compound having at least one water-soluble substituent selected from the group consisting of -CO₂H, -PO₃H₂, and -SO₃H and represented by General Formula (II) below, or a salt thereof: 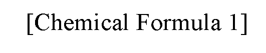 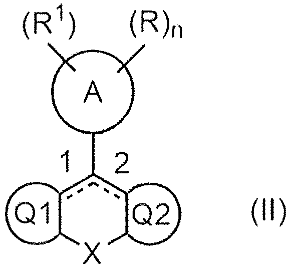
   wherein ring A is an aromatic ring which is phenyl or 5- or 6-membered heteroaryl;
   ring Q1 is a ring structure of Formula (i) or (ii) below, 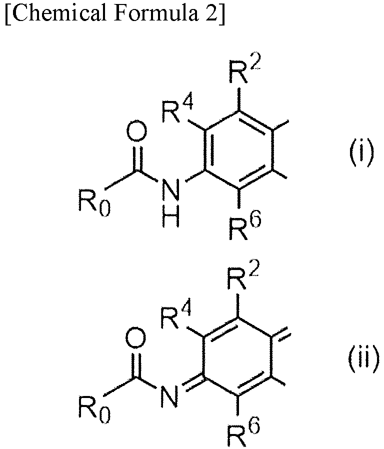
   wherein R², R⁴, R⁶ and R₀ are as defined below;
   ring Q2 is a ring structure of Formula (iii), (iv), (v), or (vi) below, 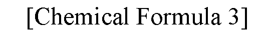 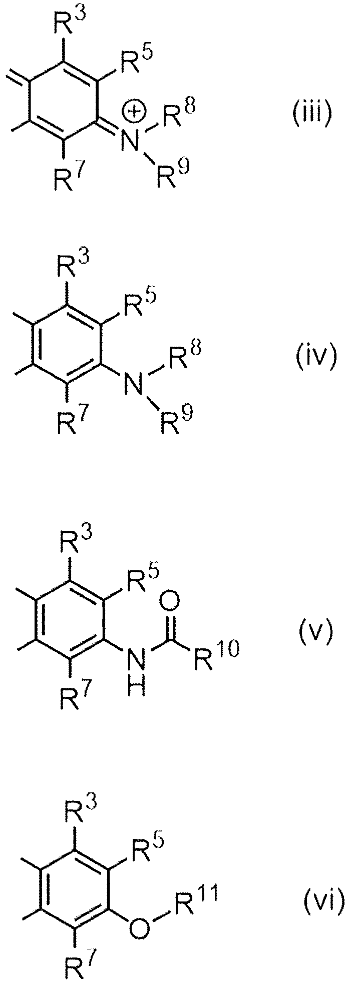
   wherein R³, R⁵, R⁷, R⁸, R⁹, R¹⁰, and R¹¹ are as defined below;
   a bond - - - represented as 1 and a bond - - - represented as 2 in an X atom-containing ring structure each represent a single bond or a double bond, provided that both of the bonds do not represent a double bond at the same time;
   R is selected from the group consisting of -CO₂H, -PO₃H₂, -SO₃H, -L-CO₂H, -L-PO₃H₂, and -L-SO₃H, wherein L is a linker;
   n is an integer of 0 to 5;
   R¹ represents a monovalent substituent optionally present on the aromatic ring, wherein the substituents may be the same or different when two or more monovalent substituents are present,
   wherein R¹, together with a carbon atom on the ring A to which R¹ is bonded, a carbon atom on the X atom-containing ring structure to which the ring A is bonded, and a part of the ring A, may form a 5- or 6-membered carbocyclic or heterocyclic ring;
   R² and R³ are each independently a hydrogen atom, a substituted or unsubstituted alkyl group having 1 to 6 carbon atoms, a halogen atom, the water-soluble substituent, or an alkyl group having the water-soluble substituent and having 1 to 6 carbon atoms;
   R⁴ and R⁵ are each independently a hydrogen atom, a substituted or unsubstituted alkyl group having 1 to 6 carbon atoms, a halogen atom, the water-soluble substituent, or an alkyl group having the water-soluble substituent and having 1 to 6 carbon atoms;
   R⁶ and R⁷ are each independently a hydrogen atom, a substituted or unsubstituted alkyl group having 1 to 6 carbon atoms, a halogen atom, the water-soluble substituent, or an alkyl group having water-soluble substituent and having 1 to 6 carbon atoms;
   R⁸ and R⁹ are each independently a hydrogen atom, a substituted or unsubstituted alkyl group having 1 to 6 carbon atoms, a substituted or unsubstituted alkenyl group having 1 to 6 carbon atoms, an alkyl group having the water-soluble substituent and having 1 to 10 carbon atoms, preferably 1 to 6 carbon atoms (this alkyl group may be substituted with an aryl group, and the aryl group may be substituted with the water-soluble substituent), or an alkenyl group having the water-soluble substituent and having 1 to 10 carbon atoms, preferably 1 to 6 carbon atoms (this alkenyl group may be substituted with an aryl group, and the aryl group may be substituted with the water-soluble substituent),
   wherein R⁸ and R⁹ may together form a 4- to 7-membered heterocyclyl that may contain a nitrogen atom to which R⁸ and R⁹ are attached and may have a substituent,
   R⁸ or R⁹, or both of R⁸ and R⁹, together with R⁵ or R⁷, each may form a 5- to 7-membered heterocyclyl or heteroaryl containing a nitrogen atom to which R⁸ or R⁹ is attached, and further, the heterocyclyl or heteroaryl may have a substituent selected from the group consisting of alkyl having 1 to 6 carbon atoms, alkenyl having 2 to 6 carbon atoms, alkynyl having 2 to 6 carbon atoms, an aralkyl group having 6 to 10 carbon atoms, and an alkyl-substituted alkenyl group having 6 to 10 carbon atoms,
   the water-soluble substituent may be bonded to these heterocyclyl and heteroaryl directly or through the substituent;
   R¹¹ is a hydrogen atom, a substituted or unsubstituted alkyl group having 1 to 6 carbon atoms, a substituted or unsubstituted alkenyl group having 1 to 6 carbon atoms, an alkyl group having the water-soluble substituent and having 1 to 10 carbon atoms, preferably 1 to 6 carbon atoms (this alkyl group may be substituted with an aryl group, and the aryl group may be substituted with the water-soluble substituent), or an alkenyl group having the water-soluble substituent and having 1 to 10 carbon atoms, preferably 1 to 6 carbon atoms (this alkenyl group may be substituted with an aryl group, and the aryl group may be substituted with the water-soluble substituent);
   X is selected from an oxygen atom, Si(R^{a})(R^{b}), C(R^{a})(R^{b}), P(=O)R^{c}, S(R^{d})₂, Ge(R^{a})(R^{b}), a tellurium atom, or a bismuth atom,
   wherein R^{a} and R^{b} are each independently a substituted or unsubstituted alkyl group having 1 to 6 carbon atoms, or a substituted or unsubstituted aryl group,
   R^{c} is a substituted or unsubstituted alkyl group having 1 to 6 carbon atoms, or a substituted or unsubstituted phenyl group,
   R^{d} is a substituted or unsubstituted aryl group, which is the same or different,
   the alkyl group, the aryl group, and the phenyl group of R^{a}, R^{b}, R^{c} and R^{d} may be substituted with the water-soluble substituent; and
   R₀ and R¹⁰ are each independently selected from a partial structure of an amino acid, a partial structure of a carboxylic acid, T-O-, or a saturated or unsaturated 5- or 6-membered carbocyclic or heterocyclic group,
   wherein the partial structure of an amino acid, together with C=O to which R₀ or R¹⁰ is bonded, constitutes an amino acid, an amino acid residue, a peptide, or a part of an amino acid,
   the partial structure of a carboxylic acid, together with C=O to which R₀ or R¹⁰ is bonded, constitutes a part of a carboxylic acid,
   T is a saccharide, a partial structure of a saccharide, or a saccharide having a self-cleaving linker, and
   the partial structure of a saccharide of T, together with O to which T is bonded, constitutes a saccharide or a part of a saccharide.
[2] The fluorescent probe according to [1], wherein Q2 represents a ring structure of Formula (iii).
[3] The fluorescent probe according to [1], wherein Q2 represents a ring structure of Formula (iv), (v), or (vi).
[4] The fluorescent probe according to any one of [1] to [3], wherein n is 1 to 3.
[5] The fluorescent probe according to any one of [1] to [4], wherein at least one of R² to R⁹ and R^{a}, R^{b}, R^{c} and R^{d} is the water-soluble substituent, an alkyl group having the water-soluble substituent and having 1 to 6 carbon atoms, an aryl group having the water-soluble substituent, and a phenyl group having the water-soluble substituent.
[6] The fluorescent probe according to any one of [1] to [5], wherein X is an oxygen atom or Si(R^{a})(R^{b}).
[7] The fluorescent probe according to any one of [1] to [6], wherein R is -SO₃H or -L-SO₃H.
[8] The fluorescent probe according to any one of [1] to [7], wherein the hydrolase is a peptide bond hydrolase selected from an aminopeptidase, a peptidase, a protease, or an amidase.
[9] The fluorescent probe according to any one of [1] to [8], wherein a MiLogP value of the compound or a salt thereof is 2.56 or less.
[10] The fluorescent probe according to any one of [1] to [8], wherein a MiLogP value of a fluorescent mother nucleus of the compound or a salt thereof is less than 2.47.
[11] The fluorescent probe according to any one of [1] to [8], wherein a MiLogP value of the compound or a salt thereof is 2.56 or less, and a MiLogP value of a fluorescent mother nucleus of the compound or a salt thereof is less than 2.47.
[12] The fluorescent probe according to any one of [1] to [11], which is a fluorescent probe for a microdevice.
[13] The fluorescent probe according to any one of [1] to [12], wherein the compound represented by General Formula (II) or a salt thereof is a compound represented by any one of General Formulas (Ia) to (Ie) below or a salt thereof: 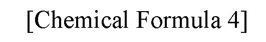 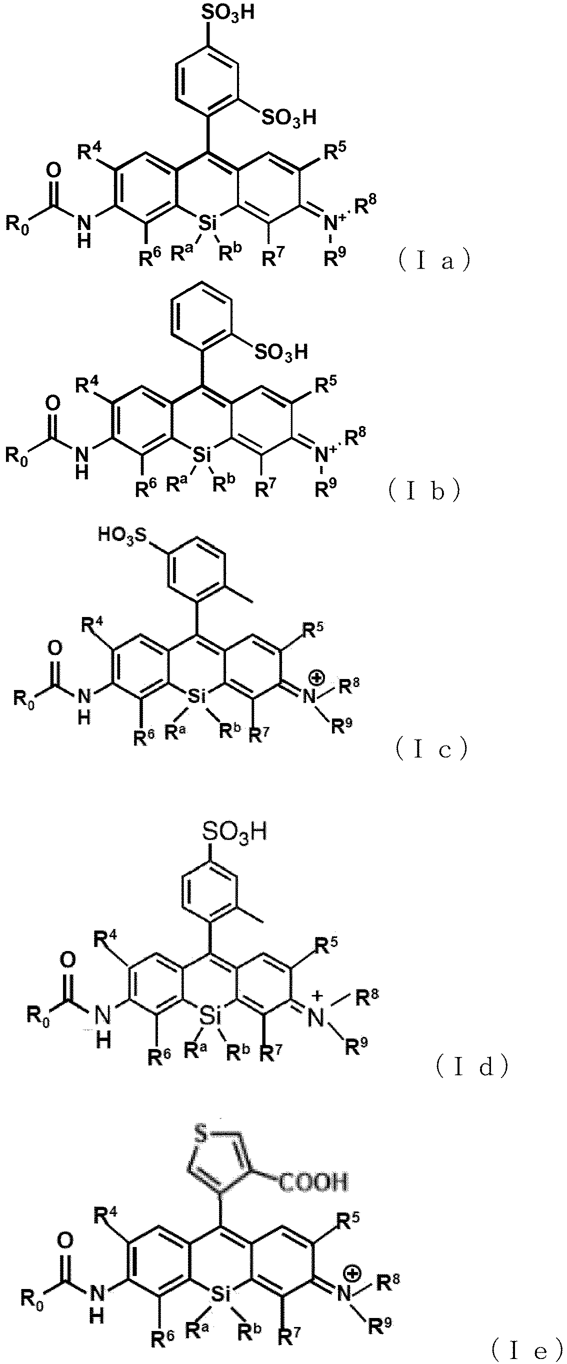 wherein R⁴ to R⁹, R^{a}, R^{b}, and R₀ are as defined in General Formula (II).
[14] The fluorescent probe according to any one of [1] to [13], wherein the compound represented by General Formula (II) or a salt thereof is a compound represented by General Formula (If) below or a salt thereof: 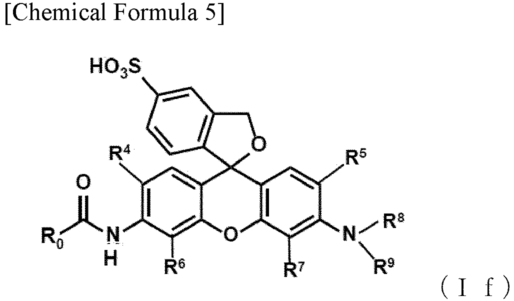 wherein R⁴ to R⁹ and R₀ are as defined in General Formula (II).
[15] The fluorescent probe according to any one of [1] to [14], wherein the compound or a salt thereof is a compound selected from the following compounds, or a salt thereof: 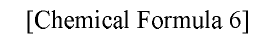 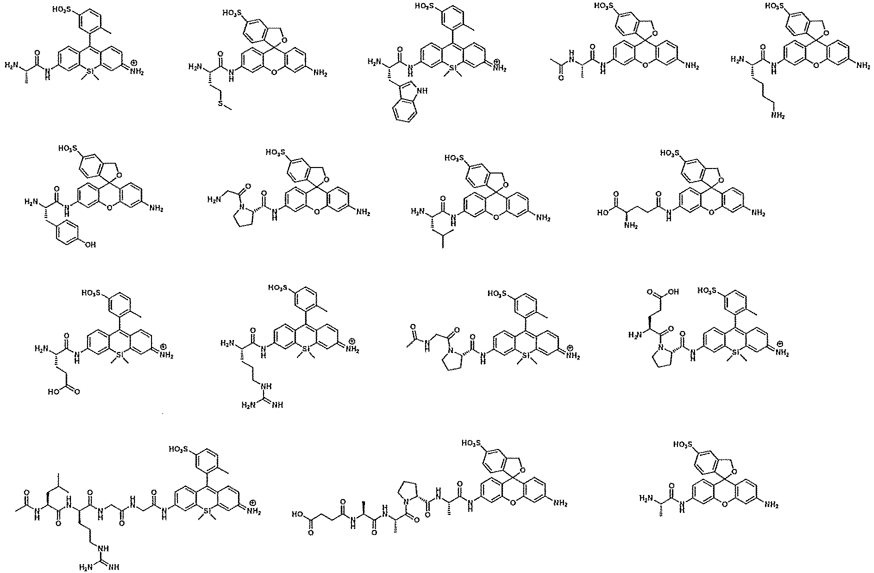
[16] A method for diagnosing pancreatic cancer or a method for predicting a possibility that a test subject from which a biological sample is derived has pancreatic cancer, the method comprising detecting, by a microdevice, single-molecule enzyme activity of CD13 in a biological sample obtained from a subject, using the fluorescent probe according to any one of [1] to [15], in which -CO-R₀ and optionally -CO-R¹⁰ is an alanine, lysine, arginine, or methionine residue in General Formula (II).
[17] A method for diagnosing pancreatic cancer or a method for predicting a possibility that a test subject from which a biological sample is derived has pancreatic cancer, the method comprising detecting, by a microdevice, single-molecule enzyme activity of DPP4 in a biological sample obtained from a subject, using the fluorescent probe according to any one of [1] to [15], in which -CO-R₀ and optionally -CO-R¹⁰ is - Pro-Xaa (Pro represents a proline residue and Xaa represents an amino acid residue such as glycine, serine, or glutamic acid) in General Formula (II).
[18] A method for diagnosing pancreatic cancer or a method for predicting a possibility that a test subject from which a biological sample is derived has pancreatic cancer, the method comprising:
   detecting, by a microdevice, single-molecule enzyme activity of CD13 in a biological sample obtained from a subject, using the fluorescent probe according to any one of [1] to [15], in which -CO-R₀ and optionally -CO-R¹⁰ is an alanine, lysine, arginine, or methionine residue in General Formula (II); and
   detecting, by a microdevice, single-molecule enzyme activity of DPP4 in the biological sample obtained from the subject, using the fluorescent probe according to any one of [1] to [15], in which -CO-R₀ and optionally -CO-R¹⁰ is -Pro-Xaa (Pro represents a proline residue and Xaa represents an amino acid residue such as glycine, serine, or glutamine) in General Formula (II).
[19] The method according to any one of [16] to [18], wherein the biological sample is a biological sample of a patient with pancreatic cancer, a patient suspected of having pancreatic cancer, or a healthy subject.
[20] A compound represented by any one of Formulas (1) to (5) below or a salt thereof: 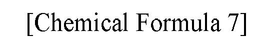 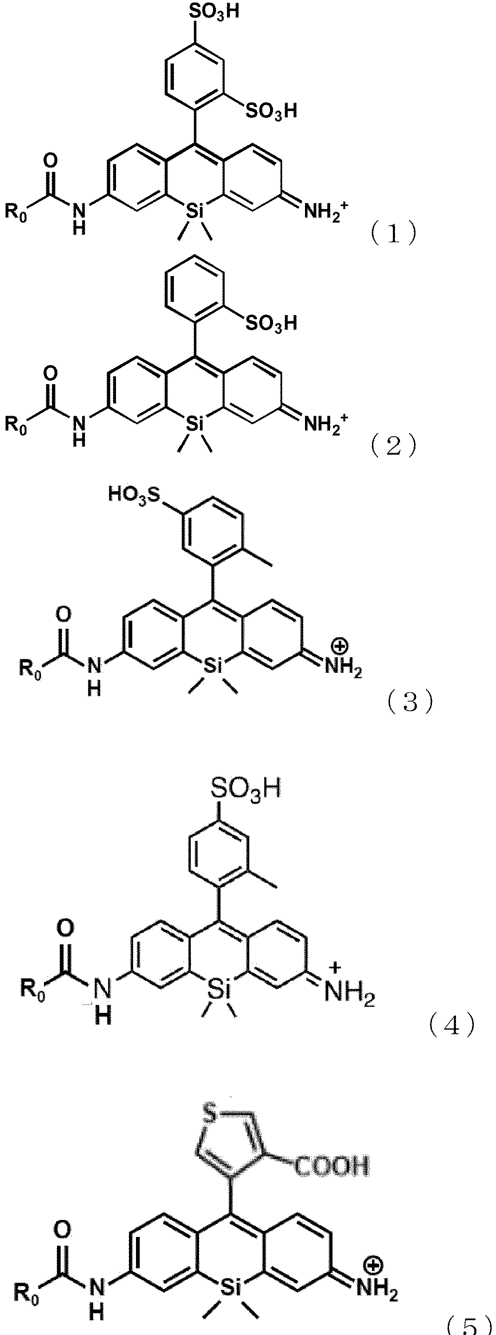 wherein
   R₀ is selected from a partial structure of an amino acid, a partial structure of a carboxylic acid, or T-O-,
   wherein the partial structure of an amino acid, together with C=O to which R₀ is bonded, constitutes an amino acid, an amino acid residue, a peptide, or a part of an amino acid,
   the partial structure of a carboxylic acid, together with C=O to which R₀ is bonded, constitutes a part of a carboxylic acid,
   T is a saccharide, a partial structure of a saccharide, or a saccharide having a self-cleaving linker, and
   the partial structure of a saccharide of T, together with O to which T is bonded, constitutes a saccharide or a part of a saccharide.
[21] A compound represented by Formula (6) below or a salt thereof: 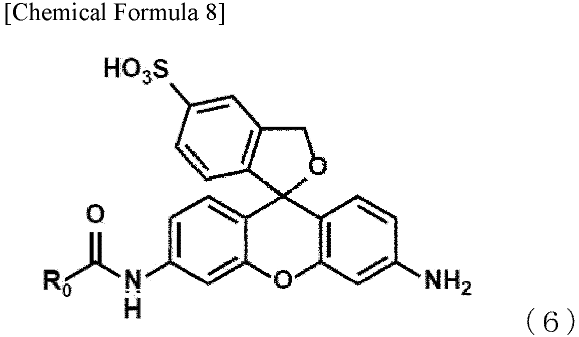 wherein
   R₀ is selected from a partial structure of an amino acid, a partial structure of a carboxylic acid, or T-O-,
   wherein the partial structure of an amino acid, together with C=O to which R₀ is bonded, constitutes an amino acid, an amino acid residue, a peptide, or a part of an amino acid,
   the partial structure of a carboxylic acid, together with C=O to which R₀ is bonded, constitutes a part of a carboxylic acid,
   T is a saccharide, a partial structure of a saccharide, or a saccharide having a self-cleaving linker, and
   the partial structure of a saccharide of T, together with O to which T is bonded, constitutes a saccharide or a part of a saccharide.
[22] The fluorescent probe according to any one of [1] to [15], wherein a solubility of the compound or a salt thereof in hexadecane in a solubility measurement test (25°C) in sealing oil is 0.3 µM or less.
[23] The fluorescent probe according to any one of [1] to [15] and [22], wherein a ratio of a fluorescence intensity of a well edge region to a fluorescence intensity of a well center region of an enzymolysate of the compound or a salt thereof in a fluorescence intensity measurement test in a microdevice is 0.6 or less.
[24] The fluorescent probe according to any one of [1] to [15], [22], and [23], wherein a ratio of a signal/noise ratio (first ratio) of an enzymolysate of the compound or a salt thereof in a microdevice to a signal/noise (second ratio) of the enzymolysate in a cuvette in an enzymolysis test of the fluorescent probe in the microdevice and the cuvette is 0.4 or more.
[101] Use of a compound represented by General Formula (III) below or a salt thereof, for the production of the compound according to any one of [1] to [15] or a salt thereof: 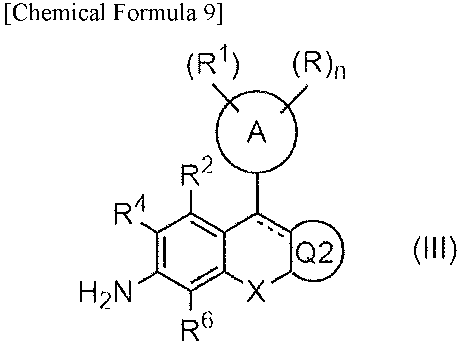 wherein ring A, ring Q2, X, n, R, R¹, R², R⁴, and R⁶ are as defined in General Formula (II), and a bond - - - represents a single bond or a double bond.
[102] Use of a compound represented by General Formula (IV) below or a salt thereof, for the production of the compound according to any one of [1] to [15] or a salt thereof: 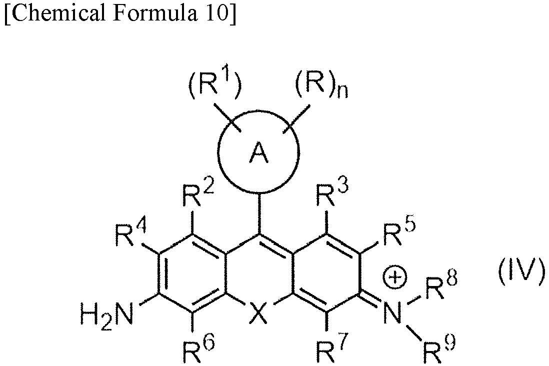 wherein ring A, X, n, R, and R¹ to R⁹ are as defined in General Formula (II).
[103] A compound represented by General Formula (V) below or a salt thereof: 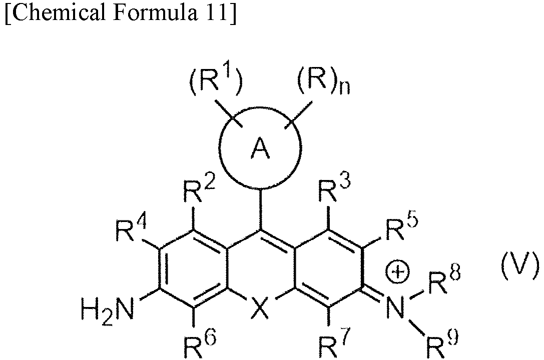 wherein
   ring A, R, R⁸, and R⁹ are as defined in General Formula (II),
   n is 1 or 2,
   R¹ is a hydrogen atom, an unsubstituted alkyl group having 1 to 4 carbon atoms, or an alkyl group substituted with a hydroxyl group and having 1 to 4 carbon atoms,
   R² to R⁷ are a hydrogen atom,
   R⁸ and R⁹ are each independently a hydrogen atom or a substituted or unsubstituted alkyl group having 1 to 4 carbon atoms, and
   X is an oxygen atom or Si(R^{a})(R^{b}) (R^{a} and R^{b} are as defined in Formula (II)).
[104] The compound according to [103] or a salt thereof, which is a compound selected from the following compounds, or a salt thereof: 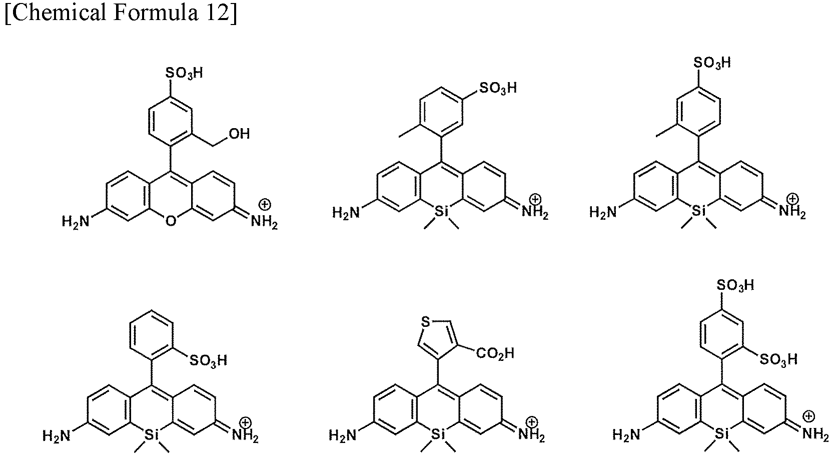
[105] Use of the compound according to [103] or [104] or a salt thereof, for the production of the compound according to any one of [1] to [15] or a salt thereof.
[201] A method of selecting a fluorescent probe, the method comprising:
   preparing the fluorescent probe according to any one of [1] to [15]; and
   selecting a fluorescent probe satisfying that (A) a solubility of the fluorescent probe and an enzymolysate thereof in hexadecane in a solubility measurement test (25°C) in sealing oil is 0.3 µM or less, (B) a ratio of a fluorescence intensity of a well edge region to a fluorescence intensity of a well center region of the enzymolysate of the fluorescent probe in a fluorescence intensity measurement test in a microdevice is 0.6 or less, and (C) a ratio of a signal/noise ratio (first ratio) of the enzymolysate in a microdevice to a signal/noise (second ratio) of the enzymolysate in a cuvette in an enzymolysis test of the fluorescent probe in the microdevice and the cuvette is 0.4 or more.

According to the present invention, the following inventions are also provided.
[301] A fluorescent probe for detecting a hydrolase for a microdevice, the fluorescent probe comprising a compound having at least one water-soluble substituent selected from the group consisting of -CO₂H, -PO₃H₂, and -SO₃H and represented by General Formula (I) below, or a salt thereof: 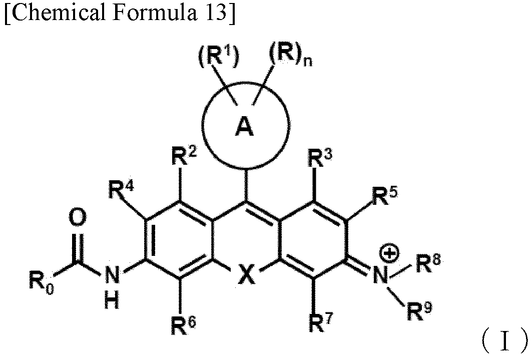 wherein 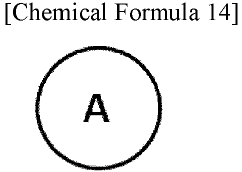
   is an aromatic ring which is phenyl or 5- or 6-membered heteroaryl;
   R is selected from the group consisting of -CO₂H, -PO₃H₂, -SO₃H, -L-CO₂H, -L-PO₃H₂, and -L-SO₃H, wherein L is a linker;
   n is an integer of 0 to 5;
   R¹ represents a monovalent substituent optionally present on the aromatic ring, wherein the substituents may be the same or different when two or more monovalent substituents are present;
   R² and R³ are each independently a hydrogen atom, a substituted or unsubstituted alkyl group having 1 to 6 carbon atoms, a halogen atom, the water-soluble substituent, or an alkyl group having the water-soluble substituent and having 1 to 6 carbon atoms;
   R⁴ and R⁵ are each independently a hydrogen atom, a substituted or unsubstituted alkyl group having 1 to 6 carbon atoms, a halogen atom, the water-soluble substituent, or an alkyl group having the water-soluble substituent and having 1 to 6 carbon atoms;
   R⁶ and R⁷ are each independently a hydrogen atom, a substituted or unsubstituted alkyl group having 1 to 6 carbon atoms, a halogen atom, the water-soluble substituent, or an alkyl group having water-soluble substituent and having 1 to 6 carbon atoms;
   R⁸ and R⁹ are each independently a hydrogen atom, a substituted or unsubstituted alkyl group having 1 to 6 carbon atoms, a substituted or unsubstituted alkenyl group having 1 to 6 carbon atoms, an alkyl group having the water-soluble substituent and having 1 to 6 carbon atoms, or an alkenyl group having the water-soluble substituent and having 1 to 6 carbon atoms,
   wherein R⁸ and R⁹ may together form a 4- to 7-membered heterocyclyl that may contain a nitrogen atom to which R⁸ and R⁹ are attached and may have a substituent,
   R⁸ or R⁹, or both of R⁸ and R⁹, together with R⁵ or R⁶, each may form a 5- to 7-membered heterocyclyl or heteroaryl containing a nitrogen atom to which R⁸ or R⁹ is attached, and further, the heterocyclyl or heteroaryl may have a substituent selected from the group consisting of alkyl having 1 to 6 carbon atoms, alkenyl having 2 to 6 carbon atoms, alkynyl having 2 to 6 carbon atoms, an aralkyl group having 6 to 10 carbon atoms, and an alkyl-substituted alkenyl group having 6 to 10 carbon atoms,
   the water-soluble substituent may be bonded to these heterocyclyl and heteroaryl directly or through the substituent;
   X is selected from an oxygen atom, Si(R^{a})(R^{b}), C(R^{a})(R^{b}), P(=O)R^{c}, S(R^{d})₂, Ge(R^{a})(R^{b}), a tellurium atom, or a bismuth atom,
   wherein R^{a} and R^{b} are each independently a substituted or unsubstituted alkyl group having 1 to 6 carbon atoms, or a substituted or unsubstituted aryl group,
   R^{c} is a substituted or unsubstituted alkyl group having 1 to 6 carbon atoms, or a substituted or unsubstituted phenyl group,
   R^{d} is a substituted or unsubstituted aryl group, which is the same or different,
   the alkyl group, the aryl group, and the phenyl group of R^{a}, R^{b}, R^{c} and R^{d} may be substituted with the water-soluble substituent; and
   R₀ is selected from a partial structure of an amino acid, a partial structure of a carboxylic acid, or T-O-,
   wherein the partial structure of an amino acid, together with C=O to which R₀ is bonded, constitutes an amino acid, an amino acid residue, a peptide, or a part of an amino acid,
   the partial structure of a carboxylic acid, together with C=O to which R₀ is bonded, constitutes a part of a carboxylic acid,
   T is a saccharide, a partial structure of a saccharide, or a saccharide having a self-cleaving linker, and
   the partial structure of a saccharide of T, together with O to which T is bonded, constitutes a saccharide or a part of a saccharide,
   a LogP value of the compound or a salt thereof being 1.09 or less.
[302] The fluorescent probe according to [301], wherein n is 1 to 3.
[303] The fluorescent probe according to any one of [301] or [302], wherein at least one of R² to R⁹ and R^{a}, R^{b}, R^{c} and R^{d} is the water-soluble substituent, an alkyl group having the water-soluble substituent and having 1 to 6 carbon atoms, an aryl group having the water-soluble substituent, and a phenyl group having the water-soluble substituent.
[304] The fluorescent probe according to any one of [301] to [303], wherein X is an oxygen atom or Si(R^{a})(R^{b}).
[305] The fluorescent probe according to [302], wherein R is -SO₃H or -L-SO₃H.
[306] The fluorescent probe according to any one of [301] to [305], wherein the hydrolase is a peptide bond hydrolase selected from an aminopeptidase, a peptidase, a protease, or an amidase.
[307] The fluorescent probe according to any one of [301] to [306], wherein the compound represented by General Formula (I) or a salt thereof is a compound represented by any one of General Formulas (Ia) to (Ie) below or a salt thereof: 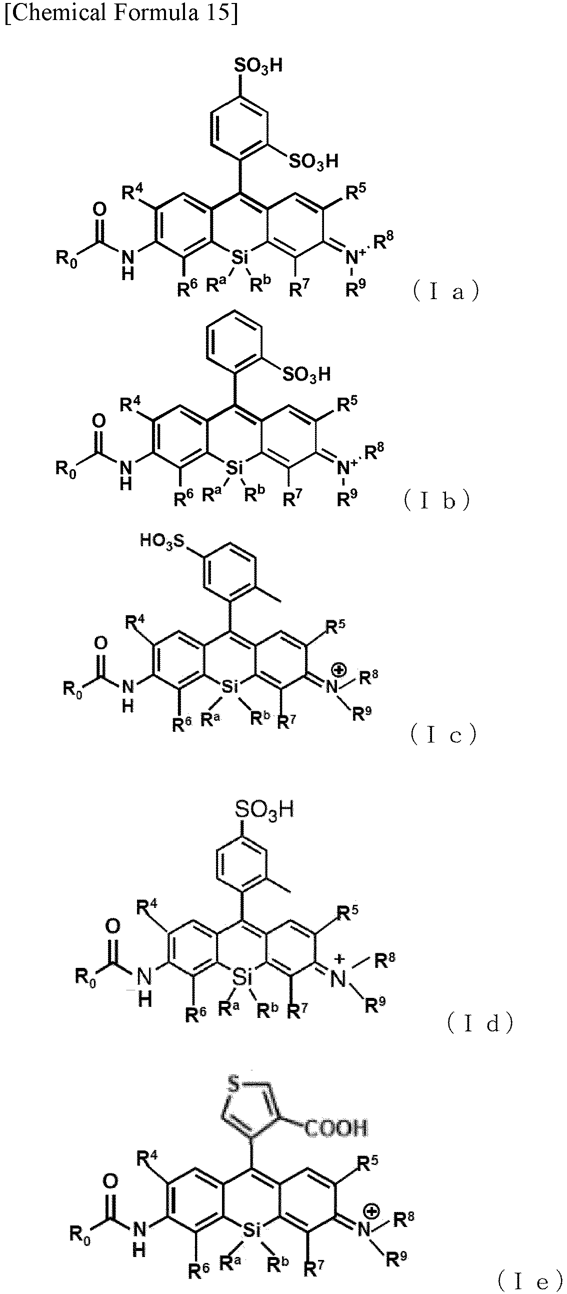 wherein R⁴ to R⁹, R^{a}, R^{b}, and R₀ are as defined in General Formula (I).
[308] The fluorescent probe according to any one of [301] to [307], wherein the compound represented by General Formula (I) or a salt thereof is a compound represented by General Formula (If) below or a salt thereof: 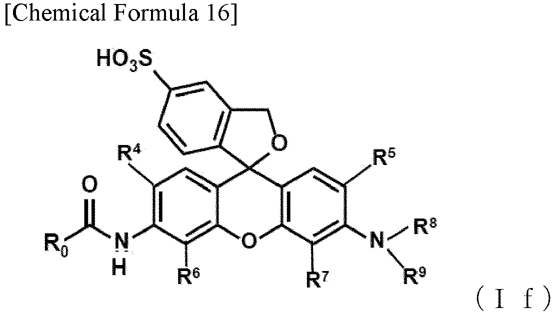 wherein R⁴ to R⁹ and R₀ are as defined in General Formula (I).
[309] A method for diagnosing pancreatic cancer, the method comprising detecting, by a microdevice, single-molecule enzyme activity of CD13 in a biological sample obtained from a subject, using the fluorescent probe according to any one of [301] to [308], in which -CO-R₀ is an alanine, lysine, arginine, or methionine residue in General Formula (I).
[310] A method for diagnosing pancreatic cancer, the method comprising detecting, by a microdevice, single-molecule enzyme activity of DPP4 in a biological sample obtained from a subject, using the fluorescent probe according to any one of [301] to [308], in which -CO-R₀ is -Pro-Xaa (Pro represents a proline residue and Xaa represents an amino acid residue such as glycine, serine, or glutamic acid) in General Formula (I).
[311] A method for diagnosing pancreatic cancer, the method comprising:
   detecting, by a microdevice, single-molecule enzyme activity of CD13 in a biological sample obtained from a subject, using the fluorescent probe according to any one of [301] to [308], in which -CO-R₀ is an alanine, lysine, arginine, or methionine residue in General Formula (I); and
   detecting, by a microdevice, single-molecule enzyme activity of DPP4 in a biological sample obtained from a subject, using the fluorescent probe according to any one of [301] to [308], in which -CO-R₀ is -Pro-Xaa (Pro represents a proline residue and Xaa represents an amino acid residue such as glycine, serine, or glutamine) in General Formula (I).
[312] A compound represented by any one of Formulas (1) to (5) below or a salt thereof: 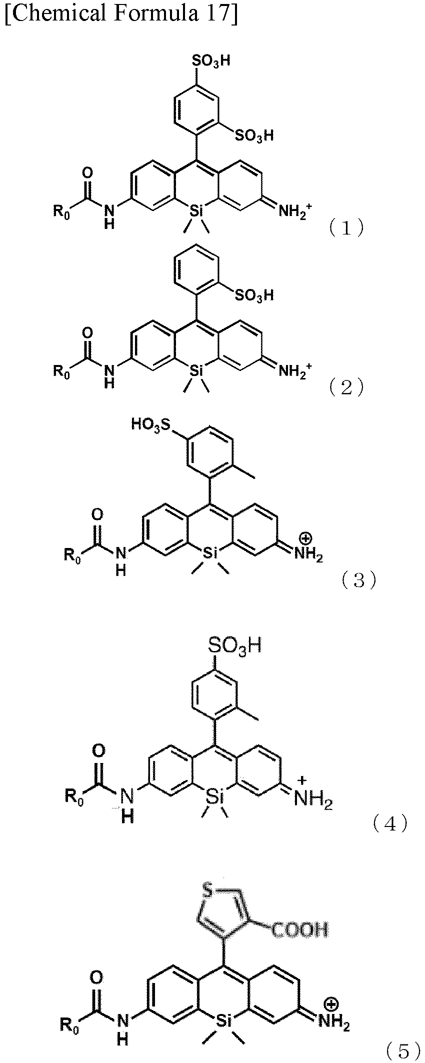 wherein
   R₀ is selected from a partial structure of an amino acid, a partial structure of a carboxylic acid, or T-O-,
   wherein the partial structure of an amino acid, together with C=O to which R₀ is bonded, constitutes an amino acid, an amino acid residue, a peptide, or a part of an amino acid,
   the partial structure of a carboxylic acid, together with C=O to which R₀ is bonded, constitutes a part of a carboxylic acid,
   T is a saccharide, a partial structure of a saccharide, or a saccharide having a self-cleaving linker, and
   the partial structure of a saccharide of T, together with O to which T is bonded, constitutes a saccharide or a part of a saccharide.
[313] A compound represented by Formula (6) below or a salt thereof: 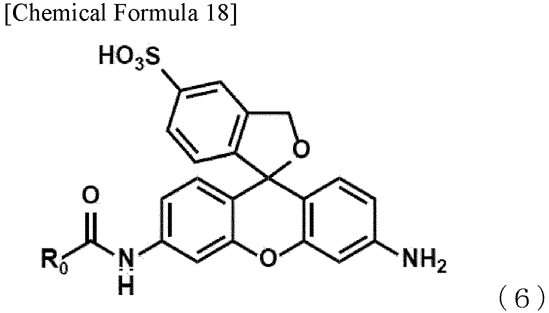 wherein
   R₀ is selected from a partial structure of an amino acid, a partial structure of a carboxylic acid, or T-O-,
   wherein the partial structure of an amino acid, together with C=O to which R₀ is bonded, constitutes an amino acid, an amino acid residue, a peptide, or a part of an amino acid,
   the partial structure of a carboxylic acid, together with C=O to which R₀ is bonded, constitutes a part of a carboxylic acid,
   T is a saccharide, a partial structure of a saccharide, or a saccharide having a self-cleaving linker, and
   the partial structure of a saccharide of T, together with O to which T is bonded, constitutes a saccharide or a part of a saccharide.

### Effects of the Invention

According to the present invention, it is possible to provide a red fluorescent probe and a green fluorescent probe that can be used in an enzyme activity detection method, and the present invention is particularly advantageous in that these fluorescent probes can be used in an enzyme activity detection method using a microdevice.

The fluorescent probe of the present invention can be effectively used for detection of pancreatic cancer or the like.

According to the present invention, for example, by using the fluorescent probe of the present invention, it is possible to provide a method for diagnosing pancreatic cancer by detecting single-molecule enzyme activity of DPP4 in blood and detecting single-molecule enzyme activity of CD13 in blood.

### BRIEF DESCRIPTION OF THE DRAWINGS

Fig. 1 is a perspective view schematically illustrating a microdevice in which a fluorescent probe of the present invention is used.
Fig. 2 shows fluorescence microscopic images of measurements performed in Examples 1 to 15 using sHMRG- and sSiR-based peptidase and protease probes.
Fig. 3 shows the results of a diagnostic experiment of pancreatic disease (pancreatic cancer) utilizing single-molecule enzyme activity of CD13 in Example 17.
Fig. 4 shows the results of a diagnostic experiment of pancreatic disease (pancreatic cancer) utilizing single-molecule enzyme activity of DPP4 in Example 18.
Fig. 5 shows a calibration curve for calculating solubility in sealing oil used in Example 19. The horizontal axis represents the concentration (µm) of the fluorescent probe, and the vertical axis represents the peak area of LC.
Fig. 6 shows the results of evaluating the degree of adsorption of a test compound to a well wall surface on the basis of a fluorescence intensity of a center region (Center) and a fluorescence intensity (AU) of an edge region (Edge) in Example 20. The image in the middle row in the center part of the drawing shows the center region where the fluorescence intensity is analyzed, and the image in the right row shows the edge region where the fluorescence intensity is analyzed.
Fig. 7 shows the results of measuring fluorescence spectra before and after an enzyme reaction using a cuvette in Example 21. A is the result for Glu-sSiR, and B is the result for GP-sHMRG.
Fig. 8 shows the results of measuring fluorescence spectra before and after an enzyme reaction using a microdevice in Example 21. A is the result for Glu-sSiR, and B is the result for GP-sHMRG. In both A and B, the image in the left row shows the result of only the assay buffer, the image in the middle row shows the result in the presence of a test compound, and the image in the right row shows the result in the presence of a test compound and an enzyme.

### DETAILED DESCRIPTION OF THE INVENTION

In the present specification, "halogen atom" means a fluorine atom, a chlorine atom, a bromine atom, or an iodine atom.

In the present specification, "alkyl" may be any aliphatic hydrocarbon group that is linear, branched, cyclic, or in a combination thereof. The number of carbon atoms in the alkyl group is not particularly limited, but is, for example, 1 to 4 (C₁₋₄), 1 to 6 (C₁₋₆), 1 to 10 (C₁₋₁₀), 1 to 15 (C₁₋₁₅), or 1 to 20 (C₁₋₂₀). When the number of carbon atoms is specified, it means an "alkyl" having the number of carbon atoms within that numerical range. For example, C₁₋₈ alkyls include methyl, ethyl, n-propyl, isopropyl, n-butyl, isobutyl, sec-butyl, tert-butyl, n-pentyl, isopentyl, neo-pentyl, n-hexyl, isohexyl, n-heptyl, n-octyl, and the like. In the present specification, an alkyl group may have one or more optional substituents. Examples of such substituents include, but are not limited to, an alkoxy group, a halogen atom, an amino group, a mono- or di-substituted amino group, a substituted silyl group, and acyl. When an alkyl group has two or more substituents, they may be the same as or different from each other. The same is also true for the alkyl moiety of other substituents (for example, an alkoxy group, an arylalkyl group, or the like) including an alkyl moiety.

In the present specification, when certain functional groups are defined as "optionally substituted", the type of substituent, substitution position, and the number of substituents are not particularly limited, and when there are two or more substituents, they may be the same as or different from each other. Examples of the substituents include, but are not limited to, an alkyl group, an alkoxy group, a hydroxyl group, a carboxyl group, a halogen atom, a sulfo group, an amino group, an alkoxycarbonyl group, and an oxo group. Other substituents may be present in these substituents. Examples of such cases include, but are not limited to, an alkyl halide group and a dialkylamino group.

In the present specification, "aryl" may be either a monocyclic aromatic hydrocarbon group or a condensed polycyclic aromatic hydrocarbon group, and may be an aromatic heterocyclic ring containing one or more heteroatoms (such as an oxygen atom, a nitrogen atom, or a sulfur atom) as ring forming atoms. In this case, the aromatic heterocyclic ring may also be referred to as "heteroaryl" or "heteroaromatic". The aryl may be attached at all possible positions, whether it is a monocyclic ring or a condensed ring. Non-limiting examples of the monocyclic aryl include a phenyl group (Ph), a thienyl group (2- or 3-thienyl group), a pyridyl group, a furyl group, a thiazolyl group, an oxazolyl group, a pyrazolyl group, a 2-pyrazinyl group, a pyrimidinyl group, a pyrrolyl group, an imidazolyl group, a pyridazinyl group, a 3-isothiazolyl group, a 3-isoxazolyl group, a 1,2,4-oxadiazole-5-yl group, and a 1,2,4-oxadiazole-3-yl group. Non-limiting examples of the condensed polycyclic aryl include a 1-naphthyl group, a 2-naphthyl group, 1-indenyl group, 2-indenyl group, a 2,3-dihydroindene-1-yl group, a 2,3-dihydroindene-2-yl group, a 2-anthryl group, an indazolyl group, a quinolyl group, an isoquinolyl group, a 1,2-dihydroisoquinolyl group, a 1,2,3,4-tetrahydroisoquinolyl group, an indolyl group, an isoindolyl group, a phthalazinyl group, a quinoxalinyl group, a benzofuranyl group, a 2,3-dihydrobenzofuran-1-yl group, a 2,3-dihydrobenzofuran-2-yl group, a 2,3-dihydrobenzothiophene-1-yl group, a 2,3-dihydrobenzothiophene-2-yl group, a benzothiazolyl group, a benzimidazolyl group, a fluorenyl group, and a thioxanthenyl group. In the present specification, an aryl group may have one or more optional substituents on the ring thereof. Examples of the substituents include, but are not limited to, an alkoxy group, a halogen atom, an amino group, a mono- or di-substituted amino group, a substituted silyl group, and acyl. When an aryl group has two or more substituents, they may be the same as or different from each other. The same is also true for the aryl moiety of other substituents (for example, an aryloxy group, an arylalkyl group, or the like) including an aryl moiety.

In the present specification, "alkoxy group" is a structure in which the above alkyl group is bonded to an oxygen atom, and examples thereof include saturated alkoxy group that is linear, branched, cyclic, or in a combination thereof. Preferred examples thereof include a methoxy group, an ethoxy group, a n-propoxy group, an isopropoxy group, a cyclopropoxy group, a n-butoxy group, an isobutoxy group, a s-butoxy group, a t-butoxy group, a cyclobutoxy group, a cyclopropylmethoxy group, a n-pentyloxy group, a cyclopentyloxy group, a cyclopropylethyloxy group, a cyclobutylmethyloxy group, a n-hexyloxy group, a cyclohexyloxy group, a cyclopropylpropyloxy group, a cyclobutylethyloxy group, and a cyclopentylmethyloxy group.

In the present specification, the "alkylene" is a divalent group composed of a linear or branched saturated hydrocarbon, and examples thereof include methylene, 1-methylmethylene, 1,1-dimethylmethylene, ethylene, 1-methylethylene, 1-ethylethylene, 1,1-dimethylethylene, 1,2-dimethylethylene, 1,1-diethylethylene, 1,2-diethylethylene, 1-ethyl-2-methylethylene, trimethylene, 1-methyltrimethylene, 2-methyltrimethylene, 1,1-dimethyltrimethylene, 1,2-dimethyltrimethylene, 2,2-dimethyltrimethylene, 1-ethyltrimethylene, 2-ethyltrimethylene, 1,1-diethyltrimethylene, 1,2-diethyltrimethylene, 2,2-diethyltrimethylene, 2-ethyl-2-methyltrimethylene, tetramethylene, 1-methyltetramethylene, 2-methyltetramethylene, 1,1-dimethyltetramethylene, 1,2-dimethyltetramethylene, 2,2-dimethyltetramethylene, and 2,2-di-n-propyltrimethylene.

"Amide" used in the present specification includes both RNR'CO- (in the case of R=alkyl, alkaminocarbonyl-) and RCONR'- (in the case of R=alkyl, alkylcarbonyl amino-).

"Ester" used in the present specification includes both ROCO- (in the case of R=alkyl, alkoxycarbonyl-) and RCOO- (in the case of R=alkyl, alkylcarbonyloxy-).

### 1. Fluorescent probe of Present Invention

According to the present invention, there is provided a fluorescent probe for detecting a hydrolase, the fluorescent probe containing a compound represented by General Formula (II) below or a salt thereof.

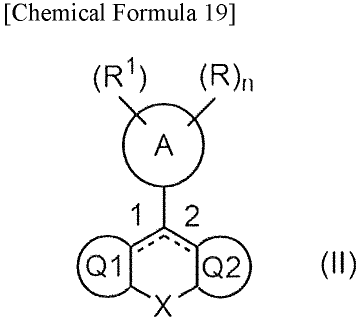

One mode of the present invention is a fluorescent probe for detecting a hydrolase, the fluorescent probe containing a compound represented by General Formula (I) below or a salt thereof.

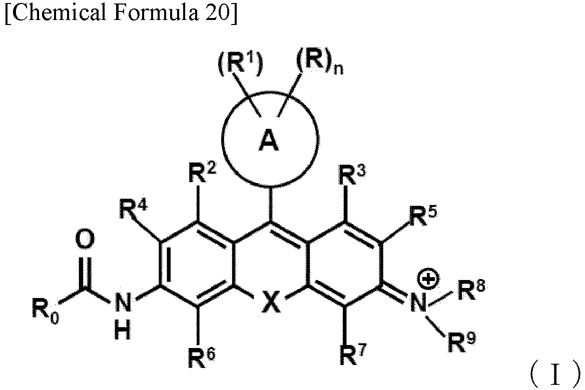

In the present specification, the compound satisfying the above-described requirements, or a salt thereof is also referred to as the "compound of the present invention", and the fluorescent probe containing the compound of the present invention is also referred to as the "fluorescent probe of the present invention".

The compound of the present invention has at least one water-soluble substituent selected from the group consisting of -CO₂H, -PO₃H₂, and -SO₃H.

As described below, the compound of the present invention can be specified by a MiLogP value or a LogP value which is an index of lipophilicity. For example, the MiLogP value of the compound of the present invention can be set to 2.56 or less, or less than 2.47, and is preferably 2.40 or less, 2.30 or less, 2.20 or less, 2.10 or less, 2.00 or less, 1.90 or less, 1.80 or less, 1.70 or less, or 1.64 or less, and more preferably 1.60 or less, 1.50 or less, 1.40 or less, 1.34 or less, 1.30 or less, 1.22 or less, 1.20 or less, 1.10 or less, or 1.00 or less (particularly preferably 0 or less). The lower limit value of the MiLogP value of the compound of the present invention is not particularly specified, but is, for example, about -3.1 or more. For a compound in which X is an oxygen atom in Formulas (I) and (II) among the compounds of the present invention, the MiLogP value can be set to 2.40 or less, 2.30 or less, 2.20 or less, 2.10 or less, 2.00 or less, 1.90 or less, 1.80 or less, or 1.70 or less, preferably 1.60 or less, 1.50 or less, 1.40 or less, 1.30 or less, 1.20 or less, 1.10 or less, or 1.00 or less (more preferably 0 or less), and the lower limit value of the MiLogP value can be set to, for example, about -3.1 or more. For a compound in which X is Si(R^{a})(R^{b}) in Formulas (I) and (II) among the compounds of the present invention, the MiLogP value can be set to 2.56 or less, 2.50 or less, 2.40 or less, 2.30 or less, 2.20 or less, 2.10 or less, 2.00 or less, 1.90 or less, 1.80 or less, 1.70 or less, or 1.64 or less, preferably 1.60 or less, 1.50 or less, 1.40 or less, 1.34 or less, 1.30 or less, 1.22 or less, 1.20 or less, or 1.10 or less (more preferably 1.00 or less), and the lower limit value of the MiLogP value can be set to, for example, about - 3.1 or more or 0 or more. In a preferred mode of the present invention, the MiLogP value of the enzymolysate of the compound of the present invention can also be specified in the same manner as described above. The enzymolysate of the compound of the present invention may have, for example, a structure of Formula (III), and the structure of Formula (III) includes a structure of a fluorescent mother nucleus.

The LogP value of the compound of the present invention can be set to less than 1.17, 1.16 or less, 1.15 or less, 1.14 or less, 1.13 or less, 1.12 or less, 1.11 or less, or 1.10 or less, and is preferably 1.09 or less, 1.08 or less, 1.07 or less, 1.06 or less, 1.05 or less, 1.04 or less, 1.03 or less, or 1.02 or less, and more preferably 1.01 or less, 1.0 or less, 0.9 or less, 0.8 or less, 0.7 or less, 0.6 or less, or 0.5 or less (particularly preferably 0.46 or less or 0.42 or less). The lower limit value of the LogP value of the compound of the present invention is not particularly specified, and the smaller the lower limit value, the higher the water solubility, but is, for example, about -3 or more. In a preferred mode of the present invention, the LogP value of the enzymolysate of the compound of the present invention can also be specified in the same manner as described above. The enzymolysate of the compound of the present invention may have, for example, a structure of Formula (III), and the structure of Formula (III) includes a structure of a fluorescent mother nucleus.

As described below, the fluorescent probe of the present invention can be preferably used as a fluorescent probe for a microdevice.

In the compound of the present invention, it is important to essentially have at least one water-soluble substituent selected from the group consisting of a carboxyl group (-CO₂H), a phosphate group (-PO₃H₂), and a sulfonate group (-SO₃H).

In the structure of the compound used in a microdevice that has been studied so far, the mother nucleus is limited to a xanthene skeleton, and a rhodamine skeleton having higher lipophilicity than the xanthene skeleton has not been studied. Since rhodamine and Si rhodamine have high lipophilicity, when a fluorescent probe using these compounds (for example, an OFF-ON type fluorescent probe emitting fluorescence by reacting with a target enzyme) is used in a method for detecting enzyme activity using a microdevice such as a multi-well chamber type microdevice, leakage of the fluorescent probe from an aqueous solution in a well of the device into an organic solvent used for sealing outside the device more easily occurs when the device is enclosed, and it has been considered that rhodamine and Si rhodamine are not suitable for measurement requiring high quantitativity and sensitivity.

As for an enzyme recognition site, in the fluorescent probes for a microdevice that have been studied so far, the water solubility of the enzyme recognition site is high (such as a phosphate ester), but in many of the fluorescent probes for detection of enzymes and the like that hydrolyze peptide bonds, glycoside bonds, and the like that are intended by the invention of the present application, the lipophilicity of the enzyme recognition site is high (such as a peptide), so that leakage of the fluorescent probe into an organic solvent becomes more problematic.

As a result of intensive studies on the above points, the present inventors have found that the above problems can be solved by introducing a specific water-soluble substituent into the molecular skeleton of rhodamine, Si-based rhodamine, or the like in the fluorescent probe of the present invention. The present inventors have also found that by introducing a specific water-soluble substituent into the molecular skeleton of rhodamine and setting a MiLogP value and/or a LogP value, which is an index of lipophilicity as the compound molecule, within a specific range, excellent applicability to a microdevice is achieved. As a result, for the first time as a fluorescent probe having a rhodamine skeleton, it has become possible to provide a fluorescent probe that can be used in an enzyme activity detection method using a microdevice.

According to one mode of the present invention, there is provided a fluorescent probe for detecting a hydrolase for a microdevice, the fluorescent probe containing a compound having at least one water-soluble substituent selected from the group consisting of -CO₂H, -PO₃H₂, and -SO₃H and represented by General Formula (II), or a salt thereof, in which a MiLogP value is less than 2.47 and/or a LogP value is 1.09 or less. According to one mode of the present invention, there is also provided a fluorescent probe for detecting a hydrolase for a microdevice, the fluorescent probe containing a compound having at least one water-soluble substituent selected from the group consisting of -CO₂H, -PO₃H₂, and -SO₃H and represented by General Formula (I), or a salt thereof, in which a MiLogP value is less than 2.47 and/or a LogP value is 1.09 or less. Hereinafter, each constituent component will be described in detail.

### (1) Compound of Present Invention

In General Formulas (I) and (II), the ring A: 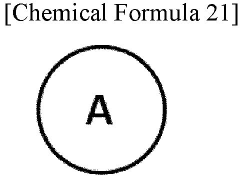 is an aromatic ring which is phenyl (benzene ring) or a 5- or 6-membered heteroaryl.

The 5- or 6-membered heteroaryl is an aromatic heterocyclic ring containing one to three heteroatoms such as oxygen, nitrogen, and sulfur, and examples thereof include, but are not limited to, pyrrole, furan, thiophene, imidazole, pyrazole, triazole, pyridine, pyran, thiopyran, pyrazine, pyrimidine, pyridazine, 1,2-dioxine, 1,4-dioxine, 1,2-dithiine, 1,4-dithiine, and triazine.

The aromatic ring is preferably phenyl (benzene ring), pyrrole, thiophene, furan, or pyridine.

In General Formula (II), when Q2 represents a ring structure of Formula (iii), a bond - - - as 1 represents a single bond, a bond - - - as 2 represents a double bond, and Q1 represents a ring structure of Formula (i).

In General Formula (II), when Q2 represents a ring structure of Formula (iv), (v), or (vi),

R¹ is bonded to a carbon atom on an X atom-containing ring structure to which ring A is directly bonded to form a spiro ring, a bond - - - as 1 and a bond - - - as 2 represent a single bond, and Q1 represents a ring structure of Formula (i), or
a bond - - - as 1 represents a double bond, a bond - - - as 2 represents a single bond, and Q1 represents a ring structure of Formula (ii).

In the present invention, the "X atom-containing ring structure" is a xanthene ring when X is an oxygen atom, but in the present invention, the "xanthene skeleton" is used to mean that X also includes a ring structure other than an oxygen atom.

In General Formulas (I) and (II), R is selected from the group consisting of - CO₂H, -PO₃H₂, -SO₃H, -L-CO₂H, -L-PO₃H₂, and -L-SO₃H. L represents a linker. That is, in the compounds represented by General Formulas (I) and (II) or salts thereof, the water-soluble substituent selected from the group consisting of -CO₂H, -PO₃H₂, and -SO₃H may be bonded to the aromatic ring directly or through a linker.

The linker of L is selected from the group consisting of an alkyl group having 1 to 10 carbon atoms, preferably 1 to 6 carbon atoms, which may have a substituent (provided that, one or more of -CH₂- of the alkyl group may be substituted by -O-, -S-, - NH-, or -CO-), an aryl group (including a heteroaryl group), a cycloalkyl group, an alkenyl group having 1 to 10 carbon atoms, preferably 1 to 6 carbon atoms, an alkoxy group having 1 to 10 carbon atoms, preferably 1 to 6 carbon atoms, an alkoxycarbonyl group (-CO-OR'), an alkylamino group (-NHR', -NR'₂), an ester group (-O-CO-R'), an amide group (-NHCOR', -CONHR', -CONR'₂), a polyethylene glycol chain, and a group formed by optionally bonding two or more groups selected from these groups. R' is a substituted or unsubstituted alkyl group or a substituted or unsubstituted aryl group, and when there are two or more of R', they may be the same as or different from each other.

As the linker of L, an amide can be suitably used from the viewpoint of ease of synthesis, but any of the above can be used from the viewpoint of physical properties of the fluorescent probe, and preferably, an alkyl group having 1 to 6 carbon atoms which may have a substituent, an amide group (more preferably -CONHR' or -CONR'₂), or a phenyl group can be used.

In the present invention, R is preferably -CO₂H, -SO₃H, -L-CO₂H, or -L-SO₃H, and R is more preferably -SO₃H or -L-SO₃H.

n is an integer of 0 to 5. In the compounds represented by General Formulas (I) and (II) or salts thereof, the water-soluble substituent can be introduced into the aromatic ring directly or through a linker. In the compounds represented by General Formulas (I) and (II) or salts thereof, the water-soluble substituent can be introduced into the xanthene skeleton directly or through a linker without being introduced into the aromatic ring (that is, n = 0), and in addition to or instead of introducing the water-soluble substituent into the xanthene skeleton directly or through a linker, the water-soluble substituent can be introduced into a cyclohexyl ring or a cycloaryl ring formed through a nitrogen atom bonded to the xanthene skeleton directly or through another substituent as described below.

In one preferred mode of the present invention, n is 1 to 5, more preferably 1 to 3.

In another preferred mode of the present invention, the water-soluble substituent is introduced only into the aromatic ring directly or through a linker, and n is 1 to 3 or 1 to 2.

When n is 2 or more, R may be the same or different.

In General Formulas (I) and (II), R¹ represents a monovalent substituent optionally present on the aromatic ring. When there are two or more monovalent substituents, they may be the same or different. The fact that R¹ is optionally present on the aromatic ring means that both a case where R¹ is present on the aromatic ring and a case where R¹ is not present on the aromatic ring are included.

The type of the monovalent substituent represented by R¹ is not particularly limited, and for example, is preferably selected from the group consisting of an alkyl group having 1 to 6 carbon atoms, an alkenyl group having 1 to 6 carbon atoms, an alkynyl group having 1 to 6 carbon atoms, an alkoxy group having 1 to 6 carbon atoms, a hydroxyl group, an alkoxycarbonyl group, a halogen atom, and an amino group.

These monovalent substituents may further have one or two or more arbitrary substituents. For example, one or two or more halogen atoms, hydroxyl groups, amino groups, alkoxy groups, and the like may exist in the alkyl group represented by R¹, and for example, the alkyl group represented by R¹ may be a halogenated alkyl group, a hydroxyalkyl group, an aminoalkyl group, or the like.

The monovalent substituent represented by R¹ is introduced into the aromatic ring separately from R (selected from the group consisting of -CO₂H, -PO₃H₂, -SO₃H, - L-CO₂H, -L-PO₃H₂, and -L-SO₃H) described above, when n is 0, R¹ may or may not be present, and when n is 1 or more, R¹ may or may not be present.

For example, in a case where the aromatic ring is a phenyl group (benzene ring), when n is 1, the monovalent substituent can be present on one to four benzene rings, and when n is 2, the monovalent substituent can be present on one to three benzene rings.

R¹, together with a carbon atom on the ring A to which R¹ is bonded, a carbon atom (spiro atom) on the X atom-containing ring structure to which the ring A is bonded, and a part of the ring A, may form a 5- or 6-membered carbocyclic or heterocyclic ring (spiro ring).

In one preferred mode of the present invention, R¹ is absent or an alkyl group having 1 to 6 carbon atoms, preferably a methyl group or an ethyl group.

In still another preferred mode of the present invention, as R¹, one alkyl group having 1 to 6 carbon atoms, preferably, methyl group, is present on the aromatic ring.

In this case, the alkyl group having 1 to 6 carbon atoms, preferably methyl group, is preferably bonded to the 2-position of the aromatic ring (the para-position when the aromatic ring is a benzene ring) with respect to the position where the alkyl group is bonded to the xanthene ring.

In General Formulas (I) and (II), R² and R³ each independently represent a hydrogen atom, a substituted or unsubstituted alkyl group having 1 to 6 carbon atoms, a halogen atom, the water-soluble substituent, or an alkyl group having the water-soluble substituent and having 1 to 6 carbon atoms.

When R² or R³ represents an alkyl group, one or two or more halogen atoms, hydroxyl groups, amino groups, alkoxy groups, and the like may exist in the alkyl group, and for example, the alkyl group represented by R² or R³ may be a halogenated alkyl group, a hydroxyalkyl group, or the like.

At least one of R² and R³ may be a water-soluble substituent selected from - CO₂H, -PO₃H₂, or -SO₃H, or an alkyl group having the water-soluble substituent and having 1 to 6 carbon atoms.

In one aspect of the present invention, R² and R³ are each independently a hydrogen atom or a halogen atom, and more preferably, both R² and R³ are a hydrogen atom.

In another aspect of the present invention, R² and R³ are each independently a hydrogen atom, a water-soluble substituent selected from -CO₂H, -PO₃H₂, or -SO₃H, or an alkyl group having the water-soluble substituent and having 1 to 6 carbon atoms.

In General Formulas (I) and (II), R⁴ and R⁵ each independently represent a hydrogen atom, a substituted or unsubstituted alkyl group having 1 to 6 carbon atoms, a halogen atom, the water-soluble substituent, or an alkyl group having the water-soluble substituent and having 1 to 6 carbon atoms, and the details thereof are the same as those described for R² and R³.

In one aspect of the present invention, R⁴ and R⁵ are each independently a hydrogen atom or a halogen atom, and more preferably, both R⁴ and R⁵ are a hydrogen atom.

In another aspect of the present invention, R⁴ and R⁵ are each independently a hydrogen atom, a water-soluble substituent selected from -CO₂H, -PO₃H₂, or -SO₃H, or an alkyl group having the water-soluble substituent and having 1 to 6 carbon atoms.

In General Formulas (I) and (II), R⁶ and R⁷ are each independently a hydrogen atom, a substituted or unsubstituted alkyl group having 1 to 6 carbon atoms, a halogen atom, the water-soluble substituent, or an alkyl group having the water-soluble substituent and having 1 to 6 carbon atoms, and the details thereof are the same as those described for R² and R³.

In one aspect of the present invention, R⁶ and R⁷ are each independently a hydrogen atom or a halogen atom, and more preferably, both R⁴ and R⁵ are a hydrogen atom.

In another aspect of the present invention, R⁶ and R⁷ are each independently a hydrogen atom, a water-soluble substituent selected from -CO₂H, -PO₃H₂, or -SO₃H, or an alkyl group having the water-soluble substituent and having 1 to 6 carbon atoms.

In General Formulas (I) and (II), R⁸ and R⁹ are each independently a hydrogen atom, a substituted or unsubstituted alkyl group having 1 to 6 carbon atoms, a substituted or unsubstituted alkenyl group having 1 to 6 carbon atoms, or an alkyl group having the water-soluble substituent and having 1 to 10 carbon atoms, preferably 1 to 6 carbon atoms (this alkyl group may be substituted with an aryl group, and the aryl group may be substituted with the water-soluble substituent) or an alkenyl group having the water-soluble substituent and having 1 to 10 carbon atoms, preferably 1 to 6 carbon atoms (this alkenyl group may be substituted with an aryl group, and the aryl group may be substituted with the water-soluble substituent).

R⁸ and R⁹ may together form a 4- to 7-membered heterocyclyl containing a nitrogen atom to which R⁸ and R⁹ are attached.

R⁸ or R⁹, or both of R⁸ and R⁹, together with R⁵ or R⁷, each may form a 5- to 7-membered heterocyclyl or heteroaryl containing a nitrogen atom to which R⁸ or R⁹ is attached, and further, the heterocyclyl or heteroaryl may have a substituent selected from the group consisting of alkyl having 1 to 6 carbon atoms, alkenyl having 2 to 6 carbon atoms, alkynyl having 2 to 6 carbon atoms, an aralkyl group having 6 to 10 carbon atoms, and an alkyl-substituted alkenyl group having 6 to 10 carbon atoms.

As ring members of the heterocyclyl or heteroaryl, one to three additional heteroatoms selected from the group consisting of an oxygen atom, a nitrogen atom, and a sulfur atom may be contained.

The water-soluble substituent may be bonded to these heterocyclyl and heteroaryl directly or through the substituent.

In one preferred mode of the present invention, both R⁸ and R⁹ are a hydrogen atom.

In General Formulas (I) and (II), R¹¹ is a hydrogen atom, a substituted or unsubstituted alkyl group having 1 to 6 carbon atoms, a substituted or unsubstituted alkenyl group having 1 to 6 carbon atoms, an alkyl group having the water-soluble substituent and having 1 to 10 carbon atoms, preferably 1 to 6 carbon atoms (this alkyl group may be substituted with an aryl group, and the aryl group may be substituted with the water-soluble substituent), or an alkenyl group having the water-soluble substituent and having 1 to 10 carbon atoms, preferably 1 to 6 carbon atoms (this alkenyl group may be substituted with an aryl group, and the aryl group may be substituted with the water-soluble substituent).

In General Formulas (I) and (II), X is selected from an oxygen atom, Si(R^{a})(R^{b}), C(R^{a})(R^{b}), P(=O)R^{c}, S(R^{d})₂, Ge(R^{a})(R^{b}), a tellurium atom, or a bismuth atom.

R^{a} and R^{b} are each independently a substituted or unsubstituted alkyl group having 1 to 6 carbon atoms or a substituted or unsubstituted aryl group.

R^{a} and R^{b} are each independently preferably an alkyl group having 1 to 3 carbon atoms, and more preferably, both R^{a} and R^{b} are a methyl group.

One or two or more halogen atoms, hydroxyl groups, amino groups, alkoxy groups, and the like may exist in the alkyl groups represented by R^{a} and R^{b}, and for example, the alkyl groups may be a halogenated alkyl group, a hydroxyalkyl group, or the like. When R^{a} and/or R^{b} is an aryl group, the aryl group may be either a monocyclic aromatic group or a fused aromatic group, and the aryl ring may contain one or two or more ring-constituting heteroatoms (for example, a nitrogen atom, an oxygen atom, a sulfur atom, or the like). The aryl group is preferably a phenyl group. One or two or more substituents may be present on the aryl ring. As the substituent, for example, one or two or more halogen atoms, hydroxyl groups, amino groups, alkoxy groups, and the like may be present.

The alkyl group and the aryl group defined as R^{a} and R^{b} may be substituted with a water-soluble substituent selected from -CO₂H, -PO₃H₂, or -SO₃H.

R^{c} is a substituted or unsubstituted alkyl group having 1 to 6 carbon atoms or a substituted or unsubstituted phenyl group.

The alkyl group or phenyl group defined as R^{c} may be substituted with the water-soluble substituent.

R^{d} is a substituted or unsubstituted aryl group, which is the same or different.

The aryl group defined as R^{d} may be substituted with the water-soluble substituent.

In one preferred mode of the present invention, X is an oxygen atom or Si(R^{a})(R^{b}).

In General Formulas (I) and (II), R₀ and R¹⁰ are each independently selected from a partial structure of an amino acid, a partial structure of a carboxylic acid, T-O-, or a saturated or unsaturated 5- or 6-membered carbocyclic or heterocyclic group.

The partial structure of an amino acid means that it constitutes an amino acid, an amino acid residue, a peptide, or a part of an amino acid together with C=O to which R₀ or R¹⁰ is bonded.

T is a saccharide, a partial structure of a saccharide, or a saccharide having a self-cleaving linker, and the partial structure of a saccharide of T, together with O to which T is bonded, constitutes a saccharide or a part of a saccharide.

Examples of the saturated or unsaturated 5- or 6-membered carbocyclic or heterocyclic group include an aryl group and a 5- or 6-membered saturated heterocyclic group containing one or two heteroatoms as ring member atoms. The heteroatoms used herein can be selected from a nitrogen atom, an oxygen atom, and a sulfur atom, and when there are two heteroatoms, the heteroatoms may be the same or different. Non-limiting examples of the saturated heterocyclic ring include a morpholine ring.

In the present specification, as for the "amino acid", any compound can be used as long as it has both an amino group and a carboxyl group, and the amino acid includes natural and non-natural compounds. The amino acid may be any of a neutral amino acid, a basic amino acid, and an acidic amino acid, it is possible to use amino acids which themselves function as a transmitter such as a neurotransmitter, and amino acids which are constituents of polypeptide compounds such as physiologically active peptides (including dipeptides, tripeptides, tetrapeptides and oligopeptides) or proteins, and the amino acid may be, for example, an α amino acid, a β amino acid, a γ amino acid, or the like. It is preferable to use an optically active amino acid as the amino acid. For example, as the α amino acid, either a D- or a L-amino acid may be used, and it may be preferable to select an optically active amino acid that functions in a living body.

In the present specification, the "amino acid residue" refers to a structure corresponding to the remaining partial structure obtained by removing a hydroxyl group from a carboxyl group of an amino acid.

The amino acid residue includes an α-amino acid residue, a β-amino acid residue, or a γ-amino acid residue.

In the present specification, the "peptide" refers to a structure in which two or more amino acids are linked by a peptide bond.

Examples of a case where R₀ or R¹⁰ together with C=O to which R₀ or R¹⁰ is bonded constitutes a part of an amino acid include a structure in which a carboxyl group of a side chain of an amino acid is bonded to -NH₂ to form a carbonyl group, thereby forming a part of an amino acid, such as the γ-glutamyl group described above.

In one mode of the present invention, the partial structure of the amino acid of R₀ and R¹⁰ is represented by Formula (A) below:

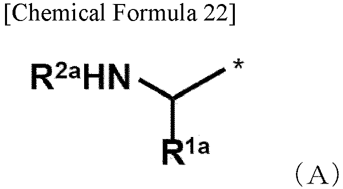

In Formula (A), R^{1a} is selected from the group consisting of a group constituting a side chain of a natural amino acid (glycine, alanine, leucine, isoleucine, valine, lysine, cysteine, threonine, arginine, asparagine, aspartic acid, glutamine, glutamic acid, serine, histidine, phenylalanine, methionine, tryptophan, tyrosine, or proline), a group constituting a side chain of a non-natural amino acid (such as citrulline or norvaline), and a substituted or unsubstituted alkyl group.

In Formula (A), R^{2a} is a hydrogen atom or an N terminal protecting group, for example, an acetyl group (-COCH₃) or succinamide (-CO-C₂H₄-COOH).

* represents a site that is bonded to C=O.

When R₀ or R¹⁰ is represented by Formula (A), R₀ or R¹⁰ together with C=O to which R₀ or R¹⁰ is bonded constitutes an amino acid residue.

When R is represented by Formula (A), the compounds represented by General Formulas (I) and (II) or salts thereof can be used as a fluorescent probe for detection of activity of an aminopeptidase.

In one mode of the present invention, the partial structure of the amino acid of R₀ and R¹⁰ is represented by Formula (B) below:

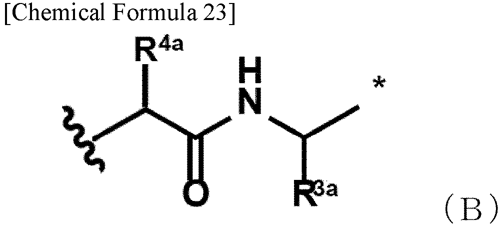

In Formula (B), R^{3a} is selected from the group consisting of a group constituting a side chain of a natural amino acid (glycine, alanine, leucine, isoleucine, valine, lysine, cysteine, threonine, arginine, asparagine, aspartic acid, glutamine, glutamic acid, serine, histidine, phenylalanine, methionine, tryptophan, tyrosine, or proline), a group constituting a side chain of a non-natural amino acid (such as citrulline or norvaline), and a substituted or unsubstituted alkyl group.

In Formula (B), R^{4a} represents a group constituting a side chain of a natural amino acid (glycine, alanine, leucine, isoleucine, valine, lysine, cysteine, threonine, arginine, asparagine, aspartic acid, glutamine, glutamic acid, serine, histidine, phenylalanine, methionine, tryptophan, tyrosine, or proline), such as hydrogen or a methyl group. R^{4a} also includes a group constituting a side chain of a non-natural amino acid (such as citrulline or norvaline). R^{4a} also includes a group other than a group constituting a side chain of a natural amino acid or a non-natural amino acid, for example, an alkyl group having various substituents.

In Formula (B), 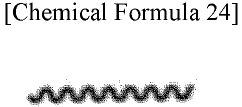 represents a structure in which Formula (B) is bonded to an amino group, an amino group subjected to N-acetylation or the like, a structure in which an amino group and an amino acid (N terminal may be subjected to acetylation or the like) are bonded to each other, or a peptide in which an amino group and a plurality of amino acids are bonded by a peptide bond (N terminal may be subjected to acetylation or the like).
* represents a site that is bonded to C=O.

When R₀ or R¹⁰ is represented by Formula (B), R₀ or R¹⁰ together with C=O to which R₀ or R¹⁰ is bonded constitutes a peptide in which two or more amino acids are linked by a peptide bond.

When R₀ or R¹⁰ is represented by Formula (B), the compound represented by General Formula (I) or (II) or a salt thereof can be used as a fluorescent probe for detection of activity of a peptidase or a protease.

In one mode of the present invention, the partial structure of the amino acid of R₀ and R¹⁰ is represented by Formula (C) below: 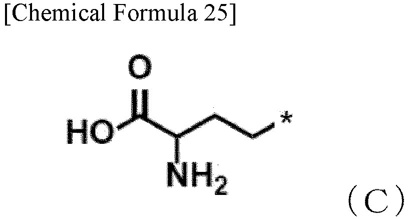 * represents a site that is bonded to C=O.

When R₀ or R¹⁰ is represented by Formula (C), R₀ or R¹⁰ together with C=O to which R₀ or R¹⁰ is bonded forms a γ-glutamyl bond, and in this case, the compounds represented by General Formulas (I) and (II) or salts thereof can be used as a fluorescent probe for detection of activity of γ-glutamyl transpeptidase (GGT).

In one preferred aspect of the present invention, the moieties of NH-CO-R₀ and -NH-CO-R¹⁰ bonded to the benzene ring of the tricyclic structure (particularly xanthene ring) of General Formulas (I) and (II) are represented by any of the following:

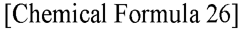

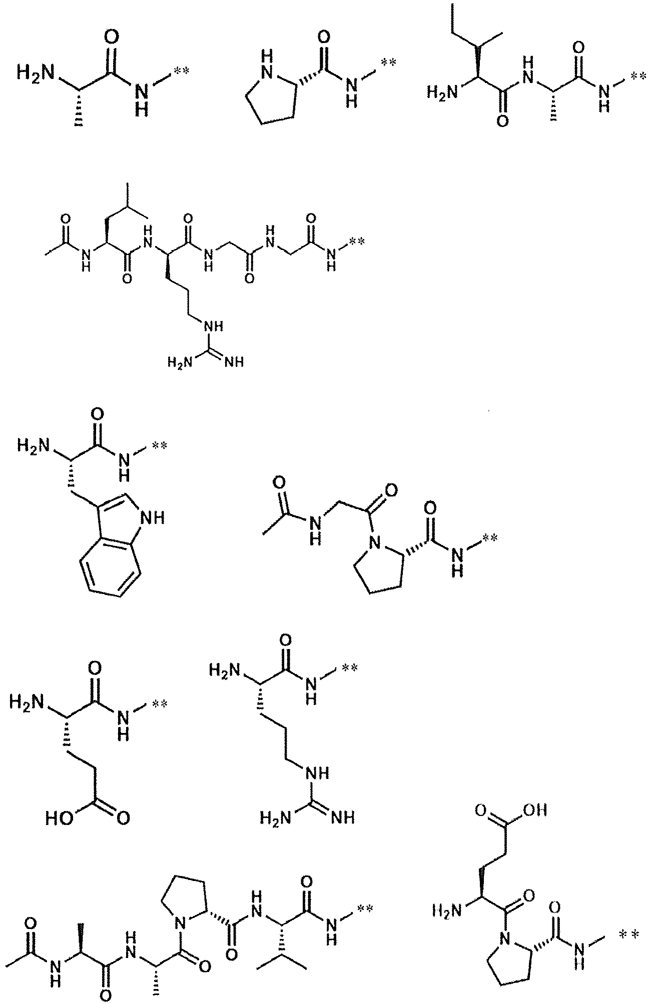

In the above formula, ** represents a site bonded to a benzene ring.

The partial structure of a carboxylic acid defined as R₀ or R¹⁰, together with C=O to which R₀ or R¹⁰ is bonded, constitutes a part of a carboxylic acid. That is, the carboxyl group of the carboxylic acid represented as R^{5a}-COOH allows an amino group bonded to a xanthene skeleton to be bonded to an amide bond to constitutes a composite group of R^{5a}-CO-NH-***. R^{5a} represents a hydrocarbon group which may have a carboxyl group at the terminal, and *** represents a site bonded to a benzene ring of a xanthene skeleton.

The carboxylic acid is preferably a saturated fatty acid, an unsaturated fatty acid, or a dicarboxylic acid.

The saturated fatty acid is preferably acetic acid (CH₃CO₂H), butyric acid (CH₃CH₂CO₂H), palmitic acid (CH₃(CH₂)₁₄CO₂H, or the like.

The unsaturated fatty acid is preferably oleic acid.

The dicarboxylic acid is preferably succinic acid (HO₂C-CH₂-CH₂-CO₂H).

When R₀ or R¹⁰ is the partial structure of a carboxylic acid, the compound represented by General Formula (I) or (II) or a salt thereof can be used as a fluorescent probe for detection of activity of an amidase.

In another aspect of the present invention, as for the partial structure of a carboxylic acid defined as R₀ or R¹⁰, R^{5a} in the composite group of R^{5a}-CO-NH-*** may be a hydrocarbon group having a site to be reduced by an oxidoreductase (*** represents a site bonded to a benzene ring of a xanthene skeleton).

Examples of the site to be reduced by an oxidoreductase include a quinone moiety, and when the quinone moiety is reduced by an oxidoreductase to form phenol, it attacks the amide moiety to cleave the amide, and a fluorescent dye is liberated, so that enzyme activity can be detected. Examples of the compound of the present invention in which such R₀ or R¹⁰ is a partial structure of a carboxylic acid are shown below.

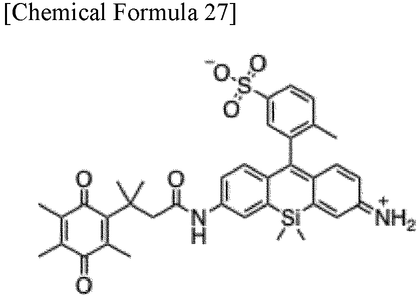

The fluorescent probe containing the above-described compound can detect the activity of the oxidoreductase.

In T-O- defined as R₀ or R¹⁰, T is a saccharide, a partial structure of a saccharide, or a saccharide having a self-cleaving linker, and the partial structure of a saccharide of T, together with O to which T is bonded, constitutes a saccharide or a part of a saccharide.

Examples of the saccharide include β-D-glucose, β-D-galactose, β-L-galactose, β-D-xylose, α-D-mannose, β-D-fucose, α-L-fucose, β-L-fucose, β-D-arabinose, β-L-arabinose, β-D-N-acetylglucosamine, and β-D-N-acetylgalactosamine, and β-D-galactose and β-D-N-acetylglucosamine are preferable.

The self-cleaving linker means a linker that spontaneously cleaves and decomposes, and examples thereof include carbamate, urea, a p-aminobenzyloxy group, and an ester group. The self-cleaving linker is well-known, and can be used in the present invention. Non-limiting examples of the self-cleaving linker include sugar hydrolase probes (J. Am. Chem. Soc. 2013, 135(1), 409-414).

As described above, the compound of the present invention essentially has at least one water-soluble substituent selected from the group consisting of a carboxyl group (-CO₂H), a phosphate group (-PO₃H₂), and a sulfonate group (-SO₃H). As a mode of introducing the water-soluble substituent, the water-soluble substituent can be introduced only into the aromatic ring of A directly or through a linker (Mode A), or the water-soluble substituent can be introduced into the xanthene skeleton directly or through a linker without being introduced into the aromatic ring (that is, n = 0) (Mode B). The water-soluble substituent can be introduced into the aromatic ring of A directly or through a linker and the water-soluble substituent can be introduced into the xanthene skeleton directly or through a linker (Mode C).

In addition to each of the above modes, the water-soluble substituent can be introduced into a cyclohexyl ring or a cycloaryl ring formed through a nitrogen atom bonded to the xanthene skeleton directly or through another substituent (Mode D), and the water-soluble substituent can be introduced only into a cyclohexyl ring or a cycloaryl ring formed through a nitrogen atom bonded to the xanthene skeleton directly or through another substituent (Mode E).

In any cases of Modes A to E, the compound of the present invention has a MiLogP value of less than 2.47 or a LogP value of 1.09 or less, which is an index of lipophilicity. When the MiLogP value is less than 2.47 or the LogP value is 1.09 or less, the microdevice applicability of the compound of the present invention is favorable.

The LogP value is a partition coefficient of octanol/water, represents a ratio of equilibrium concentrations of substances dissolved between two phases of octanol and water, and is an index indicating whether the component is hydrophobic (lipid-soluble) or hydrophilic (water-soluble). In the present invention, the LogP value is a value measured at room temperature (25°C), and the details of the measurement method will be described in Examples.

The LogP value can also be estimated based on the chemical structure. Software and programs for estimating the LogP value are available to those skilled in the art, and in the present invention, for example, the LogP value can be estimated using a program provided by Molinspiration Cheminformatics. The LogP value thus obtained is referred to as a MiLogP value in the present invention.

One preferred mode of the fluorescent probe of the present invention is a fluorescent probe containing at least one compound of compounds represented by General Formulas (Ia) to (Ie) below or a salt thereof.

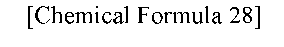

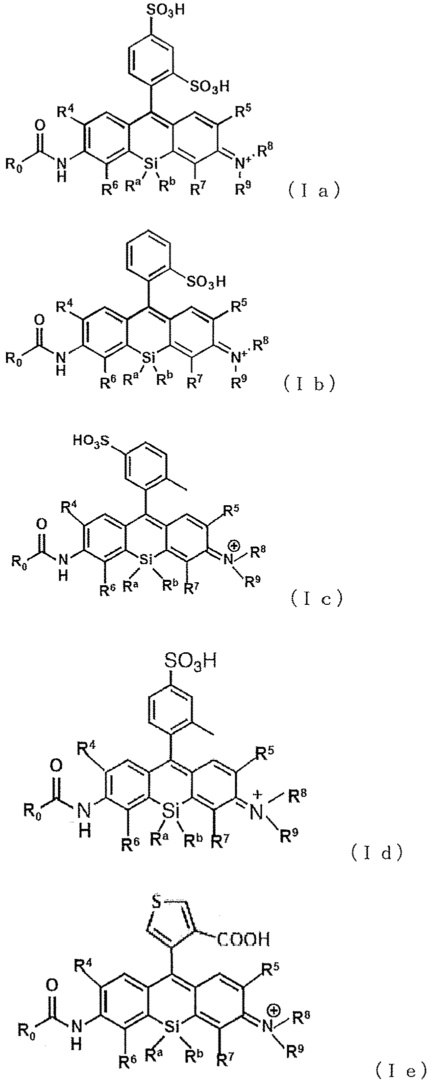

In Formulas (Ia) to (Ie), R⁴ to R⁹, R^{a}, R^{b}, and R₀ are as defined in General Formulas (I) and (II).

Another preferred mode of the fluorescent probe of the present invention is a fluorescent probe containing a compound represented by General Formula (If) below or a salt thereof.

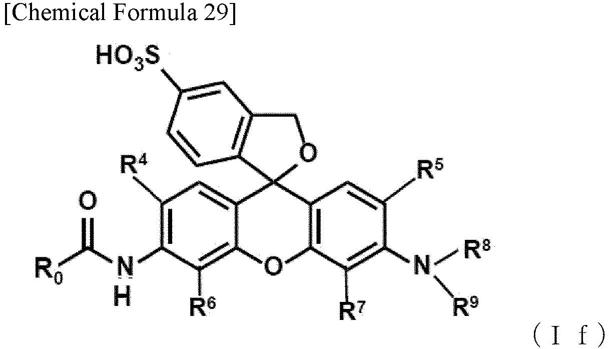

In Formula (If), R⁴ to R⁹ and R₀ are as defined in General Formulas (I) and (II).

The compound of the present invention, unless specifically stated otherwise, also includes stereoisomers thereof such as tautomers, geometric isomers (for example, E-form, Z-form, and the like), and enantiomers. That is, when one or two or more asymmetrical carbons are contained in the compound of the present invention, it can take either an (R)-form or (S)-form, each independently, in accordance with the stereochemistry of asymmetrical carbons, and sometimes exists as an enantiomer or diastereomer or other such stereoisomer of the derivative. Therefore, stereoisomers of a pure form, any mixtures of stereoisomers, racemates, and the like can be used as active ingredients of the fluorescent probe of the present invention, and are all encompassed within the scope of the present invention.

### (2) Fluorescent Probe for Detecting Hydrolase

The fluorescent probe of the present invention is suitably used for detecting a hydrolase.

Examples of the hydrolase include a peptide bond hydrolase and an O-glycosyl compound hydrolase.

The peptide bond hydrolase includes an amidase, an aminopeptidase, a peptidase, and a protease. Examples of the peptide bond hydrolase that can be detected by the fluorescent probe of the present invention include, but are not limited to, aminopeptidase N, γ-glutamyl transpeptidase, dipeptidyl peptidase, elastase, fatty acid amide hydrolase, caspase, acyl amino acid releasing enzyme, fibroblast activation protein, and SUMO specific protease.

Examples of the O-glycosyl compound hydrolase that can be detected by the fluorescent probe of the present invention include, but are not limited to, β-galactosidase and β-GlcNAcase.

There are enzymes exhibiting a plurality of enzyme activities depending on reaction conditions, and even such enzymes are treated as hydrolases in the present invention as long as they have hydrolase activity. For example, a transferase such as γ-glutamyltransferase is a hydrolase because it exhibits hydrolase activity in a reverse reaction.

A method of using the fluorescent probe of the present invention is not particularly limited, and the fluorescent probe can be used in the same manner as a conventionally known fluorescent probe. Usually, the compound of the present invention or a salt thereof may be dissolved in an aqueous medium such as physiological saline or a buffer solution, a mixture of an aqueous medium and a water-miscible organic solvent such as ethanol, acetone, ethylene glycol, dimethyl sulfoxide, or dimethylformamide, or the like, this solution may be added to an appropriate buffer solution containing cells and tissues, and a fluorescence spectrum is measured. The fluorescent probe of the present invention may be used in the form of a composition in combination with an appropriate additive. For example, the fluorescent probe of the present invention can be combined with an additive such as a buffering agent, a solubilizing agent, or a pH adjusting agent.

### (3) Fluorescent Probe for Microdevice

The fluorescent probe of the present invention can be suitably used in an enzyme activity detection method using a microdevice. Therefore, by providing the fluorescent probe of the present invention in a well of a microdevice, the fluorescent probe can be used in a method for detecting enzyme activity of a target hydrolase in a biological sample.

The microdevice includes a microchamber type device, a liposome, a droplet, and the like.

Hereinafter, a microdevice will be described using a microchamber type device as an example.

The material for the microdevice is not particularly limited, and examples thereof include glass materials, silicon, and plastics containing a dendritic polymer or copolymer. Examples of the glass materials include soda lime glass, Pyrex (registered trademark) glass, Vycor (registered trademark) glass, and quartz glass. Examples of the resin polymer include poly(vinyl chloride), poly(vinyl alcohol), poly(methyl methacrylate), poly(vinyl acetate-co-maleic anhydride), poly(dimethylsiloxane) monomethacrylate, a cyclic olefin polymer, a fluorocarbon polymer, polystyrene, polypropylene, and polyethyleneimine. Examples of the copolymer include poly(vinyl acetate-co-maleic anhydride), poly(styrene-co-maleic anhydride), and poly(ethylene-co-acrylic acid) or derivatives thereof.

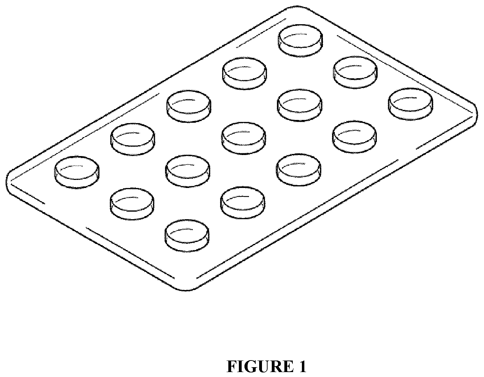

Examples of the shape of the microdevice include a multi-well plate in which an arbitrary number of wells (for example, microwells) are arranged as illustrated in Fig. 1. The number of wells is, for example, 1 or more and 10,000,000 or less, for example, 10 or more and 500,000 or less, for example, about 100,000, or the like, per plate.

The pore diameter of the well of the microdevice may be, for example, 10 nm or more and 10 µm or less, may be, for example, 100 nm or more and 10 µm or less, and may be, for example, 1 µm or more and 10 µm or less.

The depth of the well of the microdevice may be, for example, 10 nm or more and 100 µm or less, may be, for example, 100 nm or more and 80 µm or less, and may be, for example, 200 nm or more and 70 µm or less.

When the pore diameter and depth are within the above ranges, one molecule of the target enzyme can be captured in the well, and the enzyme activity of each molecule of the enzyme in the biological sample can be detected.

The microdevice may include one kind of the above-described fluorescent probe of the present invention in one well.

This makes it possible to detect the fluorescence intensity of one kind of the fluorescent probe of the present invention with respect to one molecule of the target enzyme in the biological sample and to compare the enzyme activities of one molecule of the target enzyme with each other.

The amount of the fluorescent probe of the present invention contained in one well of the microdevice may be, for example, 100 nM or more and 1000 µM or less, may be, for example, 1 µM or more and 1000 µM or less, and may be, for example, 10 µM or more and 1000 µM or less.

As a method of using the microdevice, first, a solution containing a biological sample is added to the microdevice. Sealing oil is then added dropwise to enclose the target enzyme in the biological sample in the wells of the microdevice.

The fluorescent probe of the present invention may be added to the solution containing a biological sample.

### 2. Method for Detecting Enzyme Activity in Biological Sample

Still another implementation of the present invention is a method for detecting activity of a plurality of enzymes in a biological sample using one or more kinds of the compound of the present invention (hereinafter, also referred to as the "detection method 1 of the present invention").

In the detection method 1 of the present invention, a microdevice can be suitably used.

The microdevice used in the detection method 1 of the present invention includes the above-described compound of the present invention.

The material and shape of the microdevice that can be used in the detection method 1 of the present invention are as described in detail for the fluorescent probe of the present invention.

The microdevice may include one kind of the compound of the present invention in one well.

This makes it possible to detect one kind of the fluorescent probe of the present invention with respect to one molecule of the enzyme in the biological sample and to compare the enzyme activities of one molecule of each of the plurality of enzymes and the compound of the present invention.

The amount of the fluorescent probe of the present invention contained in one well of the microdevice may be, for example, 100 nM or more and 1000 µM or less, may be, for example, 1 µM or more and 1000 µM or less, and may be, for example, 10 µM or more and 1000 µM or less.

As a method of using the microdevice, first, a solution containing a biological sample is added to the microdevice. Sealing oil is then added dropwise to enclose the enzyme in the biological sample in the wells of the microdevice. The fluorescent probe of the present invention may be added to the solution containing a biological sample.

According to the present invention, there is provided a method for detecting activity of a plurality of enzymes in a biological sample, the method comprising bringing the biological sample (for example, a biological sample, a biopsy sample, a body fluid sample, and an aqueous solution which are isolated from a subject) into contact with the compound of Formula (I) or the compound of Formula (II). In a certain mode, the biological sample may be a blood sample (for example, a serum sample or a plasma sample). This method may further include measuring the fluorescence of the compound after contacting the compound and the aqueous solution. When the compound emits fluorescence, the presence or absence of the fluorescence indicates the presence of the enzyme in the aqueous solution, and the intensity of the fluorescence indicates the intensity of the activity of the enzyme in the aqueous solution.

One mode of the detection method 1 of the present invention is a method for detecting peptide bond hydrolase activity in a biological sample, the method comprising bringing the compound of the present invention into contact with a plurality of enzymes.

In one preferred aspect of the detection method 1 of the present invention, contacting is performed in the presence of serum.

According to the detection method 1 of the present invention, enzyme activity of a plurality of enzymes in a biological sample can be detected.

The detection method 1 of the present invention will be described in detail below.

### [Step 1]

First, a solution containing a biological sample is added to a microdevice including the compound of the present invention or the compound group (library) of the present invention. Examples of the biological sample include a biological sample, a biopsy sample, a body fluid sample, and an aqueous solution which are isolated from a subject. The biological sample may be a blood sample (for example, a serum sample or a plasma sample).

As the pH of the solution containing a biological sample, any value may be selected. For example, the pH of the solution can be set to a value close to that in the living body, and in this case, the pH may be, for example, 6.0 or more and 8.0 or less. In order to narrow down the target to a specific enzyme group, the pH of the solution can also be made acidic so as to reach an optimum pH.

A solution containing a biological sample and a solution containing the fluorescent probe of the present invention may be separately prepared, the ratio of the biological sample and the fluorescent probe may be appropriately adjusted and mixed, and the mixed solution may be added to the microdevice.

The protein concentration of the biological sample may be usually, for example, 1 pM or more and 100 pM or less, and may be, for example, 10 pM or more and 100 pM or less, as the concentration of a "target protein".

The upper limit of the total protein concentration in the sample may be, for example, about 10 µM.

As a method for measuring the protein concentration of a biological sample, for example, a method using an antibody antigen reaction (for example, ELISA method or the like) can be used as a method for measuring the concentration of a "target protein". Examples of a method for measuring the total protein concentration in a sample include colorimetric methods using a reaction between a protein and a reagent (such as a bicinchoninic acid (BCA) method, Bradford method, Lowry method, and a biuret method).

The biological sample may be diluted using various aqueous solvents or the like so as to have the above concentration. Examples of the aqueous solvent include water, physiological saline, phosphate buffered saline (PBS), tris buffered saline (TBS), and HEPES buffered saline, but are not limited thereto.

### [Step 2]

Sealing oil is then added dropwise to enclose the enzyme in the biological sample in the wells of the microdevice. The sealing oil may be any known sealing oil that is usually used for enclosing a sample in a microdevice, and examples thereof include fluorine-based oil (such as FC-40) and aliphatic hydrocarbon (such as hexadecane).

### [Step 3]

Next, fluorescence in the well of the microdevice is detected using a fluorescence scanner, a fluorescence microscope, or the like. The enzyme activity can be evaluated from the detected fluorescence intensity.

What enzyme is contained in the well of the microdevice in which the enzyme activity is detected can be determined, for example, by comparison with an enzyme activity pattern with a separately prepared target protein.

The detection method 1 of the present invention can further include observing a fluorescence response using a fluorescence imaging means. As a means for observing a fluorescence response, a fluorometer having a wide measurement wavelength can be used, but the fluorescence response can also be visualized using a fluorescence imaging means capable of displaying the fluorescence response as a two-dimensional image. By using the fluorescence imaging means, the fluorescence response can be two-dimensionally visualized, so that the target enzyme can be instantly visually recognized. As a fluorescence imaging device, a device known in the art can be used. In some cases, it is also possible to detect a reaction between the sample to be measured and the fluorescent probe by a change in ultraviolet-visible absorption spectrum (for example, a change in absorbance at a specific absorption wavelength).

In one mode of the detection method 1 of the present invention, by bringing one kind of the fluorescent probe of the present invention into contact with a plurality of enzymes in a biological sample, the fluorescence intensity of one kind of the fluorescent probe of the present invention can be detected with respect to one molecule of the plurality of enzymes in the biological sample, and the enzyme activities of one molecule of the plurality of enzymes can be compared. In the step 1, the enzyme activity of one molecule of enzyme can be measured by preparing a well (for example, a plurality of wells) containing one molecule of enzyme and a plurality of fluorescent probes.

### 3. Detection of Biomarker

The fluorescent probe of the present invention can be used as a fluorescent probe for detection of one or more kinds of hydrolase activity depending on the kind of the functional group introduced as R₀ or R¹⁰, and the fluorescent probe of the present invention can be further used for detection of a biomarker specific to a predetermined disease to diagnose the disease.

That is, still another implementation of the present invention is a method for diagnosing a disease state by detecting single-molecule hydrolase activity using the fluorescent probe of the present invention (hereinafter, also referred to as the "diagnostic method of the present invention").

Non-limiting examples of the diagnostic method of the present invention are shown below.

That is, still another implementation of the present invention is a method for detecting activity of CD13 in a biological sample, the method comprising bringing the compound of the present invention (hereinafter, also referred to as "Compound A of the present invention"), in which -CO-R₀ or -CO-R¹⁰ is an alanine, lysine, arginine, or methionine residue in General Formula (I) or (II), into contact with a biological sample in an aqueous solution, an increase in fluorescence intensity in the aqueous solution indicating the presence of activity of CD13.

Still another implementation of the present invention is a method for diagnosing pancreatic cancer (hereinafter, also referred to as the "diagnostic method 1 of the present invention"), the method comprising detecting, by a microdevice, single-molecule enzyme activity of CD13 in a biological sample obtained from a subject, using a fluorescent probe containing the compound of the present invention, in which -CO-R₀ or -CO-R¹⁰ is an alanine, lysine, arginine, or methionine residue in General Formula (I) or (II).

Still another mode of the present invention is a method for diagnosing pancreatic cancer or a method for predicting a possibility that a subject from which a biological sample is derived has pancreatic cancer (hereinafter, also referred to as the "diagnostic or prediction method 1 of the present invention"), the method comprising (a) bringing, by a microdevice, a biological sample obtained from a subject into contact with a fluorescent probe containing the compound of the present invention, in which - CO-R₀ or -CO-R¹⁰ is an alanine, lysine, arginine, or methionine residue in General Formula (I) or (II), and (b) measuring a fluorescence intensity of the biological sample brought into contact with the fluorescent probe.

The diagnostic method 1 of the present invention and the diagnostic or prediction method 1 of the present invention are based on the detection of the enzyme activity of one molecule of CD13 by Compound A of the present invention.

Still another implementation of the present invention is a method for detecting activity of DPP4 in a biological sample, the method comprising bringing the compound of the present invention (hereinafter, also referred to as "Compound B of the present invention"), in which CO-R₀ or -CO-R¹⁰ is -Pro-Xaa (Pro represents a proline residue and Xaa represents an amino acid residue such as glycine, serine, or glutamic acid) in General Formula (I) or (II), into contact with a biological sample in an aqueous solution, an increase in fluorescence intensity in the aqueous solution indicating the presence of activity of DPP4.

Still another implementation of the present invention is a method for diagnosing pancreatic cancer (hereinafter, also referred to as the "diagnostic method 2 of the present invention"), the method comprising detecting, by a microdevice, single-molecule enzyme activity of DPP4 in a biological sample obtained from a subject, using a fluorescent probe containing the compound of the present invention, in which -CO-R₀ or -CO-R¹⁰ is -Pro-Xaa (Pro represents a proline residue and Xaa represents an amino acid residue such as glycine, serine, or glutamic acid) in General Formula (I) or (II).

Another mode of the present invention is a method for diagnosing pancreatic cancer or a method for predicting a possibility that a subject from which a biological sample is derived has pancreatic cancer (hereinafter, also referred to as the "diagnostic or prediction method 2 of the present invention"), the method comprising (a) bringing, by a microdevice, a biological sample obtained from a subject into contact with a fluorescent probe containing the compound of the present invention, in which -CO-R₀ or -CO-R¹⁰ is -Pro-Xaa (Pro represents a proline residue and Xaa represents an amino acid residue such as glycine, serine, or glutamic acid) in General Formula (I) or (II), and (b) measuring a fluorescence intensity of the biological sample brought into contact with the fluorescent probe.

The diagnostic method 2 of the present invention and the diagnostic or prediction method 2 of the present invention are based on the detection of the enzyme activity of one molecule of DPP4 by Compound B of the present invention.

Still another mode of the present invention is a method for diagnosing pancreatic cancer, the method comprising: detecting, by a microdevice, single-molecule enzyme activity of CD13 in a biological sample obtained from a subject, using a fluorescent probe containing the compound of the present invention, in which -CO-R₀ or -CO-R¹⁰ is an alanine, lysine, arginine, or methionine residue in General Formula (I) or (II); and
detecting, by a microdevice, single-molecule enzyme activity of DPP4 in a biological sample obtained from a subject, using a fluorescent probe containing the compound of the present invention, in which -CO-R₀ or -CO-R¹⁰ is -Pro-Xaa (Pro represents a proline residue and Xaa represents an amino acid residue such as glycine, serine, or glutamic acid) in General Formula (I) or (II).

The diagnostic method 1 (or 2) of the present invention and the diagnostic or prediction method 1 (or 2) of the present invention can be performed by preparing a solution containing a biological sample and Compound A (or B) of the present invention, adding the solution to a microdevice, enclosing the added solution in each well, incubating the microdevice in which the solution is enclosed, and then measuring the fluorescence intensity of each well with a fluorescence microscope.

Specifically, the diagnostic method 1 of the present invention and the diagnostic or prediction method 1 of the present invention are performed, for example, as follows, but are not limited thereto.

Compound A of the present invention is diluted with an assay buffer to a predetermined concentration (for example, 100 µM). The composition of the assay buffer can be appropriately determined, and can be, for example, 100 mM HEPES-NaOH (pH 7.4), 1 mM MgCl₂, 1 mM CaCl₂, 3 mM Triton X-100.

Next, a biological sample, preferably a blood sample (for example, a serum sample or a plasma sample) collected from a subject (including both of a patient with pancreatic cancer and a healthy subject) is diluted with the above assay buffer (for example, about 10,000 times), and mixed with a diluted solution of Compound A of the present invention at a predetermined ratio (for example, equal amount).

The mixed solution of both was added to a microdevice, followed by the addition of sealing oil to enclose the solution in each well.

The microdevice in which the solution is enclosed is incubated under predetermined conditions (for example, 37°C for 2 hours), and then the fluorescence intensity of each well is measured with a fluorescence microscope.

The number of wells in which the detected fluorescence intensity is equal to or higher than a predetermined intensity (for example, 2,500 AU or more), that is, the number of wells in which CD13 is considered to be enclosed is measured.

In order to diagnose that the subject from which the biological sample is derived is pancreatic cancer and predict a possibility of pancreatic cancer from the number of wells in which CD13 is considered to be enclosed as measured above, an ROC curve for determination based on the result of CD13 activity measurement using Compound A of the present invention is created, and the possibility of pancreatic cancer is determined by the ROC curve.

Specifically, the diagnostic method 2 of the present invention and the diagnostic or prediction method 2 of the present invention are performed, for example, as follows, but are not limited thereto.

Compound B of the present invention is diluted with an assay buffer to a predetermined concentration (for example, 100 µM). The composition of the assay buffer can be appropriately determined, and can be 100 mM HEPES-NaOH (pH 7.4), 1 mM MgCl₂, 1 mM CaCl₂, 3 mM Triton X-100.

Next, a biological sample, preferably a blood sample (for example, a serum sample or a plasma sample) collected from a subject (including both of a patient with pancreatic cancer and a healthy subject) is diluted with the above assay buffer (for example, about 2,500 times), and mixed with a diluted solution of Compound B of the present invention at a predetermined ratio (for example, equal amount).

The mixed solution of both was added to a microdevice, followed by the addition of sealing oil to enclose the solution in each well.

The microdevice in which the solution is enclosed is incubated under predetermined conditions (for example, 25°C for 2 hours), and then the fluorescence intensity of each well is measured with a fluorescence microscope.

The histogram created based on the fluorescence intensity of each well is approximated to a curve including two normal distributions. A well having a fluorescence intensity higher than the minimum value of the approximate curve is defined as a high activity group, a well having a fluorescence intensity lower than the minimum value of the approximate curve is defined as a low activity group, and the ratio of the high activity group to the total of these groups is measured.

In order to diagnose that the subject from which the biological sample is derived is pancreatic cancer and predict a possibility of pancreatic cancer from the number of wells in which DPP4 is considered to be enclosed as measured above, an ROC curve for determination based on the result of DPP4 activity measurement using Compound B of the present invention is created, and the possibility of pancreatic cancer is determined by the ROC curve.

Non-limiting examples of Compound A of the present invention include the following compounds.

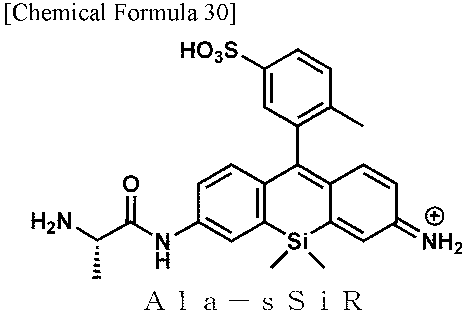

Non-limiting examples of Compound B of the present invention include the following compounds.

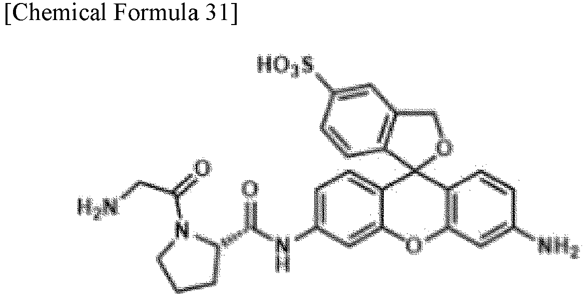

### 4. Compounds 2 and 3 of Present Invention

Still another implementation of the present invention is a compound represented by any one of Formulas (1) to (5) below or a salt thereof (hereinafter, also referred to as "Compound 2 of the present invention").

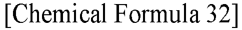

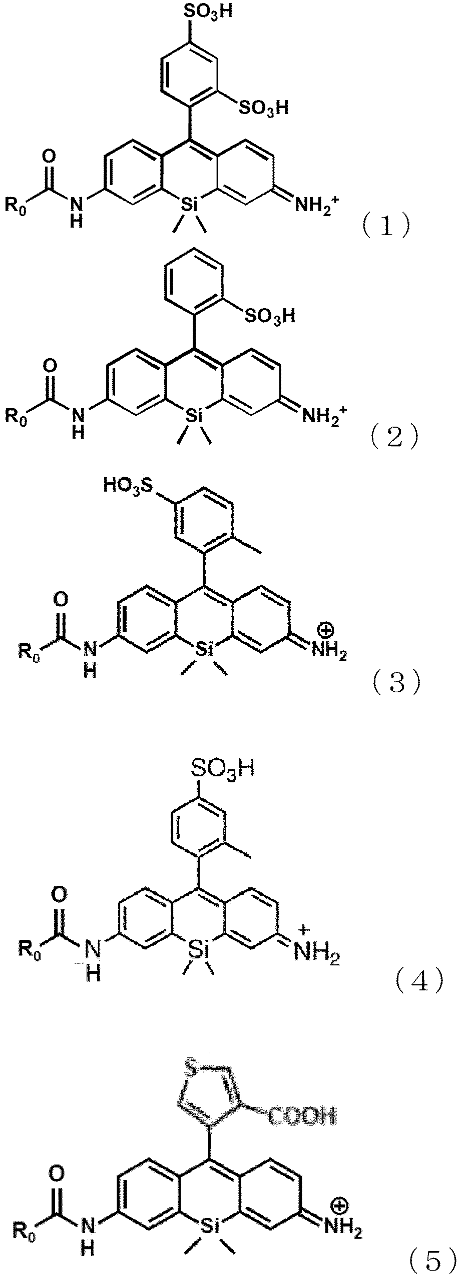

In Formulas (1) to (5), R₀ is selected from a partial structure of an amino acid, a partial structure of a carboxylic acid, or T-O-.

The partial structure of an amino acid, together with C=O to which R₀ is bonded, constitutes an amino acid, an amino acid residue, a peptide, or a part of an amino acid.

The partial structure of a carboxylic acid, together with C=O to which R₀ is bonded, constitutes a part of a carboxylic acid.

T is a saccharide, a partial structure of a saccharide, or a saccharide having a self-cleaving linker, and the partial structure of a saccharide of T, together with O to which T is bonded, constitutes a saccharide or a part of a saccharide.

The details of the partial structure of an amino acid, the partial structure of a carboxylic acid, and T-O- are as described in detail for General Formulas (I) and (II).

Still another implementation of the present invention is a compound represented by Formula (6) below or a salt thereof (hereinafter, also referred to as "Compound 3 of the present invention").

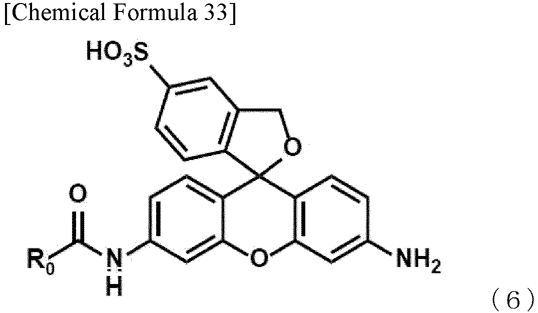

In Formula (6), R₀ is selected from a partial structure of an amino acid, a partial structure of a carboxylic acid, or T-O-.

The partial structure of an amino acid, together with C=O to which R₀ is bonded, constitutes an amino acid, an amino acid residue, a peptide, or a part of an amino acid.

The partial structure of a carboxylic acid, together with C=O to which R₀ is bonded, constitutes a part of a carboxylic acid.

T is a saccharide, a partial structure of a saccharide, or a saccharide having a self-cleaving linker, and the partial structure of a saccharide of T, together with O to which T is bonded, constitutes a saccharide or a part of a saccharide.

The details of the partial structure of an amino acid, the partial structure of a carboxylic acid, and T-O- are as described in detail for General Formulas (I) and (II).

Specific examples of Compound 2 of the present invention are given below, but are not limited thereto.

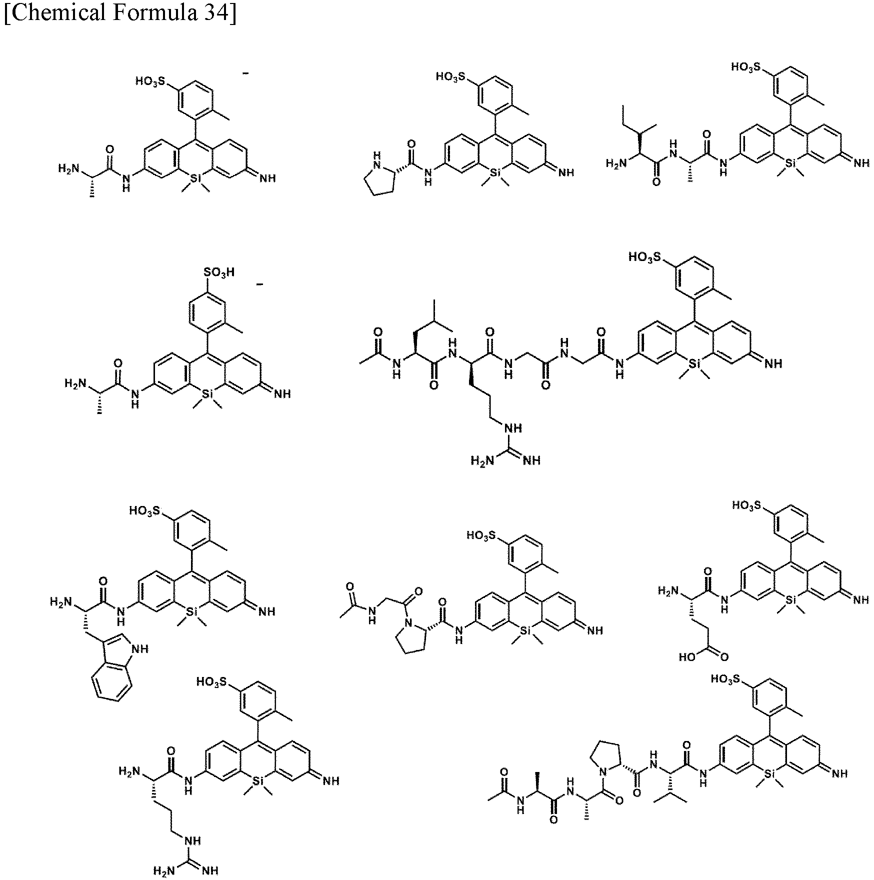

Specific examples of Compound 3 of the present invention are given below, but are not limited thereto.

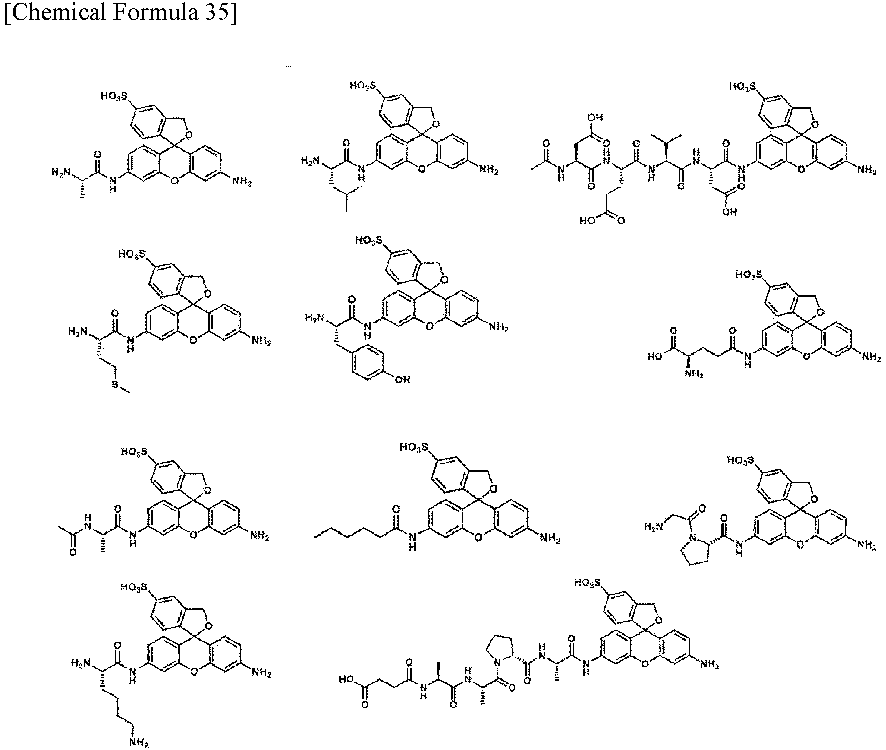

The compounds represented by Formulas (1) to (6), unless specifically stated otherwise, also include their stereoisomers such as tautomers, geometric isomers (for example, E-form, Z-form, and the like), and enantiomers. That is, when one or two or more asymmetrical carbons are contained in the compounds represented by Formulas (1) to (6), it can take either an (R)-form or (S)-form, each independently, in accordance with the stereochemistry of asymmetrical carbons, and sometimes exists as an enantiomer or diastereomer or other such stereoisomer of the derivative.

The method for producing the compounds represented by Formulas (1) to (6) is not particularly limited, but synthesis methods for typical compounds among the compounds included in Formulas (1) to (6) were specifically shown in Examples of the present specification. Those skilled in the art can produce the compounds included in Formulas (1) to (6) by suitably changing or modifying the starting raw materials, reaction reagents, reaction conditions, and the like as necessary while referring to Examples and the following schemes of the present specification.

### 5. Intermediate

A compound represented by General Formula (III) or a salt thereof is useful, for example, as an intermediate for producing the compound of the present invention. That is, according to the present invention, there is provided use of the compound represented by General Formula (III) or a salt thereof, for producing the compound of the present invention.

A compound represented by General Formula (IV) or a salt thereof is useful, for example, as an intermediate for producing the compound of the present invention. That is, according to the present invention, there is provided use of the compound represented by General Formula (IV) or a salt thereof, for producing the compound of the present invention.

According to the present invention, there is also provided a compound represented by General Formula (V) or a salt thereof. This compound is useful, for example, as an intermediate for producing the compound of the present invention. That is, according to the present invention, there is provided use of the compound represented by General Formula (V) or a salt thereof, for producing the compound of the present invention.

In General Formula (V), A preferably represents phenyl or thiophenyl. R preferably represents -COOH or -SO₃H. When A is a phenyl group, R preferably represents -SO₃H. When A is a thiophenyl group, R preferably represents -COOH.

In General Formula (V), n is preferably 1. R⁸ and R⁹ preferably represent a hydrogen atom. X preferably represents an oxygen atom or Si(-CH₃)₂.

More preferred examples of the compound represented by General Formula (V) or a salt thereof include a compound in which A represents phenyl or thiophenyl, R represents -COOH or -SO₃H, n is 1, R⁸ and R⁹ represent a hydrogen atom, X represents an oxygen atom or Si(-CH₃)₂, or a salt thereof. Particularly preferred examples of the compound represented by General Formula (V) or a salt thereof include a compound in which A represents phenyl or thiophenyl, R represents -COOH or -SO₃H, n is 1, R⁸ and R⁹ represent a hydrogen atom, X represents an oxygen atom or Si(-CH₃)₂, when A is a phenyl group, R represents -SO₃H, and when A is a thiophenyl group, R represents - COOH, or a salt thereof.

### 6. Method of Selecting Fluorescent Probe

According to the present invention, there is provided a method of selecting a fluorescent probe excellent in suitability from the fluorescent probe of the present invention. Examples of an index for evaluating the suitability of the fluorescent probe include the above (A), (B), and (C). That is, the selection method of the present invention includes at least preparing one or more fluorescent probes of the present invention and selecting a fluorescent probe satisfying the conditions (A), (B), and (C). Since (A), (B), and (C) are indices for evaluating the suitability in a microdevice, a fluorescent probe selected by the selection method of the present invention can be particularly preferably used as a fluorescent probe for a microdevice.

In (A), a solubility measurement test (25°C) in sealing oil is performed, and a case where the solubility of a test compound in hexadecane is 0.4 µM or less or 0.3 µM or less, preferably 0.2 µM or less, can be determined that (A) is satisfied. The solubility measurement test in sealing oil can be performed by measuring the concentration of the test compound transferred to hexadecane when 1 µL of a 10 mM DMSO solution of the test compound is added with respect to 100 µL of hexadecane as sealing oil, and the mixture is sonicated and suspended at room temperature (25°C) for 1 minute. The solubility measurement test in sealing oil of (A) can be performed specifically according to Example 19.

In (B), a fluorescence intensity measurement test in a microdevice is performed, and a case where a ratio of a fluorescence intensity of a well edge region to a fluorescence intensity of a well center region is 0.7 or less or 0.6 or less, preferably 0.5 or less, can be determined that (B) is satisfied. The fluorescence intensity measurement in a microdevice can be performed by adding a test compound to a microdevice, photographing an image of a well in which the compound is encapsulated with a microscope, and measuring a ratio of the fluorescence intensity of the edge region to the fluorescence intensity of the center region. When the radius of the well is regarded as 1, the center region and the edge region can be specified by the following ratio. Center region: region from the center to a radius of 0.48 to 0.63 (Region 1), preferably region from the center to a radius of 0.53 to 0.58 (Region 2). Edge region: region of the entire well excluding Region 1, preferably region of the entire well excluding Region 2. The fluorescence intensity measurement test in a microdevice of (B) can be performed specifically according to Example 20.

In (C), the enzymolysis test of the test compound in a microdevice and a cuvette is performed to calculate a signal/noise ratio, and a case where a ratio of a signal/noise ratio (first ratio) of the enzymolysate in the microdevice to a signal/noise ratio (second ratio) of the enzymolysate in the cuvette is 0.2 or more, 0.3 or more, or 0.4 or more, preferably 0.5 or more, can be determined that (C) is satisfied. In the calculation of the signal/noise ratio, the fluorescence spectra before and after the enzyme reaction are compared, and the ratio of the fluorescence intensity at a wavelength at which the fluorescence intensity is maximized can be calculated as the signal/noise ratio of the enzymolysate. The measurement and calculation of the ratio of the signal/noise ratios in the microdevice of (C) and the cuvette can be performed specifically according to Example 21.

In the present invention, among the compound of the present invention, and the compound of General Formula (III) and a salt thereof, those satisfying at least one, at least two, or all of (A), (B), and (C) can be preferably provided.

### EXAMPLES

Hereinafter, the present invention will be described with reference to Examples; however, the present invention is not limited thereto.

### [Sample and Measurement Method]

Reagents and solvents for organic synthesis were supplied from Tokyo Chemical Industry Co., Ltd. (TCI), Wako Pure Chemical Industries, Ltd., or Aldrich Chemical Company, and were used without further purification.

Proton nuclear magnetic resonance (¹H NMR) spectra were recorded with JEOL JMN-LA400 instrument.

Mass spectra were measured with JEOL JMS-T100LP AccuTOF^{™} LC-plus4G.

### [Synthesis Example 1]

### Synthesis Example of sHMRG

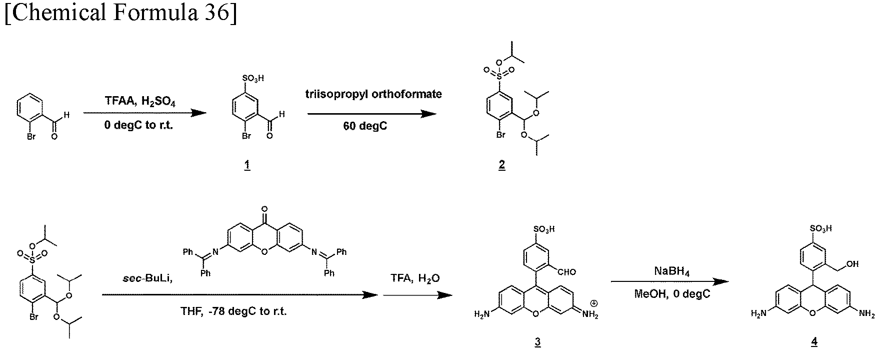

### (1) Synthesis of Compound 1

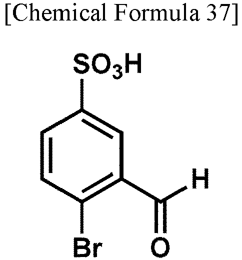

In a dried flask, 2.88 mL (54.1 mmol) of concentrated sulfuric acid was placed, and 15.7 mL (113.6 mmol) of trifluoroacetic anhydride was slowly added thereto at 0°C under an argon atmosphere. Thereafter, the temperature was returned to room temperature, and the mixture was stirred for 3 hours. When the solution became uniform, 6.25 mL (54.1 mmol) of 2-bromo-benzaldehyde was then added portionwise at 0°C, the temperature was returned to room temperature, and the mixture was stirred for 17 hours. Thereafter, water was added at 0°C. Subsequently, TFAA and water were distilled off under reduced pressure. The residue was then purified by MPLC (eluent: A/B = 95/5 > 5/95, A: 0.1% TFA-containing 100% H₂O, B: 0.1% TFA-containing 100% CH₃CN). The eluent was evaporated, and then the product was freeze-dried to obtain Compound 1 (982 mg, 3.71 mmol, yield: 6.8%).

Analysis results of the obtained compound by ¹H-NMR and ¹³C-NMR are shown below.

¹H-NMR (400 MHz, DMSO-D6) δ 10.17 (s, 1H), 7.98 (m, 1H), 7.74 (m, 2H).

¹³C-NMR (100 MHz, CD₃OD) δ 192.0, 148.9, 134.3, 133.2, 133.1, 127.4, 126.1.

HRMS (ESI⁻): Calcd. for [M-H]⁻, 262.90137, Found, 262.89719 (-4.17 mmu).

### (2) Synthesis of Compound 2

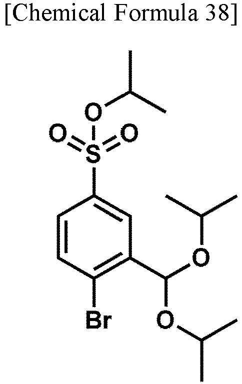

In a dried flask, 338 mg (1.28 mmol) of Compound 1 dissolved in 2.8 mL (12.8 mmol) of triisopropyl orthoformate was added, and the mixture was stirred at 60°C for 1 hour. Subsequently, the solvent was distilled off under reduced pressure. Subsequently, the obtained solid was purified by column chromatography (silica; ethyl acetate/hexane = 0/100 > 20/80), and the eluent was evaporated to obtain 357 mg of Compound 2 (0.87 mmol, yield: 68%).

Analysis results of the obtained compound by ¹H-NMR, ¹³C-NMR, and a high-resolution mass spectrometer (HR-MS) are shown below.

¹H-NMR (400 MHz, CDCl₃) δ 8.22 (m, 1H), 7.68 (m, 2H), 4.76 (sep, J = 5.9 Hz, 1H), 3.89 (sep, J = 6.2 Hz, 2H), 1.28 (d, J = 5.9 Hz, 6H), 1.18 (dd, J = 13.0 Hz, 6.2 Hz, 12H)
¹³C-NMR (100 MHz, CDCl₃) δ 141.2, 137.0, 133.7, 128.7, 128.5, 128.1, 97.9, 69.3, 23.0, 22.9, 22.5.
HRMS (ESI⁺): Calcd. for [M+Na]⁺, 431.05038, Found, 431.04944 (-0.94 mmu).

### (3) Synthesis of Compound 3

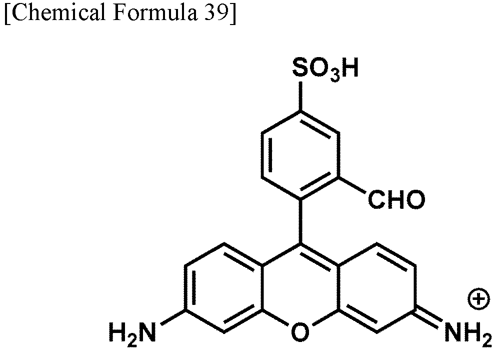

In a dried flask, 213 mg (0.52 mmol) of Compound 2 was placed, dissolved in dehydrated tetrahydrofuran, and stirred at -78°C for 30 minutes under an argon atmosphere. Subsequently, 0.4 mL (0.52 mmol) of 1.3 M secondary butyl lithium was added portionwise thereto, and the mixture was stirred at -78°C for 30 minutes under an argon atmosphere. Subsequently, 45 mg (0.081 mmol) of 3,6-bis(diphenylmethyleneamino)xanthone dissolved in dehydrated tetrahydrofuran was further added portionwise to the reaction solution, and then the mixture was stirred at room temperature for 1.5 hours under an argon atmosphere. TFA and water were then added to the reaction solution, and the mixture was stirred at room temperature for 3.5 hours. Subsequently, the solvent was distilled off under reduced pressure. Subsequently, the obtained solid was purified by MPLC (eluent: A/B = 95/5 > 5/95, A: 0.1% TFA-containing 100% H₂O, B: 0.1% TFA-containing 100% CH₃CN), the eluent was evaporated, and then the product was freeze-dried to obtain 9 mg of Compound 3 (0.023 mmol, yield: 28%).

### (4) Synthesis of Compound 4

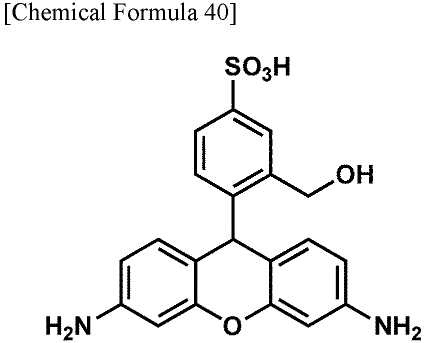

In a dried flask, 130 mg (0.33 mmol) of Compound 3 was placed, dissolved in methanol, and cooled to 0°C, 90 mg (2.38 mmol) of sodium borohydride was then added portionwise, and the mixture was stirred for 30 minutes. Subsequently, the solvent was distilled off under reduced pressure, and then the obtained solid was purified by MPLC (eluent: A/B = 95/5 > 5/95, A: 0.1 M TEAA (triethylamine acetate)-containing 100% H₂O, B: 0.1 M TEAA-containing 100% CH₃CN) and desalted with Sep-Pak C18 (Waters). The eluent was evaporated, and then the product was freeze-dried to obtain 68 mg of Compound 4 (0.17 mmol, yield: 52%).

Analysis results of the obtained compound by ¹H-NMR, ¹³C-NMR, and a high-resolution mass spectrometer (HR-MS) are shown below.

¹H-NMR (400 MHz, CD₃OD) δ 7.91 (d, J = 1.8 Hz, 1H), 7.60 (dd, J = 8.0, 2.1 Hz, 1H), 7.13 (d, J = 7.8 Hz, 1H), 6.62 (d, J = 8.2 Hz, 2H), 6.39 (d, J = 2.3 Hz, 2H), 6.29 (dd, J = 8.2, 2.3 Hz, 2H), 5.42 (s, 1H), 4.61 (s, 2H)
¹³C-NMR (100 MHz, CD₃OD) δ 151.4, 147.5, 142.7, 138.8, 130.5, 129.8, 125.1, 124.7, 121.2, 113.2, 110.9, 102.0, 46.5, 7.9
HRMS (ESI⁺): Calcd. for [M+H]⁺, 399.10147m Found, 399.09779 (-3.68 mmu).

### [Synthesis Example 2]

### Synthesis Example of sHMRG-Based Peptidase and Protease Probes

sHMRG-based peptidase and protease probes (PB101 to PB111) shown in Table 1 below were synthesized by the following procedures using a compound (Compound 4) obtained by reducing sHMRG.

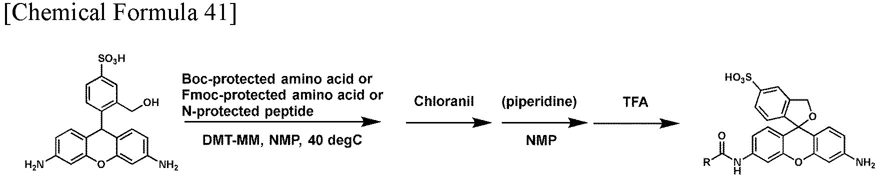

1. Compound 4 (11 µmol), DMT-MM (3 µmol), and Boc-protected amino acid or Fmoc-protected amino acid, or N-terminal protected peptide (5 µmol) were mixed in a 1.5 mL test tube, and the mixture was dissolved in 30 µL of NMP.
2. The reaction mixture was stirred at 40°C.
3. Chloranil (23 µmol) was added and the reaction mixture was stirred.
4. The solution was purified by MPLC (eluent: A/B = 95/5 > 5/95, A: 0.1% TFA-containing 100% H₂O, B: 0.1% TFA-containing 100% CH₃CN), the eluent was evaporated, and then the product was freeze-dried.
5. When the raw material was Fmoc-protected, 50 µL of piperidine and 50 µL of NMP were added to the obtained solid, and the reaction mixture solution was stirred. When the raw material was not Fmoc-protected, this step was not performed and the process proceeded to Step 6.
6. 1000 µL of TFA was added and the reaction mixture was stirred.
7. The solution was purified by MPLC (eluent: A/B = 95/5 > 5/95, A: 0.1% TFA-containing 100% H₂O, B: 0.1% TFA-containing 100% CH₃CN), the eluent was evaporated, and then the product was freeze-dried to obtain a compound of interest.

### [Table 1]

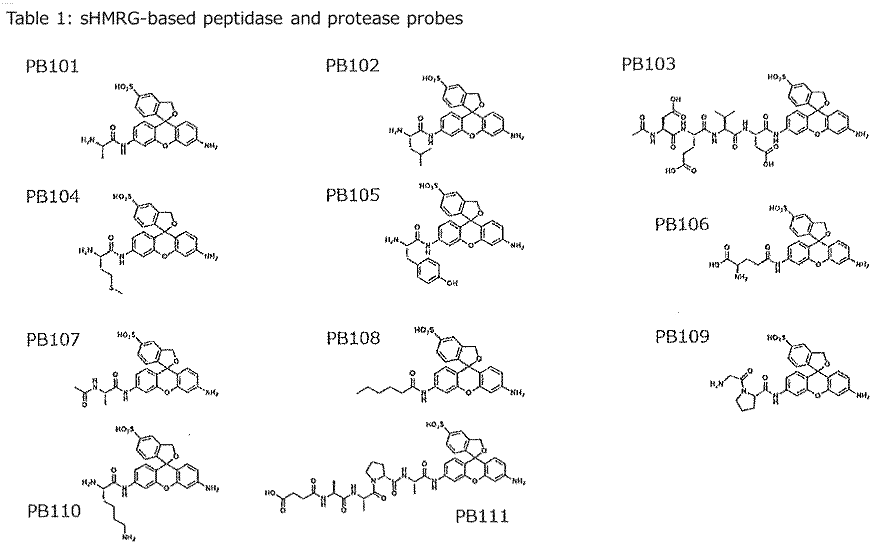

The instrumental data of the compound are as follows.
HPLC condition: A/B = 100/0 > 0/100 (5 min), A = 0.1% Formic acid H₂O, B = 0.1%

Formic acid CH₃CN (A-sHMRG, M-sHMRG: a mixture of diastereomers)

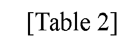

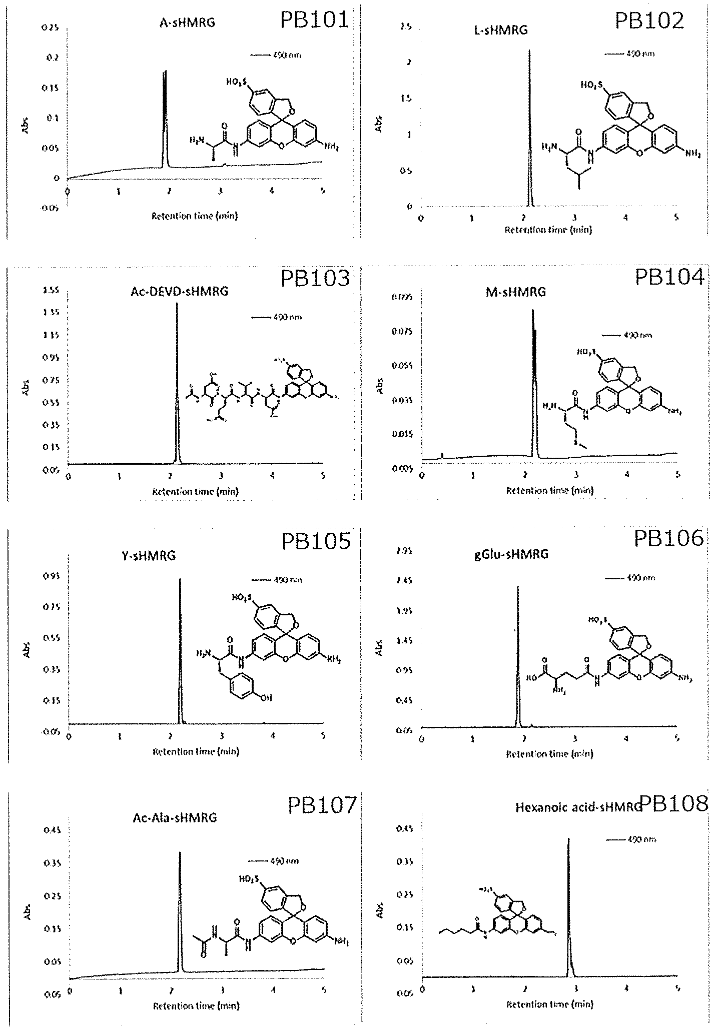

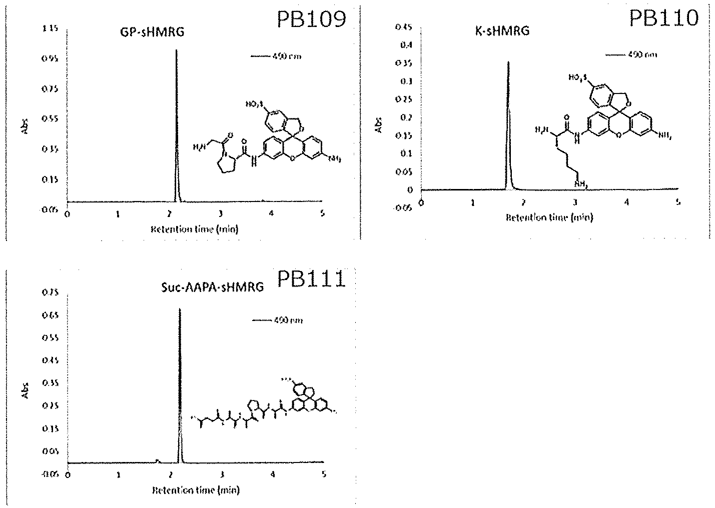

### [Synthesis Example 3]

### Synthesis Example 1 of sSiR

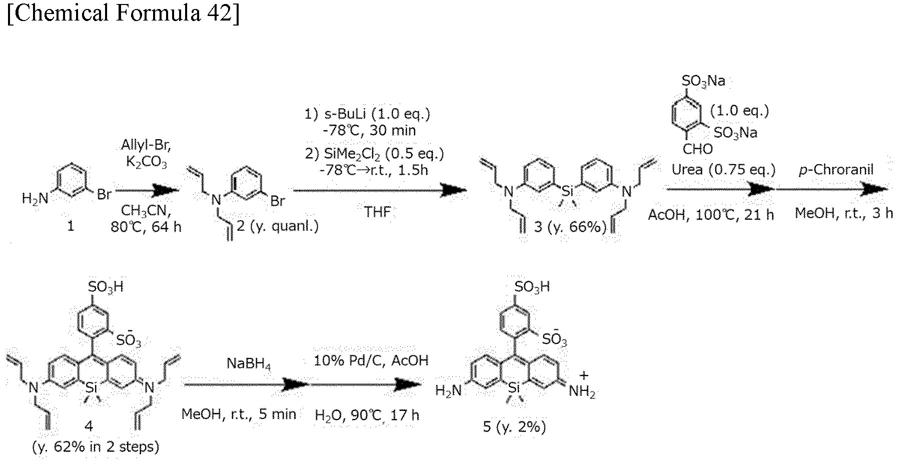

### (1) Synthesis of Compound 2

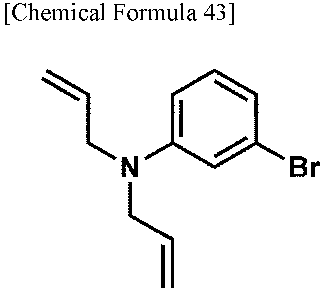

In a dried flask, 7.7 g (63.8 mmol) of allyl bromide, 5.0 g (29.0 mmol) of 3-bromoaniline, and 8.0 g (58.0 mmol) of potassium carbonate were placed, dissolved in 60 mL of acetonitrile, and stirred at 80°C overnight. The temperature was then returned to room temperature, and the mixture was filtered with cotton to remove potassium carbonate. Subsequently, after washing with ethyl acetate, the solvent was distilled off under reduced pressure. Subsequently, the residue was purified by column chromatography (silica; hexane 100%), and the eluent was evaporated to obtain 7.57 g of Compound 2 (30.0 mmol, yield: 47%).

### (2) Synthesis of Compound 3

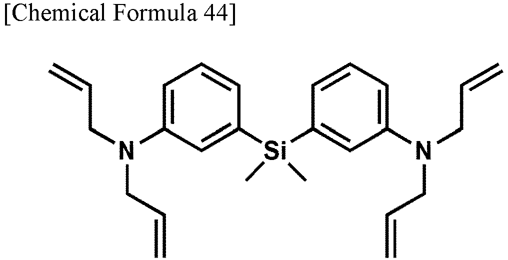

In a dried flask, 7.57 g (30.0 mmol) of Compound 2 was placed, dissolved in dehydrated tetrahydrofuran, and stirred at -78°C for 30 minutes under an argon atmosphere. Subsequently, 23.1 mL (30.0 mmol) of 1.3 M secondary butyl lithium was added portionwise thereto, and the mixture was stirred at -78°C for 30 minutes under an argon atmosphere. Subsequently, 1794 µL (15.02 mmol) of dichloridomethylsilane was further added portionwise to the reaction solution, and then the mixture was stirred at room temperature for 1.5 hours under an argon atmosphere. Subsequently, 10 mL of 2N HClaq. and NaHCO₃aq. were added to neutralize the reaction solution, the reaction solution was extracted with dichloromethane, washed with saturated saline, and then the solvent was distilled off under reduced pressure. Subsequently, the residue was purified by column chromatography (silica; dichloromethane/hexane), and the eluent was evaporated to obtain 4.0 g of Compound 3 (9.93 mmol, yield: 66%).

Analysis results of the obtained compound by ¹H-NMR and ¹³C-NMR are shown below.

¹H-NMR (400 MHz, CDCl₃) δ 7.20-7.16 (m, 2H), 6.88-6.84 (m, 4H), 6.71-6.68 (m, 2
H), 5.87-5.78 (m, 4H), 5.17-5.11 (m, 8H), 3.89 (d, J = 5.0 Hz, 8H), 0.48 (s, 6H)
¹³C-NMR (100 MHz, CDCl₃) δ 148.0, 139.0, 134.3, 128.5, 122.3, 118.2, 116.2, 113.3, 53.0, -2.3

### (3) Synthesis of Compound 4

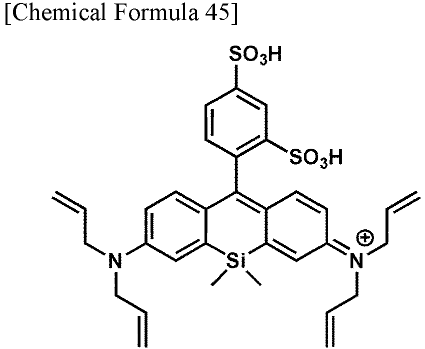

In a dried flask, 1.0 g (2.48 mmol) of Compound 3, 769 mg (2.48 mmol) of 4-formylbenzene-1,3-disulfonic acid disodium salt, and 5 mL of acetic acid were placed and stirred at 100°C for 1 hour under an argon atmosphere. Then, 112 mg (1.86 mmol) of urea was added thereto, and the mixture was stirred for 18 hours. Subsequently, acetic acid was distilled off under reduced pressure, 10 mL of methanol and 94 mg (2.48 mmol) of sodium borohydride were added, and the mixture was stirred at 0°C. Subsequently, the solvent was distilled off under reduced pressure, the obtained residue was purified by MPLC (eluent: A/B = 90/10 > 60/40, A: 0.1% TFA-containing 100% H₂O, B: 0.1% TFA-containing 100% CH₃CN), the eluent was evaporated, 10 mL of methanol and 10 mL of dichloromethane were added to the obtained residue, 610 mg (2.48 mmol) of chloranil was added thereto, and the mixture was stirred at room temperature for 5 hours. Subsequently, the solvent was distilled off under reduced pressure, the residue was then purified by column chromatography (silica; dichloromethane/methanol), and the eluent was evaporated to obtain 990 mg of Compound 4 (1.53 mmol, yield: 62%).

Analysis results of the obtained compound by ¹H-NMR and ¹³C-NMR are shown below.

¹H-NMR (400 MHz, CD₃OD) δ 8.62 (d, J = 1.8 Hz, 1H), 8.00 (dd, J = 7.8, 1.8 Hz, 1H), 7.26-7.24 (m, 3H), 7.07 (d, J = 9.6 Hz, 2H), 6.72 (dd, J = 9.6, 2.7 Hz, 2H), 5.96-5.87 (m, 4H), 5.27-5.17 (m, 8H), 4.27 (d, J = 4.6 Hz, 8H), 0.54(s, 3H), 0.52 (s, 3H)
¹³C-NMR (100 MHz, CD₃OD) δ 170.5, 153.9, 148.4, 145.7, 144.1, 142.7, 138.1, 131.4, 130.6, 128.7, 126.8, 125.6, 120.9, 116.7, 113.8, 53.2, -2.5, -3.11

### (4) Synthesis of Compound 5

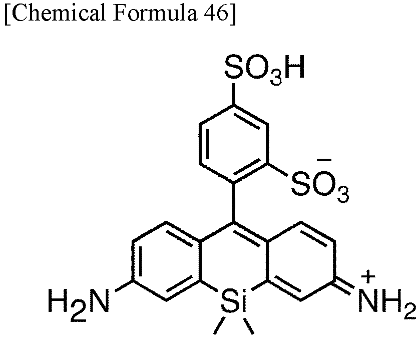

In a dried flask, 53 mg (0.081 mmol) of Compound 4 and 31 mg (0.81 mmol) of sodium borohydride were placed, dissolved in 1 mL of methanol, and mixed at room temperature. Subsequently, the solvent was distilled off under reduced pressure, and 1/3 (corresponding to 0.027 mmol) of the total amount was used for the next reaction. To the obtained residue, 100 µL (1.75 mmol) of acetic acid and 2 mL of water were added, and the mixture was degassed by an aspirator, then degassed again with the addition of a palladium carbon catalyst, and stirred at 90°C for 17 hours under an argon atmosphere. Then, the palladium carbon catalyst was removed by Celite filtration, and the solvent was distilled off under reduced pressure. Subsequently, the obtained residue was purified by HPLC (eluent: A/B = 90/10 > 50/50, A: 0.1% TFA-containing 100% H₂O, B: 0.1% TFA-containing 100% CH₃CN), and the eluent was evaporated to obtain 0.2 mg of Compound 5 (0.41 µmol, yield: 1.5%).

Analysis results of the obtained compound by ¹H-NMR are shown below.
¹H-NMR (400 MHz, CD₃OD/D₂O = 1:1) δ 8.48 (d, J = 2.0 Hz, 1H), 8.01 (dd, J = 7.6, 2.0 Hz, 1H), 7.26 (d, J = 7.6 Hz, 1H), 7.21 (d, J = 2.4 Hz, 2H), 6.98 (d, J = 8.4 Hz, 2H), 6.59 (dd, J = 8.4, 2.4 Hz, 2H), 0.53 (s, 3H), 0.52 (s, 3H)

### [Synthesis Example 4]

### Synthesis Example 2 of sSiR

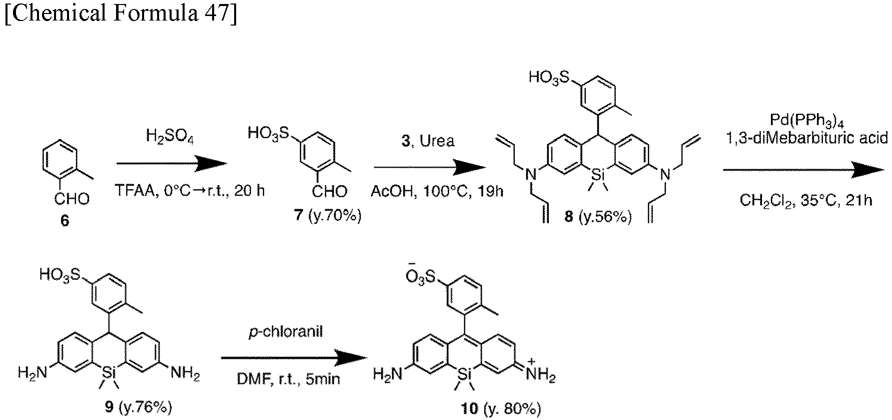

### (1) Synthesis of Compound 7

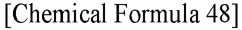

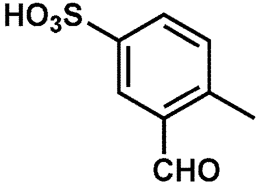

In a dried flask, 1.1 mL (20.8 mmol) of concentrated sulfuric acid was placed, and 6.0 mL (43.7 mmol) of trifluoroacetic anhydride was slowly added thereto at 0°C under an argon atmosphere. Thereafter, the temperature was returned to room temperature, and the mixture was stirred for 3.5 hours. When the solution became uniform, 2.5 mL (20.8 mmol) of 2-methylbenzaldehyde was then added portionwise at 0°C, the temperature was returned to room temperature, and the mixture was stirred for 20 hours. Thereafter, water was added at 0°C. Subsequently, trifluoroacetic acid and water were distilled off under reduced pressure. The residue was then purified by MPLC (eluent: A/B = 95/5 > 5/95, A: 0.1% TFA-containing 100% H₂O, B: 0.1% TFA-containing 100% CH₃CN). The eluent was evaporated, and then the product was freeze-dried to obtain Compound 7 (2.9 g, 14.5 mmol, yield: 70%).

Analysis results of the obtained compound by ¹H-NMR are shown below.
¹H-NMR (400 MHz, CD₃CN) δ 10.27 (s, 1H), 8.22 (d, J = 1.8 Hz, 1H), 7.94 (dd, J = 8.0, 2.1 Hz, 1H), 7.50 (d, J = 7.8 Hz, 1H), 2.70 (s, 3H)

### (2) Synthesis of Compound 8

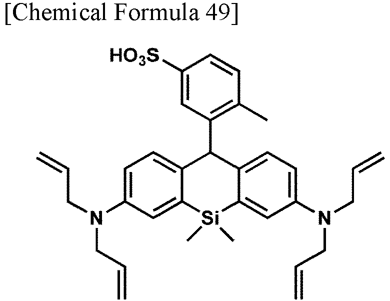

In a dried flask, 1.77 g (4.34 mmol) of Compound 3, 868 mg (4.34 mmol) of Compound 7, 130 mg (2.17 mmol) of urea, and 20 mL of acetic acid were placed and stirred at 90°C for 17 hours under an argon atmosphere. Subsequently, the solvent was distilled off under reduced pressure, the residue was then purified by column chromatography (silica; dichloromethane/methanol), and the eluent was evaporated to obtain 1220 mg of Compound 8 (2.09 mmol, yield: 48%).

### (3) Synthesis of Compound 9

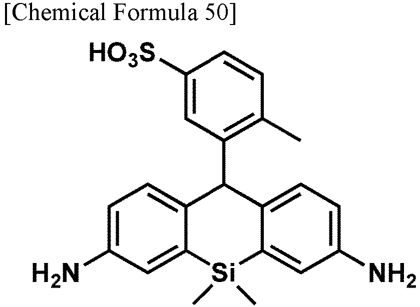

In a dried flask, 350 mg (0.616 mmol) of Compound 8 and 577 mg (3.70 mmol) of N,N-dimethylbarbituric acid were placed, dissolved in 20 mL of dichloromethane, and then degassed by an aspirator. Subsequently, 28 mg (0.024 mmol) of tetrakis(triphenylphosphine)palladium(0) was added thereto, the solvent was degassed again by an aspirator, and then the mixture was stirred at 35°C for 1 hour under an argon atmosphere. Subsequently, the solvent was distilled off under reduced pressure, the residue was then purified by column chromatography (silica; dichloromethane/methanol), and the eluent was evaporated to obtain 60 mg of Compound 9 (0.142 mmol, yield: 23%).

Analysis results of the obtained compound by ¹H-NMR and ¹³C-NMR are shown below.

¹H-NMR (400 MHz, CD₃OD) δ 7.60 (d, J = 1.8 Hz, 1H), 7.49 (dd, J = 8.2, 1.8 Hz, 1H), 7.15 (d, J = 8.2 Hz, 1H), 6.99 (d, J = 2.5 Hz, 2H), 6.79 (d, J = 8.5 Hz, 2H), 6.59 (dd, J = 8.5, 2.5 Hz, 2H), 5.59 (s, 1H), 2.25 (s, 3H), 0.58 (s, 3H), 0.36 (s, 3H)
¹³C-NMR (100 MHz, CD₃OD) δ 148.0, 145.3, 143.1, 139.2, 138.7, 134.6, 131.9, 130.8, 129.1, 124.0, 120.4, 118.4, 51.1, 20.3, -0.4, -0.8

### (4) Synthesis of Compound 10

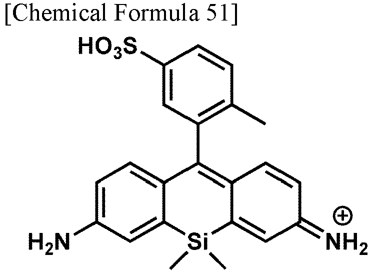

In a dried flask, 211 mg (0.497 mmol) of Compound 9, 246 mg (1 mmol) of chloranil, 4 mL of DMF, and 4 mL of dichloromethane were added, and the mixture was stirred at room temperature for 5 minutes. Subsequently, the reaction solution was purified by column chromatography (silica; dichloromethane/methanol), and the eluent was evaporated to obtain 168 mg of Compound 10 (0.398 mmol, yield: 80%).

Analysis results of the obtained compound by ¹H-NMR and ¹³C-NMR are shown below.
¹H-NMR (400 MHz, CD₃OD) δ 7.88 (dd, J = 8.0, 1.8 Hz, 1H), 7.53 (d, J = 1.8 Hz, 1H), 7.46 (d, J = 8.0 Hz, 1H), 7.18 (d, J = 2.5 Hz, 2H), 6.97 (d, J = 9.4 Hz, 2H), 6.53 (dd, J = 9.4, 2.5 Hz, 2H), 2.05 (s, 3H), 0.50 (s, 3H), 0.49 (s, 3H) ¹³C-NMR (100 MHz, CD₃OD) δ 168.8, 157.2, 149.0, 142.6, 142.3, 138.7, 138.1, 130.1, 126.9, 126.1, 123.3, 115.7, 109.5, 18.0, -2.8, -3.1

### [Synthesis Example 5]

### Synthesis Example 3 of sSiR

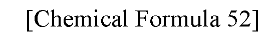

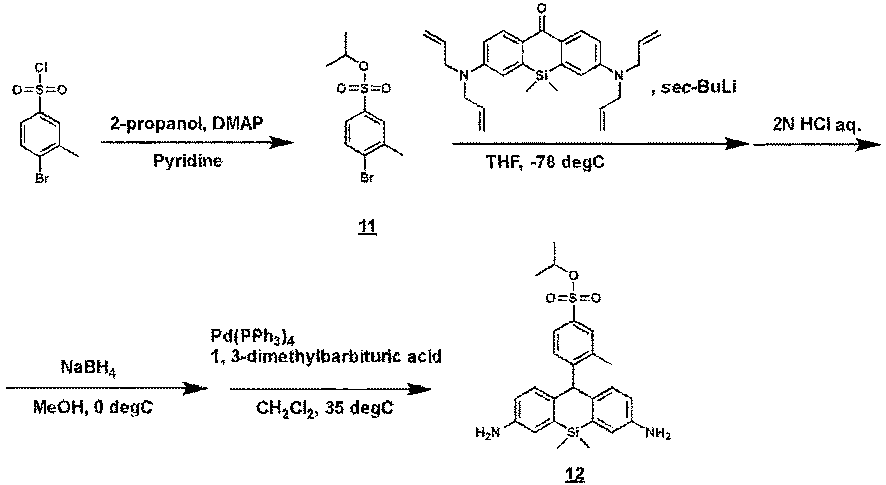

### (1) Synthesis of Compound 11

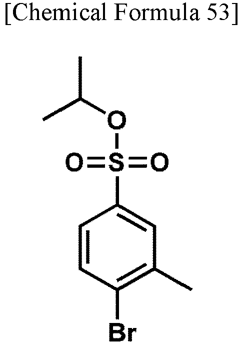

In a dried flask, 1620 mg (6 mmol) of 4-bromo-3-methylbenzenesulfonyl chloride, 732 mg (6 mmol) of 4-dimethylaminopyridine, 30 mL of 2-propanol, and 12 mL of pyridine were placed and stirred at room temperature for 2 hours. Subsequently, the solvent was distilled off under reduced pressure, the residue was purified by column chromatography (silica; ethyl acetate/hexane), and the eluent was evaporated to obtain 1008 mg of Compound 11 (3.44 mmol, yield: 57%).

Analysis results of the obtained compound by ¹H-NMR are shown below.
¹H-NMR (400 MHz, CDCl₃) δ 7.75 (d, J = 1.8 Hz, 1H), 7.69 (d, J = 8.7 Hz, 1H), 7.56 (dd, J = 8.2, 2.3 Hz, 1H), 4.71 (sep, J = 5.9 Hz, 1H), 2.47 (s, 3H), 1.28 (d, J = 5.9 Hz, 6H)

### (2) Synthesis of Compound 12

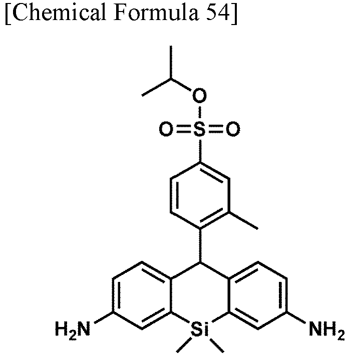

In a dried flask, 667 mg (2.27 mmol) of Compound 11 was placed, dissolved in dehydrated tetrahydrofuran, and stirred at -78°C for 30 minutes under an argon atmosphere. Subsequently, 1.75 mL (2.27 mmol) of 1.3 M secondary butyl lithium was added portionwise thereto, and the mixture was stirred at -78°C for 30 minutes under an argon atmosphere. Subsequently, 200 mg (0.45 mmol) of N,N,N',N'-tetraallyldiamino-Si-xanthene dissolved in dehydrated tetrahydrofuran was further added portionwise to the reaction solution, and then the mixture was stirred at room temperature for 1.5 hours under an argon atmosphere. To the reaction solution, 0.1 N HClaq. and water were then added, and the mixture was stirred at room temperature. Next, the mixture was extracted with dichloromethane, washed with saturated saline, and then dehydrated with sodium sulfate. Subsequently, the solvent was distilled off under reduced pressure, the residue was purified by column chromatography (silica; dichloromethane/methanol), and the solvent was evaporated. Subsequently, the obtained residue was dissolved in methanol, and cooled to 0°C, 8.3 mg (0.22 mmol) of sodium borohydride was then added portionwise, and the mixture was stirred. Next, the mixture was extracted with dichloromethane, washed with saturated saline, and then dehydrated with sodium sulfate. Thereafter, the solvent was distilled off under reduced pressure. To the obtained residue, 78 mg (0.5 mmol) of 1,3-dimethylbarbituric acid and 23 mg (0.02 mmol) of tetrakis(triphenylphosphine)palladium(0) were added, and the mixture was dissolved in 10 mL of dichloromethane and then stirred at 35°C for 3 hours under an argon atmosphere. Next, the mixture was neutralized with a saturated aqueous sodium bicarbonate solution, then extracted with dichloromethane, and washed with saturated saline. The mixture was then dehydrated with sodium sulfate, and the solvent was distilled off under reduced pressure. The obtained residue was purified by column chromatography (silica; ethyl acetate/hexane), and the eluent was evaporated to obtain 6 mg of Compound 12 (0.013 mmol, yield: 2.9%).

Analysis results of the obtained compound by ¹H-NMR are shown below.
¹H-NMR (400 MHz, CDCl₃) δ 7.66 (d, J = 1.8 Hz, 1H), 7.58 (dd, J = 8.2, 1.8 Hz, 1H), 7.19 (d, J = 8.2 Hz, 1H), 6.91 (d, J = 2.1 Hz, 2H), 6.72 (d, J = 8.7 Hz, 2H), 6.56 (dd, J = 8.2, 2.7 Hz, 2H), 5.62 (s, 1H), 4.75 (sep, J = 6.4 Hz, 1H), 2.35 (s, 3H), 1.27 (d, J = 6.4 Hz, 6H), 0.53 (s, 3H), 0.40 (s, 3H)

### [Synthesis Example 6]

### Synthesis Example of sSiR-Based Peptidase and Protease Probes

sSiR-based peptidase and protease probes (PB201 to PB211) shown in Table 3 were synthesized by the following procedures using a compound (Compound 9 or Compound 12) obtained by reducing sSiR.

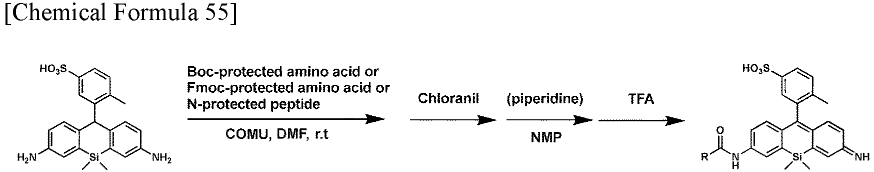

1. Compound 9 or Compound 12 (11.8 µmol), COMU (4.7 µmol), and Boc-protected amino acid or Fmoc-protected amino acid, or N-terminal protected peptide (4.7 µmol) were mixed in a 1.5 mL test tube, and the mixture was dissolved in 60 µL of DMF.
2. The reaction mixture was stirred at room temperature.
3. Chloranil (23.5 µmol) was added and the reaction mixture was stirred.
4. The solution was purified by MPLC (eluent: A/B = 95/5 > 5/95, A: 0.1% TFA-containing 100% H₂O, B: 0.1% TFA-containing 100% CH₃CN), the eluent was evaporated, and then the product was freeze-dried.
5. When the raw material was Fmoc-protected, 50 µL of piperidine and 50 µL of NMP were added to the obtained solid, and the reaction mixture solution was stirred. When the raw material was not Fmoc-protected, this step was not performed and the process proceeded to Step 6.
6. 1000 µL of TFA was added and the reaction mixture was stirred.
7. The solution was purified by MPLC (eluent: A/B = 95/5 > 5/95, A: 0.1% TFA-containing 100% H₂O, B: 0.1% TFA-containing 100% CH₃CN), the eluent was evaporated, and then the product was freeze-dried to obtain a compound of interest.

### [Table 3]

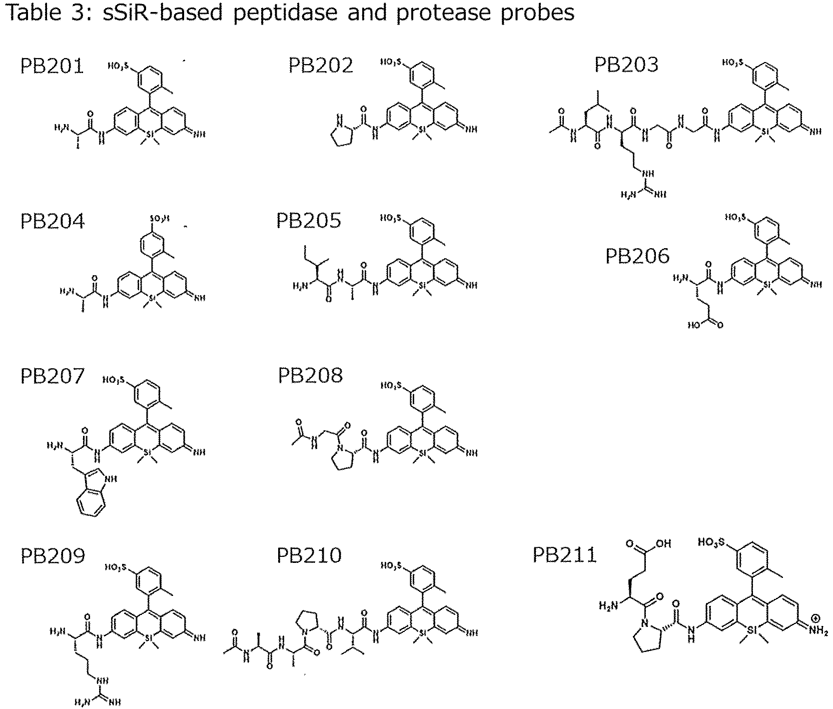

The instrumental data of the compound are shown below.
HPLC condition: A/B = 95/5 > 5/95 (5 min), A= 0.1% Formic acid H₂O, B = 0.1% Formic acid CH₃CN.

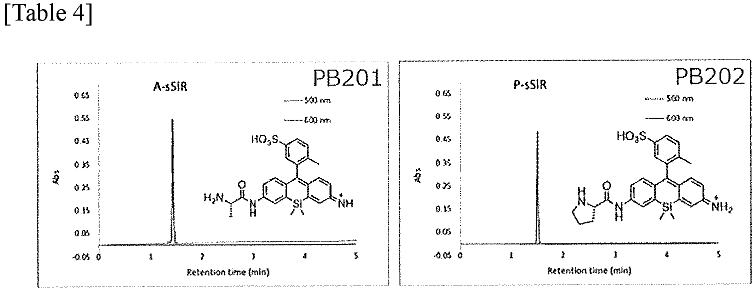

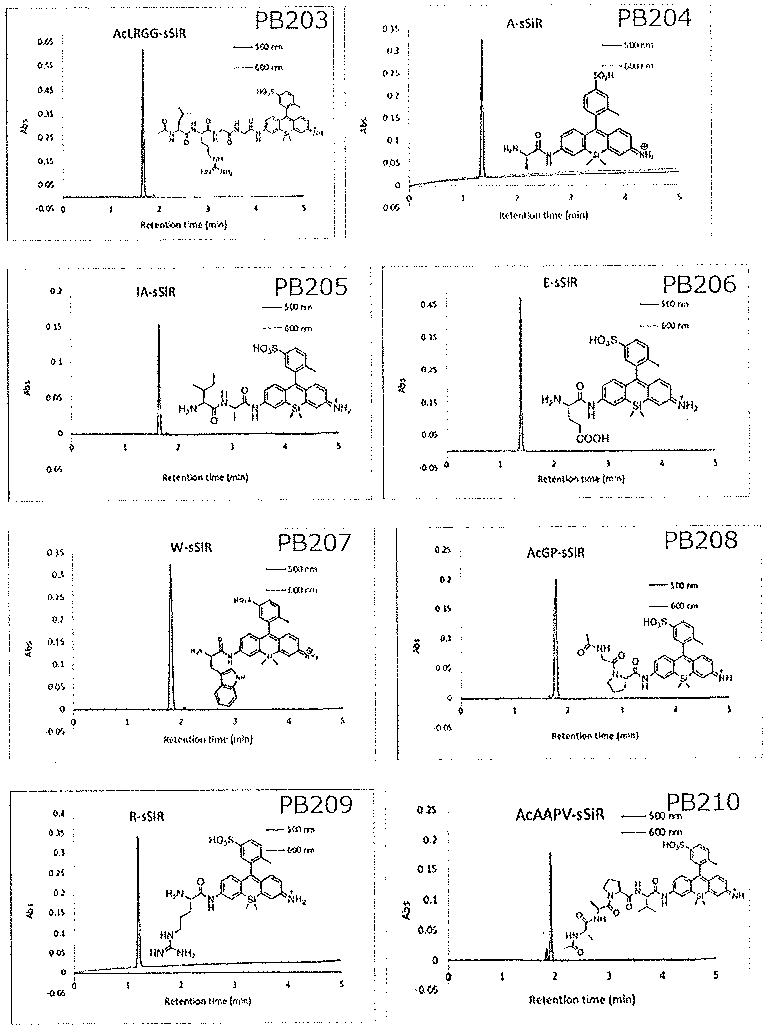

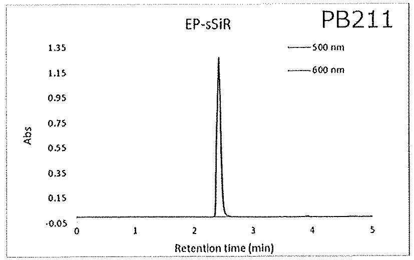

### [Synthesis Example 7]

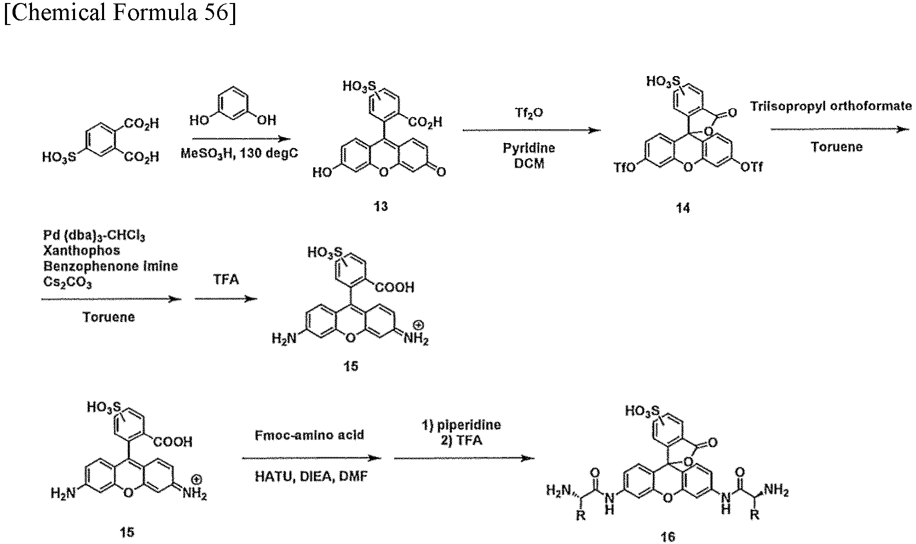

### Synthesis of Compound 13

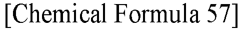

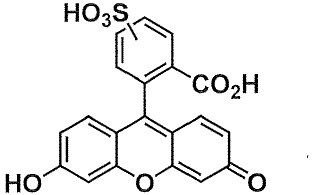

This synthetic method is performed with reference to Tetrahedron, 2005, 61, 12, 3097-3105.

4-Sulfophthalic acid, resorcinol, and methanesulfonic acid are mixed, heated at 130°C, and stirred. Water is added to the reaction solution, and the precipitated solid is washed with water and dried under reduced pressure to obtain a compound of interest.

### Synthesis of Compound 14

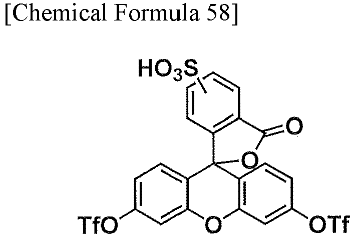

Compound 13 is dissolved in dichloromethane and stirred under argon substitution. Pyridine is added to the reaction solution, and subsequently, trifluoromethanesulfonic anhydride is added to the reaction solution. The reaction solution is washed with 2 N hydrochloric acid and then extracted with dichloromethane. The solution is dehydrated with sodium sulfate and then evaporated. The residue is purified by MPLC, and the eluent is evaporated. Thereafter, the product is freeze-dried under reduced pressure to obtain a compound of interest.

### Synthesis of Compound 15

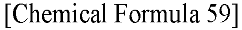

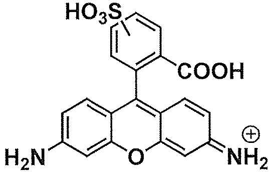

Compound 14 and triisopropyl orthoformate are dissolved in toluene and stirred to protect sulfonic acid with triisopropyl. The reaction solution is evaporated, and then the residue, Pd(dba)₃-CHCl₃, Xantphos, benzophenone imine, and cesium carbonate are dissolved in toluene and stirred. Thereafter, TFA is added and stirred, and deprotection is performed. After the reaction solution is evaporated, the residue is purified by MPLC, and the eluent is evaporated. Thereafter, the product is freeze-dried under reduced pressure to obtain a compound of interest.

### Synthesis of Compound 16

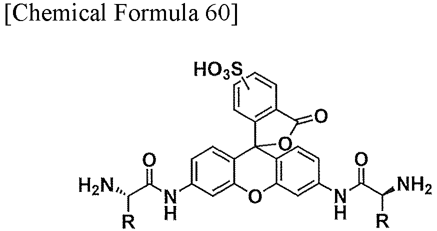

The synthesis of Compound 16 is performed with reference to J. Am. Chem. Soc. 2013, 135, 16, 6002-6005. Compound 15, Fmoc-protected amino acid, HATU, and DIEA are dissolved in DMF and stirred to condense the amino acid. Thereafter, piperidine is added and stirred to deprotect the Fmoc group, and then trifluoroacetic acid is added and stirred to deprotect the amino acid side chain. The reaction solution is purified by MPLC, and the eluent is evaporated. Thereafter, the product is freeze-dried under reduced pressure to obtain a compound of interest.

### [Synthesis Example 8]

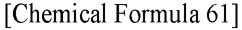

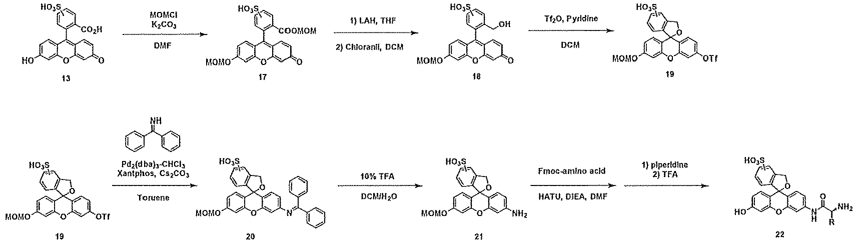

wherein MOMCl represents chloromethyl methyl ether (Cl-CH₃-O-CH₃), and -MOM represents a residue of chloromethyl methyl ether (-CH₃-O-CH₃).

### Synthesis of Compound 17

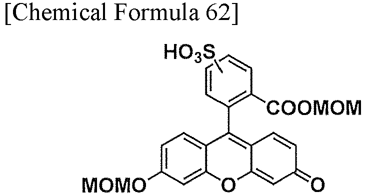

The synthesis of Compounds 17 to 21 is performed with reference to Nat Commun. 2015, 6, 6463. Compound 13 is dissolved in DMF and potassium carbonate is added to the reaction solution. Thereafter, chloromethyl methyl ether is added to the reaction solution and stirred. Ethyl acetate and a saturated aqueous ammonium chloride solution are added to the reaction solution and stirred. Thereafter, water and 85% phosphoric acid are added to the reaction solution, and the pH of the reaction solution is adjusted to around 3. The ethyl acetate layer was extracted, then washed with saturated saline, and dehydrated with sodium sulfate. After the reaction solution is evaporated, the residue is purified by column chromatography, and the eluent is evaporated. Thereafter, the product is dried under reduced pressure to obtain a compound of interest.

### Synthesis of Compound 18

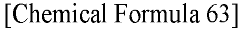

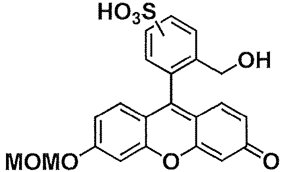

Compound 17 is dissolved in THF, and then LAH is added to the reaction solution. Thereafter, methanol is added to the reaction solution to stop the reaction, and the reaction solution is evaporated. The residue is dissolved in dichloromethane and washed with Rochelle's salt aqueous solution. The organic layer is extracted, dehydrated with sodium sulfate, and then evaporated. The residue is dissolved in dichloromethane and chloranil is added and stirred. The reaction solution is filtered, then washed with water, and dehydrated with sodium sulfate. The residue obtained by evaporating the solution is purified by column chromatography, and the eluent is evaporated. Thereafter, the product is dried under reduced pressure to obtain a compound of interest.

### Synthesis of Compound 19

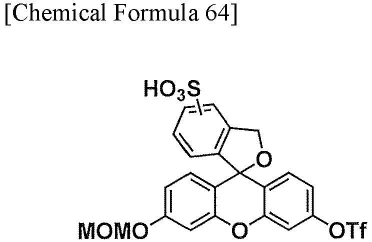

Compound 18 is dissolved in dichloromethane, and pyridine is added to the reaction solution. Thereafter, trifluoromethanesulfonic anhydride dissolved in dichloromethane is added to the reaction solution and stirred. The reaction solution is evaporated, the obtained residue is purified by column chromatography, and the eluent is evaporated. Thereafter, the product is dried under reduced pressure to obtain a compound of interest.

### Synthesis of Compound 20

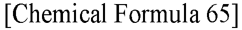

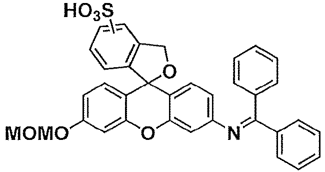

Compound 19, Pd₂(dba₃)-CHCl₃, Xantphos, and cesium carbonate are dissolved in toluene and stirred under argon substitution. Benzophenone imine is added and stirred, and then the reaction solution is dissolved in dichloromethane and filtered with Celite. After the filtrate is evaporated, the obtained residue is purified by column chromatography, and the eluent is evaporated. Thereafter, the product is dried under reduced pressure to obtain a compound of interest.

### Synthesis of Compound 21

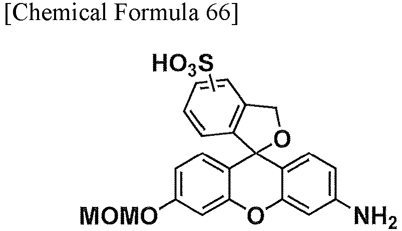

Compound 20 is dissolved in dichloromethane, 10% TFA/water is added thereto, and the mixture is stirred. Thereafter, the reaction solution is made basic with a 1 M NaOH aqueous solution, and then extracted with dichloromethane. The solution is dehydrated with sodium sulfate and then evaporated. The obtained residue is purified by column chromatography, and the eluent is evaporated. Thereafter, the product is dried under reduced pressure to obtain a compound of interest.

### Synthesis of Compound 22

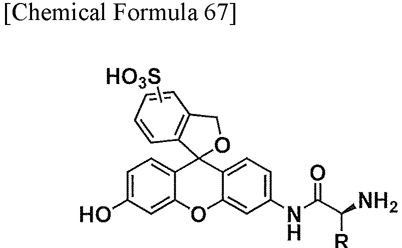

Compound 21, Fmoc-protected amino acid, HATU, and DIEA are dissolved in DMF and stirred to condense the amino acid. Thereafter, piperidine is added and stirred to deprotect the Fmoc group, and then trifluoroacetic acid is added and stirred to deprotect the amino acid side chain. The reaction solution is purified by MPLC, and the eluent is evaporated. Thereafter, the product is freeze-dried under reduced pressure to obtain a compound of interest.

### <Measurement of LogP Value>

The relationship between the index (LogP) of lipophilicity and the microdevice compatibility was examined for the following compounds including the compounds (Compounds 4, 5, and 10) of the fluorescent mother nucleus obtained in the above Synthesis Examples. The results are shown in Table 5 below.

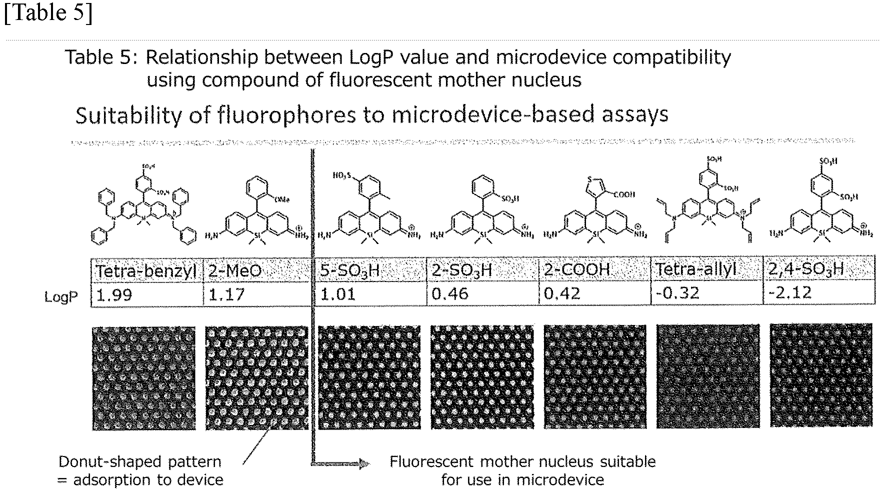

A protocol for measuring logP will be described below.

### (Protocol)

(1) 4 µL of 10 mM Stock (calculated at e = 91,000) was added with respect to 500 µL of MilliQ and 500 µL of 1-octanol, mixed well, and then centrifuged.
(2) 50 µL of the solution of each layer was collected, 50 µL of MeOH was mixed, and the mixture was analyzed by LC-MS.
(3) The concentration ratio was calculated from the area ratio of each peak, and LogP was calculated.
(Peak area: unsubstituted product: @600 nM, disubstituted product: @629 nm, trisubstituted product: @638 nm, tetrasubstituted product: @655 nm)

In Table 5, the compounds are arranged in the order of the measured value of logP value, which is an index of lipophilicity, from left to right; however, in the case of a compound having high lipophilicity (specifically, LogP > 1.01), a donut-shaped fluorescence image is obtained by adsorption to a microdevice, and it is suggested that the compound is not suitable for an assay using a microdevice.

The leftmost compound had two sulfonic acids, but had very high lipophilicity due to the substitution of four benzyl groups, and this also provided a donut-shaped fluorescence image, and in the measurement using a confocal microscope, leakage to the outside of the device was also observed, and thus applicability to a microdevice was low.

The above results have been studied using a compound of a fluorescent mother nucleus which is a structure analog exhibiting observable fluorescence (one having a structure after the fluorescent probe is metabolized to an enzyme), and it is considered that the same tendency is exhibited because the structure is similar for the compound of the present invention (fluorescent probe) in which a partial structure of an amino acid, a partial structure of a saccharide, or the like is introduced into an amino group of a xanthene skeleton.

### <Calculation of MiLogP Value>

For the compound for which the LogP value was actually measured (Table 5), a MiLogP (Molinspiration calculated logP) value was calculated using a program: Molinspiration property engine v2022.08 (https://www.molinspiration.com/cgi-bin/properties) provided by Molinspiration Cheminformatics. The results are shown in Table 6 below.

### [Table 6]

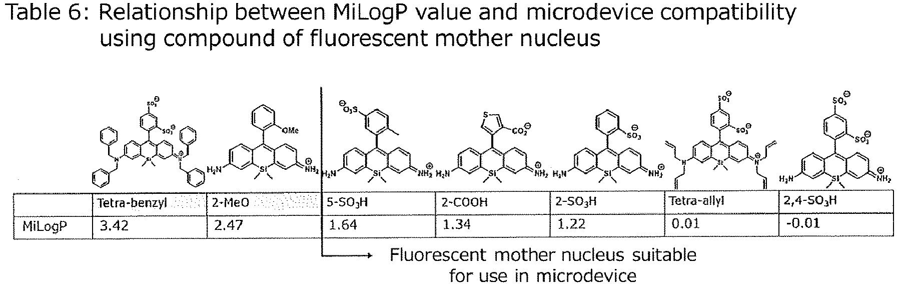

As shown in Table 6, for the compound of the present invention, the MiLogP value was almost correlated with the actually measured LogP value. That is, it is considered that the compound of the present invention can be specified not only by the LogP value but also by the MiLogP value.

For the fluorescent probes (Examples 1 to 16) recognized to be suitable for use in enzyme activity assays in a microdevice, the MiLogP value was calculated, and the results thereof were as follows.
Ala-sHMRG (PB101): -1.86
Leu-sHMRG (PB102): -0.55
Met-sHMRG (PB104): -1.41
Tyr-sHMRG (PB105): -0.88
Lys-sHMRG (PB110): -1.62
gGlu-sHMRG (PB106): -2.26
GP-sHMRG (PB109): -2.52
Ac-Ala-sHMRG (PB107): -1.59
Suc-AAPA-sHMRG (PB111): -3.08
Ala-sSiR (PB201): 0.95
Trp-sSiR (PB207): 2.56
Glu-sSiR (PB206): 0.54
Arg-sSiR (PB209): 0.13
Ac-GP-sSiR (PB208): 0.63
Ac-LRGG-sSiR (PB203): 0.23
EP-sSiR (PB211): 0.28

Since the fluorescent probe is recognized to be suitable for use in an enzyme activity assay in a microdevice, it was shown that the MiLogP value can be used as an index for determining the suitability of the compound of the present invention as a fluorescent probe in a microdevice.

### <Assay Example>

### Activity Measurement in Human Blood Using sHMRG- and sSiR-Based Peptidase and Protease Probes

Hereinafter, an enzyme activity assay using the sHMRG-based fluorescent probe (X is an oxygen atom) and the sSiR-based fluorescent probe (X is Si(R^{a})(R^{b})) will be shown, but it can be understood by those skilled in the art that the compound of the present invention in which X is an atom other than an oxygen atom and a silicon atom also functions as a fluorescent probe in response to an enzyme reaction. For example, an example of a fluorescent probe in which X is a carbon atom is described in WO 2018/151260 A, and an example of a fluorescent probe in which X is a germanium atom is described in Communications Chemistry volume 2, Article number: 94 (2019).

### [Example 1]

### Assay of Enzyme Activity Using Ala-sHMRG (PB101)

The assay of enzyme activity was performed using Ala-sHMRG having the following structure. The assay protocol is shown below.

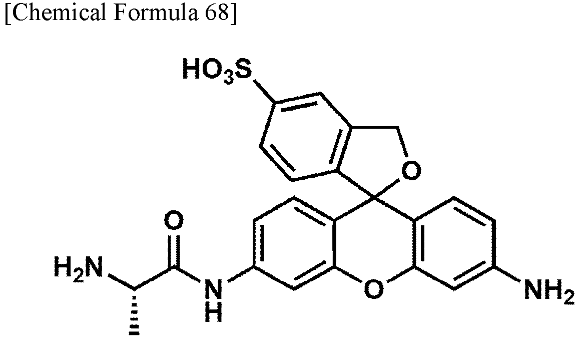

### Assay Protocol

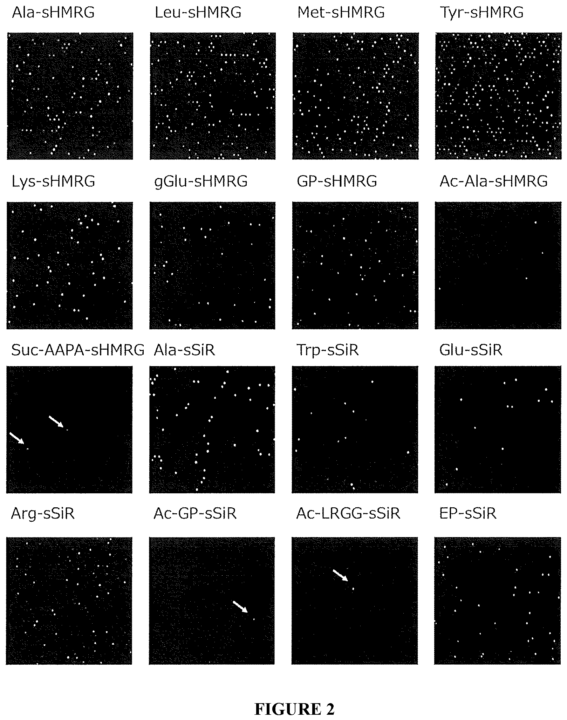

1. Ala-sHMRG as a fluorescent probe for detecting aminopeptidase activity was diluted with an assay buffer to 100 µM. The composition of the assay buffer is 100 mM HEPES-NaOH (pH 7.4), 1 mM MgCl₂, 0.1% BSA, 1 mM DTT, 1 mM Triton X-100,
2. Next, a human plasma sample was diluted by 500 times with the above assay buffer, and mixed in an equal amount with the diluted solution of the probe. At this time, the final concentrations of both are 50 µM for Ala-sHMRG and 1,000 fold dilution for the human plasma sample.
3. The mixed solution of both was then added to a microdevice, followed by the addition of sealing oil to enclose the solution in each well.
4. The microdevice in which the solution was enclosed was incubated at 37°C for 4 hours, and then the fluorescence intensity of each well was measured with a fluorescence microscope. In the wells in which the enzyme reacting with Ala-sHMRG was enclosed, an increase in fluorescence intensity was observed. Fig. 2 shows a fluorescence microscopic image.

### [Example 2]

### Assay of Enzyme Activity Using Leu-sHMRG (PB102)

The assay of enzyme activity was performed using Leu-sHMRG having the following structure. The assay protocol is shown below.

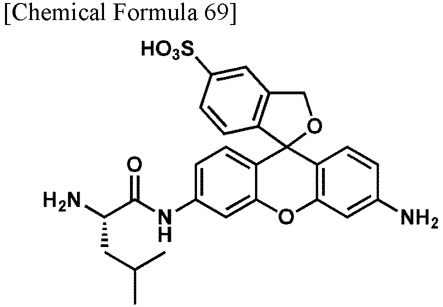

### Assay Protocol

1. Leu-sHMRG as a fluorescent probe for detecting aminopeptidase activity was diluted with an assay buffer to 100 µM. The composition of the assay buffer is 100 mM HEPES-NaOH (pH 7.4), 1 mM MgCl₂, 1 mM DTT, 3 mM Triton X-100.
2. Next, a human plasma sample was diluted by 2,500 times with the above assay buffer, and mixed in an equal amount with the diluted solution of the probe. At this time, the final concentrations of both are 50 µM for Leu-sHMRG and 5,000 fold dilution for the human plasma sample.
3. The mixed solution of both was then added to a microdevice, followed by the addition of sealing oil to enclose the solution in each well.
4. The microdevice in which the solution was enclosed was incubated at 37°C for 4 hours, and then the fluorescence intensity of each well was measured with a fluorescence microscope. In the wells in which the enzyme reacting with Leu-sHMRG was enclosed, an increase in fluorescence intensity was observed. Fig. 2 shows a fluorescence microscopic image.

### [Example 3]

### Assay of Enzyme Activity Using Met-sHMRG (PB104)

The assay of enzyme activity was performed using Met-sHMRG having the following structure. The assay protocol is shown below.

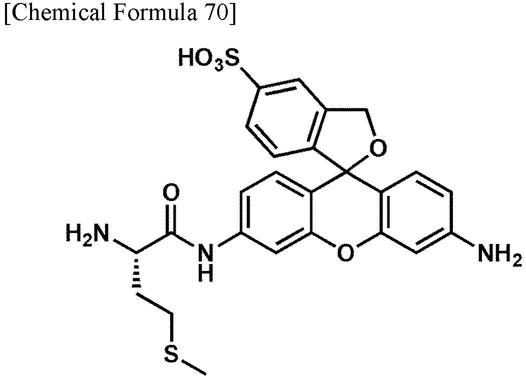

### Assay Protocol

5. Met-sHMRG as a fluorescent probe for detecting aminopeptidase activity was diluted with an assay buffer to 100 µM. The composition of the assay buffer is 100 mM HEPES-NaOH (pH 7.4), 1 mM MgCl₂, 1 mM CaCl₂, 1 mM DTT, 3 mM Triton X-100.
6. Next, a human plasma sample was diluted by 500 times with the above assay buffer, and mixed in an equal amount with the diluted solution of the probe. At this time, the final concentrations of both are 50 µM for Met-sHMRG and 1,000 fold dilution for the human plasma sample.
7. The mixed solution of both was then added to a microdevice, followed by the addition of sealing oil to enclose the solution in each well.
8. The microdevice in which the solution was enclosed was incubated at 37°C for 2 hours, and then the fluorescence intensity of each well was measured with a fluorescence microscope. In the wells in which the enzyme reacting with Met-sHMRG was enclosed, an increase in fluorescence intensity was observed. Fig. 2 shows a fluorescence microscopic image.

### [Example 4]

### Assay of Enzyme Activity Using Tyr-sHMRG (PB105)

The assay of enzyme activity was performed using Tyr-sHMRG having the following structure. The assay protocol is shown below.

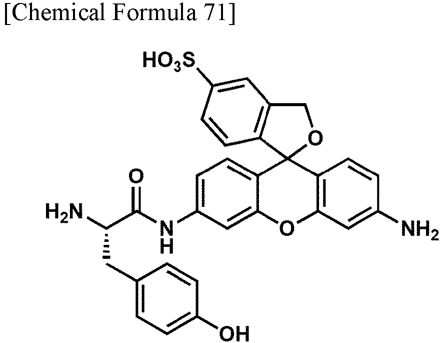

### Assay Protocol

9. Tyr-sHMRG as a fluorescent probe for detecting aminopeptidase activity was diluted with an assay buffer to 100 µM. The composition of the assay buffer is 100 mM HEPES-NaOH (pH 7.4), 1 mM MgCl₂, 1 mM CaCl₂, 1 mM DTT, 3 mM Triton X-100.
10. Next, a human plasma sample was diluted by 500 times with the above assay buffer, and mixed in an equal amount with the diluted solution of the probe. At this time, the final concentrations of both are 50 µM for Tyr-sHMRG and 1,000 fold dilution for the human plasma sample.
11. The mixed solution of both was then added to a microdevice, followed by the addition of sealing oil to enclose the solution in each well.
12. The microdevice in which the solution was enclosed was incubated at 37°C for 4 hours, and then the fluorescence intensity of each well was measured with a fluorescence microscope. In the wells in which the enzyme reacting with Tyr-sHMRG was enclosed, an increase in fluorescence intensity was observed. Fig. 2 shows a fluorescence microscopic image.

### [Example 5]

### Assay of Enzyme Activity Using Lys-sHMRG (PB110)

The assay of enzyme activity was performed using Lys-sHMRG having the following structure. The assay protocol is shown below.

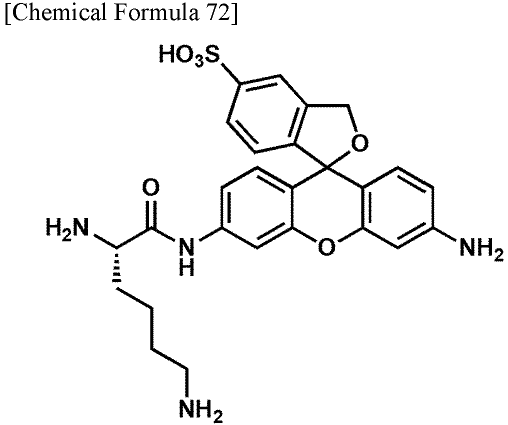

### Assay Protocol

1. Lys-sHMRG as a fluorescent probe for detecting aminopeptidase activity was diluted with an assay buffer to 100 µM. The composition of the assay buffer is 100 mM HEPES-NaOH (pH 7.4), 1 mM MgCl₂, 1 mM CaCl₂, 1 mM DTT, 3 mM Triton X-100.
2. Next, a human plasma sample was diluted by 10,000 times with the above assay buffer, and mixed in an equal amount with the diluted solution of the probe. At this time, the final concentrations of both are 50 µM for Lys-sHMRG and 20,000 fold dilution for the human plasma sample.
3. The mixed solution of both was then added to a microdevice, followed by the addition of sealing oil to enclose the solution in each well.
4. The microdevice in which the solution was enclosed was incubated at 37°C for 2 hours, and then the fluorescence intensity of each well was measured with a fluorescence microscope. In the wells in which the enzyme reacting with Lys-sHMRG was enclosed, an increase in fluorescence intensity was observed. Fig. 2 shows a fluorescence microscopic image.

### [Example 6]

### Assay of Enzyme Activity Using gGlu-sHMRG (PB106)

The assay of enzyme activity was performed using gGlu-sHMRG having the following structure. The assay protocol is shown below.

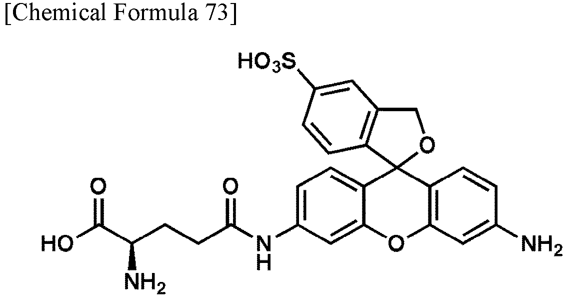

### Assay Protocol

1. gGlu-sHMRG as a fluorescent probe for detecting aminopeptidase activity was diluted with an assay buffer to 100 µM. The composition of the assay buffer is 100 mM HEPES-NaOH (pH 7.4), 1 mM MgCl₂, 1 mM CaCl₂, 1 mM DTT, 3 mM Triton X-100.
2. Next, a human plasma sample was diluted by 500 times with the above assay buffer, and mixed in an equal amount with the diluted solution of the probe. At this time, the final concentrations of both are 50 µM for gGlu-sHMRG and 1,000 fold dilution for the human plasma sample.
3. The mixed solution of both was then added to a microdevice, followed by the addition of sealing oil to enclose the solution in each well.
4. The microdevice in which the solution was enclosed was incubated at 37°C for 2 hours, and then the fluorescence intensity of each well was measured with a fluorescence microscope. In the wells in which the enzyme reacting with gGlu-sHMRG was enclosed, an increase in fluorescence intensity was observed. Fig. 2 shows a fluorescence microscopic image.

### [Example 7]

### Assay of Enzyme Activity Using GP-sHMRG (PB109)

The assay of enzyme activity was performed using GP-sHMRG having the following structure. The assay protocol is shown below.

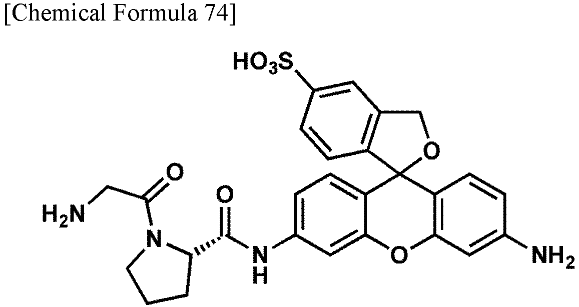

### Assay Protocol

1. GP-sHMRG as a fluorescent probe for detecting peptidase activity was diluted with an assay buffer to 100 µM. The composition of the assay buffer is 100 mM HEPES-NaOH (pH 7.4), 1 mM MgCl₂, 1 mM CaCl₂, 1 mM DTT, 3 mM Triton X-100.
2. Next, a human plasma sample was diluted by 2,500 times with the above assay buffer, and mixed in an equal amount with the diluted solution of the probe. At this time, the final concentrations of both are 50 µM for GP-sHMRG and 5,000 fold dilution for the human plasma sample.
3. The mixed solution of both was then added to a microdevice, followed by the addition of sealing oil to enclose the solution in each well.
4. The microdevice in which the solution was enclosed was incubated at 25°C for 2 hours, and then the fluorescence intensity of each well was measured with a fluorescence microscope. In the wells in which the enzyme reacting with GP-sHMRG was enclosed, an increase in fluorescence intensity was observed. Fig. 2 shows a fluorescence microscopic image.

### [Example 8]

### Assay of Enzyme Activity Using Ac-Ala-sHMRG (PB107)

The assay of enzyme activity was performed using Ac-Ala-sHMRG having the following structure.

The assay protocol is shown below.

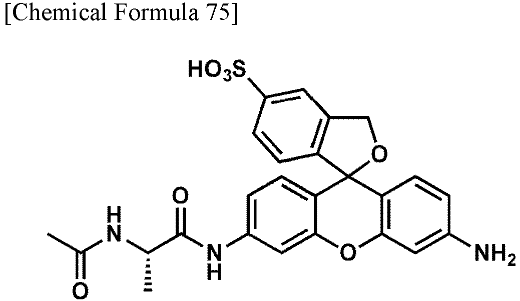

### Assay Protocol

1. Ac-Ala-sHMRG as a fluorescent probe for detecting peptidase activity was diluted with an assay buffer to 100 µM. The composition of the assay buffer is 100 mM HEPES-NaOH (pH 7.4), 1 mM MgCl₂, 1 mM CaCl₂, 1 mM DTT, 3 mM Triton X-100.
2. Next, a human plasma sample was diluted by 250 times with the above assay buffer, and mixed in an equal amount with the diluted solution of the probe. At this time, the final concentrations of both are 50 µM for Ac-Ala-sHMRG and 500 fold dilution for the human plasma sample.
3. The mixed solution of both was then added to a microdevice, followed by the addition of sealing oil to enclose the solution in each well.
4. The microdevice in which the solution was enclosed was incubated at 37°C for 4 hours, and then the fluorescence intensity of each well was measured with a fluorescence microscope. In the wells in which the enzyme reacting with Ac-Ala-sHMRG was enclosed, an increase in fluorescence intensity was observed. Fig. 2 shows a fluorescence microscopic image.

### [Example 9]

### Assay of Enzyme Activity Using Suc-AAPA-sHMRG (PB111)

The assay of enzyme activity was performed using Suc-AAPA-sHMRG having the following structure. The assay protocol is shown below.

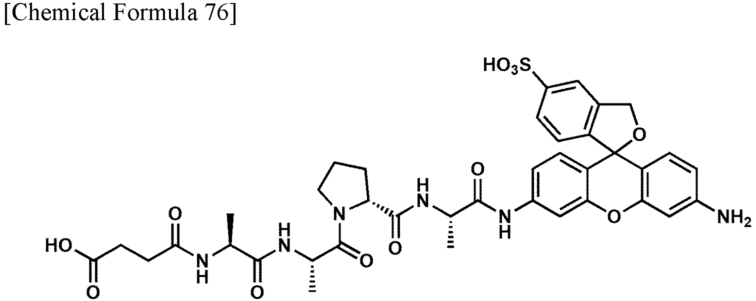

### Assay Protocol

1. Suc-AAPA-sHMRG as a fluorescent probe for detecting protease activity was diluted with an assay buffer to 200 µM. The composition of the assay buffer is 100 mM HEPES-NaOH (pH 7.4), 1 mM MgCl₂, 1 mM CaCl₂, 1 mM DTT, 3 mM Triton X-100.
2. Next, a human plasma sample was diluted by 250 times with the above assay buffer, and mixed in an equal amount with the diluted solution of the probe. At this time, the final concentrations of both are 100 µM for Suc-AAPA-sHMRG and 500 fold dilution for the human plasma sample.
3. The mixed solution of both was then added to a microdevice, followed by the addition of sealing oil to enclose the solution in each well.
4. The microdevice in which the solution was enclosed was incubated at 37°C for 4 hours, and then the fluorescence intensity of each well was measured with a fluorescence microscope. In the wells in which the enzyme reacting with Suc-AAPA-sHMRG was enclosed, an increase in fluorescence intensity was observed. Fig. 2 shows a fluorescence microscopic image.

### [Example 10]

### Assay of Enzyme Activity Using Ala-sSiR (PB201)

The assay of enzyme activity was performed using Ala-sSiR having the following structure. The assay protocol is shown below.

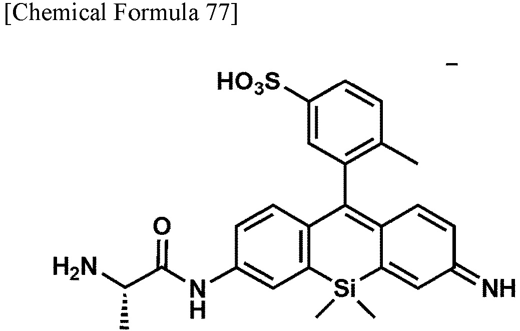

### Assay Protocol

1. Ala-sSiR as a fluorescent probe for detecting aminopeptidase activity was diluted with an assay buffer to 100 µM. The composition of the assay buffer is 100 mM HEPES-NaOH (pH 7.4), 1 mM MgCl₂, 1 mM CaCl₂, 1 mM DTT, 3 mM Triton X-100.
2. Next, a human plasma sample was diluted by 10,000 times with the above assay buffer, and mixed in an equal amount with the diluted solution of the probe. At this time, the final concentrations of both are 50 µM for Ala-sSiR and 20,000 fold dilution for the human plasma sample.
3. The mixed solution of both was then added to a microdevice, followed by the addition of sealing oil to enclose the solution in each well.
4. The microdevice in which the solution was enclosed was incubated at 37°C for 2 hours, and then the fluorescence intensity of each well was measured with a fluorescence microscope. In the wells in which the enzyme reacting with Ala-sSiR was enclosed, an increase in fluorescence intensity was observed. Fig. 2 shows a fluorescence microscopic image.

### [Example 11]

### Assay of Enzyme Activity Using Trp-sSiR (PB207)

The assay of enzyme activity was performed using Trp-sSiR having the following structure. The assay protocol is shown below.

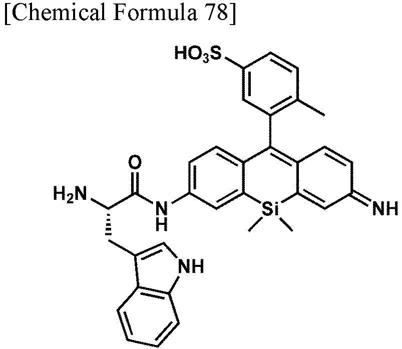

### Assay Protocol

1. Trp-sSiR as a fluorescent probe for detecting aminopeptidase activity was diluted with an assay buffer to 100 µM. The composition of the assay buffer is 100 mM HEPES-NaOH (pH 7.4), 1 mM MgCl₂, 1 mM CaCl₂, 1 mM DTT, 3 mM Triton X-100.
2. Next, a human plasma sample was diluted by 500 times with the above assay buffer, and mixed in an equal amount with the diluted solution of the probe. At this time, the final concentrations of both are 50 µM for Trp-sSiR and 1,000 fold dilution for the human plasma sample.
3. The mixed solution of both was then added to a microdevice, followed by the addition of sealing oil to enclose the solution in each well.
4. The microdevice in which the solution was enclosed was incubated at 37°C for 4 hours, and then the fluorescence intensity of each well was measured with a fluorescence microscope. In the wells in which the enzyme reacting with Trp-sSiR was enclosed, an increase in fluorescence intensity was observed. Fig. 2 shows a fluorescence microscopic image.

### [Example 12]

### Assay of Enzyme Activity Using Glu-sSiR (PB206)

The assay of enzyme activity was performed using Glu-sSiR having the following structure. The assay protocol is shown below.

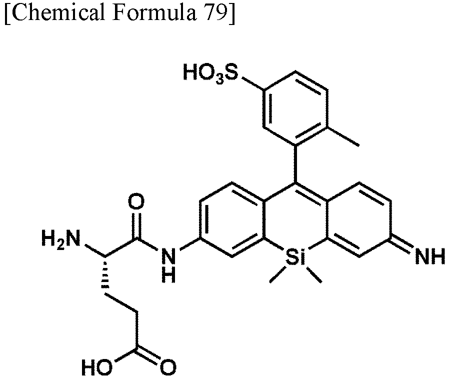

### Assay Protocol

1. Glu-sSiR as a fluorescent probe for detecting aminopeptidase activity was diluted with an assay buffer to 100 µM. The composition of the assay buffer is 100 mM HEPES-NaOH (pH 7.4), 1 mM MgCl₂, 1 mM CaCl₂, 1 mM DTT, 3 mM Triton X-100.
2. Next, a human plasma sample was diluted by 500 times with the above assay buffer, and mixed in an equal amount with the diluted solution of the probe. At this time, the final concentrations of both are 50 µM for Glu-sSiR and 1,000 fold dilution for the human plasma sample.
3. The mixed solution of both was then added to a microdevice, followed by the addition of sealing oil to enclose the solution in each well.
4. The microdevice in which the solution was enclosed was incubated at 37°C for 2 hours, and then the fluorescence intensity of each well was measured with a fluorescence microscope. In the wells in which the enzyme reacting with Glu-sSiR was enclosed, an increase in fluorescence intensity was observed. Fig. 2 shows a fluorescence microscopic image.

### [Example 13]

### Assay of Enzyme Activity Using Arg-sSiR (PB209)

The assay of enzyme activity was performed using Arg-sSiR having the following structure. The assay protocol is shown below.

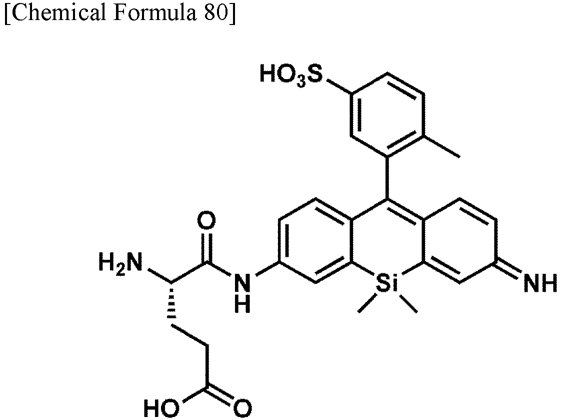

### Assay Protocol

1. Arg-sSiR as a fluorescent probe for detecting aminopeptidase activity was diluted with an assay buffer to 100 µM. The composition of the assay buffer is 100 mM HEPES-NaOH (pH 7.4), 1 mM MgCl₂, 1 mM CaCl₂, 1 mM DTT, 3 mM Triton X-100.
2. Next, a human plasma sample was diluted by 500 times with the above assay buffer, and mixed in an equal amount with the diluted solution of the probe. At this time, the final concentrations of both are 50 µM for Arg-sSiR and 1,000 fold dilution for the human plasma sample.
3. The mixed solution of both was then added to a microdevice, followed by the addition of sealing oil to enclose the solution in each well.
4. The microdevice in which the solution was enclosed was incubated at 37°C for 4 hours, and then the fluorescence intensity of each well was measured with a fluorescence microscope. In the wells in which the enzyme reacting with Arg-sSiR was enclosed, an increase in fluorescence intensity was observed. Fig. 2 shows a fluorescence microscopic image.

### [Example 14]

### Assay of Enzyme Activity Using Ac-GP-sSiR (PB208)

The assay of enzyme activity was performed using Ac-GP-sSiR having the following structure. The assay protocol is shown below.

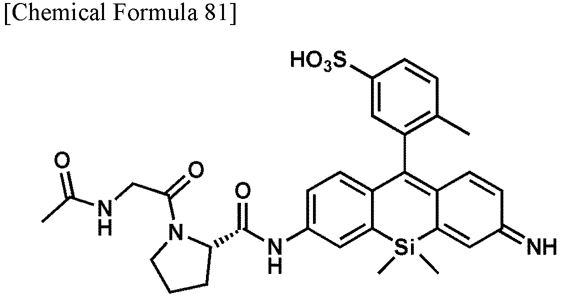

### Assay Protocol

1. Ac-GP-sSiR as a fluorescent probe for detecting peptidase activity was diluted with an assay buffer to 100 µM. The composition of the assay buffer is 100 mM HEPES-NaOH (pH 7.4), 1 mM MgCl₂, 1 mM CaCl₂, 1 mM DTT, 3 mM Triton X-100.
2. Next, a human plasma sample was diluted by 10,000 times with the above assay buffer, and mixed in an equal amount with the diluted solution of the probe. At this time, the final concentrations of both are 50 µM for Ac-GP-sSiR and 20,000 fold dilution for the human plasma sample.
3. The mixed solution of both was then added to a microdevice, followed by the addition of sealing oil to enclose the solution in each well.
4. The microdevice in which the solution was enclosed was incubated at 37°C for 2 hours, and then the fluorescence intensity of each well was measured with a fluorescence microscope. In the wells in which the enzyme reacting with Ac-GP-sSiR was enclosed, an increase in fluorescence intensity was observed. Fig. 2 shows a fluorescence microscopic image.

### [Example 15]

### Resuslt of Assay of Enzyme Activity Using Ac-LRGG-sSiR (PB203)

The assay of enzyme activity was performed using Ac-LRGG-sSiR having the following structure. The assay protocol is shown below.

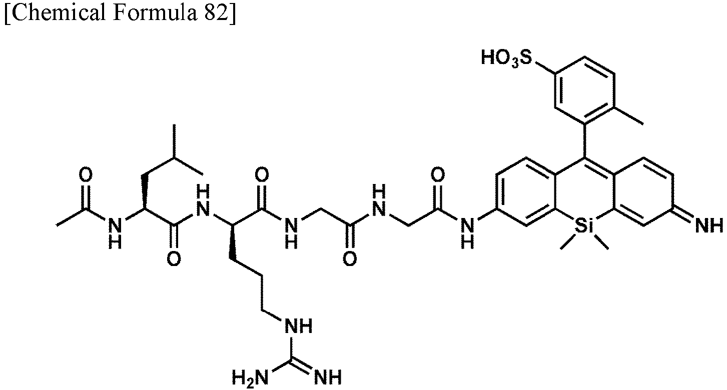

### Assay Protocol

1. Ac-LRGG-sSiR as a fluorescent probe for detecting protease activity was diluted with an assay buffer to 100 µM. The composition of the assay buffer is 100 mM HEPES-NaOH (pH 7.4), 1 mM MgCl₂, 1 mM CaCl₂, 1 mM DTT, 3 mM Triton X-100.
2. Next, a human plasma sample was diluted by 10,000 times with the above assay buffer, and mixed in an equal amount with the diluted solution of the probe. At this time, the final concentrations of both are 50 µM for Ac-LRGG-sSiR and 20,000 fold dilution for the human plasma sample.
3. The mixed solution of both was then added to a microdevice, followed by the addition of sealing oil to enclose the solution in each well.
4. The microdevice in which the solution was enclosed was incubated at 37°C for 2 hours, and then the fluorescence intensity of each well was measured with a fluorescence microscope. In the wells in which the enzyme reacting with Ac-LRGG-sSiR was enclosed, an increase in fluorescence intensity was observed. Fig. 2 shows a fluorescence microscopic image.

### [Example 16]

### Resuslt of Assay of Enzyme Activity Using EP-sSiR (PB211)

The assay of enzyme activity was performed using EP-sSiR having the following structure. The assay protocol is shown below.

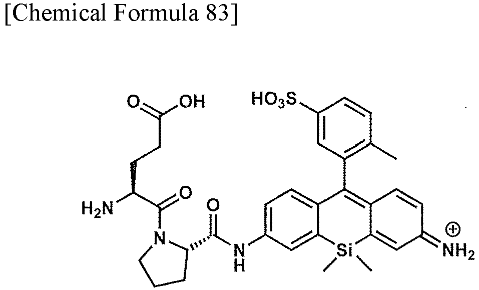

### Assay Protocol

1. EP-sSiR as a fluorescent probe for detecting protease activity was diluted with an assay buffer to 60 µM. The composition of the assay buffer is 100 mM HEPES-NaOH (pH 7.4), 1 mM MgCl₂, 1 mM CaCl₂, 0.1 mM DTT, 150 µM Triton X-100.
2. Next, a human plasma sample was diluted by 2,500 times with the above assay buffer, and mixed in an equal amount with the diluted solution of the probe. At this time, the final concentrations of both are 30 µM for EP-sSiR and 5,000 fold dilution for the human plasma sample.
3. The mixed solution of both was then added to a microdevice, followed by the addition of sealing oil to enclose the solution in each well.
4. The microdevice in which the solution was enclosed was incubated at 25°C for 2 hours, and then the fluorescence intensity of each well was measured with a fluorescence microscope. In the wells in which the enzyme reacting with EP-sSiR was enclosed, an increase in fluorescence intensity was observed. Fig. 2 shows a fluorescence microscopic image.

### <Example of Detection of Pancreatic Cancer Biomarker>

Next, an experiment of detecting a pancreatic cancer biomarker was performed using the fluorescent probe of the present invention.

### [Example 17]

### Diagnostic Experiment of Pancreatic Disease (Pancreatic Cancer) Utilizing Single-Molecule Enzyme Activity of CD13

In order to detect the single-molecule enzyme activity of CD13, an experiment to verify the availability of a probe to which Met is bonded as a reaction point (in General Formula (I) or (II), an alanine residue was introduced as -CO-R₀ or - CO-R¹⁰) to detect a pancreatic cancer biomarker was performed in the following procedures.

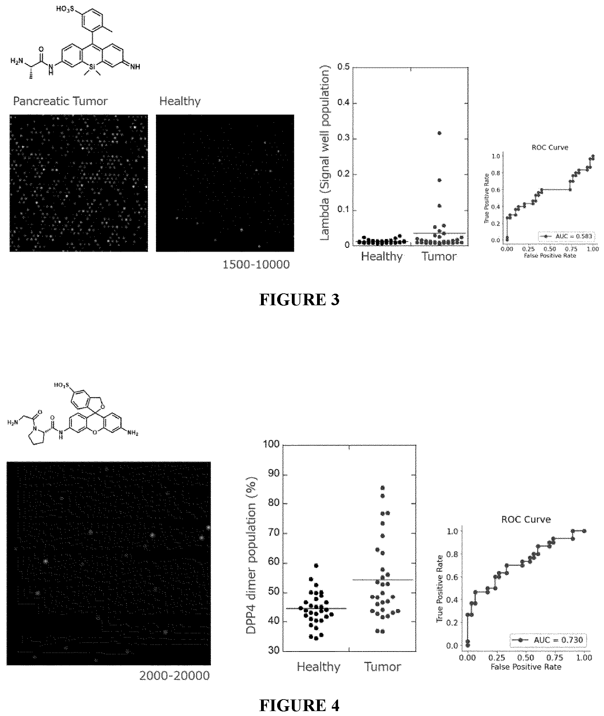

1. Ala-sSiR as a CD13 detecting probe was diluted with an assay buffer to 100 µM. The composition of the assay buffer is 100 mM HEPES-NaOH (pH 7.4), 1 mM MgCl₂, 1 mM CaCl₂, 1 mM DTT, 3 mM Triton X-100.
2. Next, human plasma samples collected from 30 patients with pancreatic cancer and 30 healthy subjects were each diluted by 10,000 times with the above assay buffer, and mixed in an equal amount with the diluted solution of Ala-sSiR. At this time, the final concentrations of both are 50 µM for the Ala-sSiR probe and 20,000 fold dilution for the human plasma sample.
3. The mixed solution of both was then added to a microdevice, followed by the addition of sealing oil to enclose the solution in each well.
4. The microdevice in which the solution was enclosed was incubated at 37°C for 2 hours, and then the fluorescence intensity of each well was measured with a fluorescence microscope. Fig. 3 shows a fluorescence microscopic image.
5. As for the number of wells in which the luminance is 2,500 AU or more, that is, CD13 is considered to be enclosed, a significant increase in the number of wells was observed in the patients with pancreatic cancer as compared with the healthy subjects. The results thereof are shown in Fig. 3.

As described above, it was detected that there is an example in which the activity is increased in the plasma of a patient with pancreatic cancer, and it was shown from this that it is possible to distinguish a patient with pancreatic cancer (Fig. 3).

Specifically, in this example, an ROC curve for determination based on the enzyme activity of CD13 determined based on the proportion of wells with a luminance of 2500 AU or more is created, and when a threshold is subtracted at the time when the number of wells of a signal is 3%, the sensitivity is 27% and the specificity is 100% (the right view of Fig. 3). It is possible to determine pancreatic cancer by such an ROC curve.

### [Example 18]

### Diagnostic Experiment of Pancreatic Disease (Pancreatic Cancer) Utilizing Single-Molecule Enzyme Activity of DPP4

In order to detect the single-molecule enzyme activity of DPP4, an experiment to verify the availability of the probe GP-sHMRG to which Gly-Pro is bonded as a reaction point to detect a pancreatic cancer biomarker was performed in the following procedures.

1. GP-sHMRG as a DPP4 detecting probe was diluted with an assay buffer to 100 µM. The composition of the assay buffer is 100 mM HEPES-NaOH (pH 7.4), 1 mM MgCl₂, 1 mM CaCl₂, 1 mM DTT, 3 mM Triton X-100.
2. Next, human plasma samples collected from 30 patients with pancreatic cancer and 30 healthy subjects were each diluted by 2,500 times with the above assay buffer, and mixed in an equal amount with the diluted solution of GP-sHMRG. At this time, the final concentrations of both are 50 µM for the GP-sHMRG probe and 5,000 fold dilution for the human plasma sample.
3. The mixed solution of both was then added to a microdevice, followed by the addition of sealing oil to enclose the solution in each well.
4. The microdevice in which the solution was enclosed was incubated at 25°C for 2 hours, and then the fluorescence intensity of each well was measured with a fluorescence microscope. Fig. 4 shows a fluorescence microscopic image.
5. The histogram created based on the detected fluorescence intensity of each well is approximated to a curve including two normal distributions. When a well having a fluorescence intensity higher than the minimum value of the approximate curve is defined as a high activity group, a well having a fluorescence intensity lower than the minimum value of the approximate curve is defined as a low activity group, a significant increase in the ratio of the high activity group to the total of these groups was observed in the patients with pancreatic cancer as compared with the healthy subjects. The results thereof are shown in Fig. 4.

It was detected that there is an example in which the single-molecule activity pattern is changed in the plasma of a patient with pancreatic cancer by a probe to which Gly-Pro is bonded as a reaction point, and it was shown from this that it is possible to distinguish a patient with pancreatic cancer (Fig. 4).

Specifically, in this example, an ROC curve for determination based on the enzyme activity of DPP4 determined based on the proportion of the high activity group is created, and when a threshold is subtracted at the time when the proportion of the high activity group is 55%, the sensitivity is 37% and the specificity is 97% (the right view of Fig. 4). It is possible to determine pancreatic cancer by such an ROC curve.

### <Evaluation of Suitability for Use in Microdevice>

### [Example 19]

### Study of Solubility in Sealing Oil

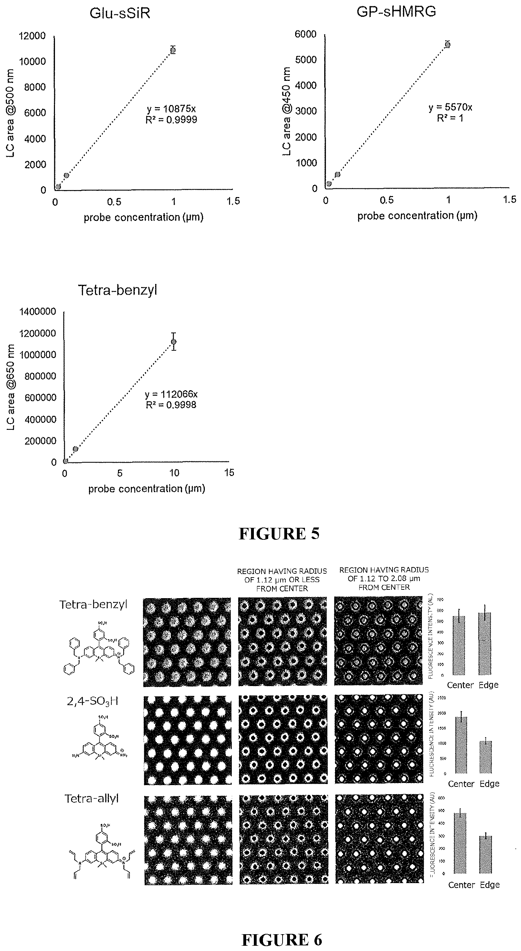

The solubility of two types of compounds Glu-sSiR (PB206/Example 12) and GP-sHMRG (PB 109/Example 7), and the leftmost compound "Tetra-benzyl" in Table 5 in sealing oil was measured as follows. That is, 1 µL of a 10 mM DMSO solution of the test compound was added with respect to 100 µL of hexadecane as sealing oil, and the mixture was sonicated and suspended at room temperature (25°C) for 1 minute. Thereafter, the solution was centrifuged for 30 seconds, 50 µL of the supernatant was collected and mixed with 100 µL of methanol, and a compound dissolved in hexadecane was extracted. Thereafter, the methanol solution from which the compound was extracted was analyzed by ultra performance liquid chromatography (UPLC) (eluent: A/B = 95/5 > 5/95, A: 0.1% formic acid-containing 100% H₂O, B: 0.1% formic acid-containing 100% CH₃CN), and the concentration of the compound in the methanol solution was calculated from the peak area. In the calculation of the solution concentration using the UPLC peak area, solutions having a plurality of concentrations were prepared and measured for each compound, then a calibration curve was prepared (Fig. 5), and the calculation was performed according to the calibration curve. The peak area was calculated for Glu-sSiR at 500 nm, for GP-sHMRG at 450 nm, and for the compound "Tetra-benzyl" at 650 nm, respectively.

Based on the calibration curve, the solubility was calculated from the analysis result of each compound as follows.

Glu-sSiR (PB206): The peak area was 1637 ± 524 (average value ± standard deviation) by three tests, and thus the solubility in hexadecane was calculated to be 0.30 ± 0.096 µM.

GP-sHMRG (PB109): The peak area was 740 ± 74 (average value ± standard deviation) by three tests, and thus the solubility in hexadecane was calculated to be 0.27 ± 0.026 µM.

Compound "Tetra-benzyl": The peak area was 152015 ± 5430 (average value ± standard deviation) by three tests, and thus the solubility in hexadecane was calculated to be 2.71 ± 0.097 µM.

Since Glu-sSiR and GP-sHMRG are recognized to be suitable for use in the enzyme activity assay in a microdevice in Examples 12 and 7, it was shown that solubility in sealing oil can be an index of suitability in the microdevice.

### [Example 20]

### Study of Evaluation of Degree of Adsorption to Well Wall Surface

For the compound "Tetra-benzyl" at the left end of Table 5 and the two compounds "Tetra-allyl" and "2,4-SO₃H" at the right end of Table 5, the degree of adsorption to the well wall surface was measured as follows. That is, these compounds were diluted with an assay buffer so as to be each 10 µM, and each solution was then added to a microdevice, followed by the addition of sealing oil to enclose the solution in each well. After incubation at 25°C for 1 hour, images of the compound encapsulated wells were photographed with a microscope. The composition of the assay buffer is 100 mM HEPES-NaOH (pH 7.4), 1 mM MgCl₂, 1 mM CaCl₂, 250 µM Triton X-100.

For the photographed images, the fluorescence intensities of a region (center region) having a radius of 1.12 µm or less from the center point of the well and a region (edge region) having a radius of more than 1.12 µm and 2.08 µm or less from the center point of the well were measured, and the ratio of the fluorescence intensity of the edge region to the fluorescence intensity of the center region (edge/center) was calculated based on the average value (Tetra-benzyl: n = 10,153, 2,4-SO₃H: n = 10,151, Tetra-allyl: n = 10,078). As a result, in the compound "Tetra-benzyl", the fluorescence intensity of the center region was 552±60 (average value ± standard deviation), the fluorescence intensity of the edge portion was 581±71 (average value ± standard deviation), and the ratio (edge/center) was 1.05, whereas in the compound "2,4-SO₃H", the fluorescence intensity of the center region was 1,880 ± 178 (average value ± standard deviation), the fluorescence intensity of the edge portion was 1,087 ± 106 (average value ± standard deviation), and the ratio (edge/center) was 0.58, and in the compound "Tetra-allyl", the fluorescence intensity of the center region was 483 ± 33 (average value ± standard deviation), the fluorescence intensity of the edge portion was 300 ± 23 (average value ± standard deviation), and the ratio (edge/center) was 0.62 (Fig. 6).

As shown in Table 5, since a donut-shaped fluorescence image due to adsorption to the microdevice was observed for the compound "Tetra-benzyl", and such a fluorescence image was not observed for the compounds "Tetra-allyl" and "2,4-SO₃H", it was shown that among the fluorescence intensities observed for the test compound in the microdevice, the ratio of the fluorescence intensity of the edge region to the fluorescence intensity of the center region can be an index of suitability in the microdevice.

### [Example 21]

### Study of Signal/Noise Ratio

For two types of compounds Glu-sSiR (PB206/Example 12) and GP-sHMRG (PB 109/Example 7), the signal/noise ratios of the enzymolysates before and after the enzyme reaction in the cuvette and the microdevice were each measured as follows. In the following experiments, the composition of the assay buffer is 100 mM HEPES-NaOH (pH 7.4), 1 mM MgCl₂, 1 mM CaCl₂, 100 mM DTT, 150 mM Triton X-100.

### (1) Measurement in Cuvette

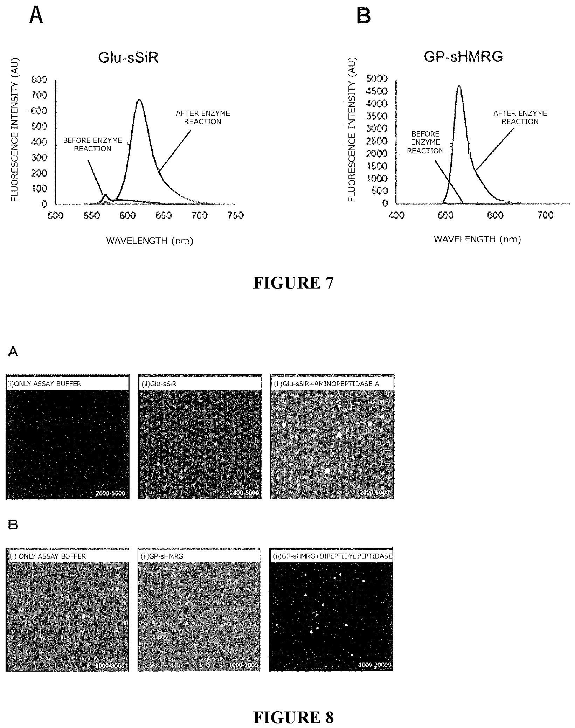

Glu-sSiR was diluted with an assay buffer so as to be 10 µM, then added to a quartz cuvette, and the fluorescence spectrum before the enzyme reaction was measured with a fluorometer (excitation wavelength: 570 nm). Thereafter, aminopeptidase A was added so that the concentration in the solution was 170 ng/mL, the solution was incubated at 25°C for 11 hours, and then the fluorescence spectrum was measured. The fluorescence spectra before and after the enzyme reaction were compared, the ratio of the values at a wavelength at which the fluorescence intensity was maximized was calculated as the signal/noise ratio of the enzymolysate, the ratio was 32 times (@616 nm) (see Fig. 7A).

GP-sHMRG was diluted with an assay buffer so as to be 10 µM, then added to a quartz cuvette, and the fluorescence spectrum before the enzyme reaction was measured with a fluorometer (excitation wavelength: 500 nm). Thereafter, dipeptidyl peptidase was added so that the concentration in the solution was 17 ng/mL, the solution was incubated at 25°C for 9 hours, and then the fluorescence spectrum was measured. The fluorescence spectra before and after the enzyme reaction were compared, the ratio of the values at a wavelength at which the fluorescence intensity was maximized was calculated as the signal/noise ratio of the enzymolysate, the ratio was 481 times (@527 nm) (see Fig. 7B).

### (2) Measurement in Microdevice

For Glu-sSiR, three kinds of solutions of (i) only an assay buffer, (ii) 30 µM of Glu-sSiR, and (iii) 30 µM of Glu-sSiR and 0.2 ng/mL of aminopeptidase A were prepared, and each solution was added to a microdevice, followed by the addition of sealing oil to enclose the solution in each well. The microdevice in which the solution was enclosed was incubated at 37°C for 4 hours, and then the fluorescence intensity of each well was measured with a fluorescence microscope. When a difference between the average values of the fluorescence intensities of the respective wells in (ii) and (i) was defined as noise, a difference between the average value of the fluorescence intensities of the wells in which the enzyme was encapsulated in (iii) and the average value of the fluorescence intensities of the respective wells in (i) was defined as a signal, and a ratio of these values was calculated as the signal/noise ratio of the enzymolysate, the ratio was 8.3 times (see Fig. 8A).

For GP-sHMRG, three kinds of solutions of (iv) only an assay buffer, (v) 30 µM of GP-sHMRG, and (vi) 30 µM of GP-sHMRG and 0.2 ng/mL of dipeptidyl peptidase were prepared, and each solution was added to a microdevice, followed by the addition of sealing oil to enclose the solution in each well. The microdevice in which the solution was enclosed was incubated at 25°C for 2 hours, and then the fluorescence intensity of each well was measured with a fluorescence microscope. When a difference between the average values of the fluorescence intensities of the respective wells in (v) and (iv) was defined as noise, a difference between the average value of the fluorescence intensities of the wells in which the enzyme was encapsulated in (vi) and the average value of the fluorescence intensities of the respective wells in (iv) was defined as a signal, and a ratio of these values was calculated as the signal/noise ratio of the enzymolysate, the ratio was 287 times (see Fig. 8B).

### (3) Discussion

When the signal/noise ratios in the two assay systems were compared, the ratio of the signal/noise ratio in the microdevice to the signal/noise ratio in the cuvette was 0.26 for Glu-sSiR and 0.59 for GP-sHMRG.

In the case of using the compound of the present invention as a fluorescent probe, the signal/noise ratio in the microdevice was inferior to the cuvette, but was not significantly deteriorated. Since Glu-sSiR and GP-sHMRG are recognized to be suitable for use in the enzyme activity assay in a microdevice in Examples 12 and 7, it was shown that the ratio of the signal/noise ratios in the cuvette and the microdevice can be an index of suitability in the microdevice.

## Claims

1. A fluorescent probe for detecting a hydrolase, the fluorescent probe comprising a compound having at least one water-soluble substituent selected from the group consisting of -CO₂H, -PO₃H₂, and -SO₃H and represented by General Formula (II) below, or a salt thereof: 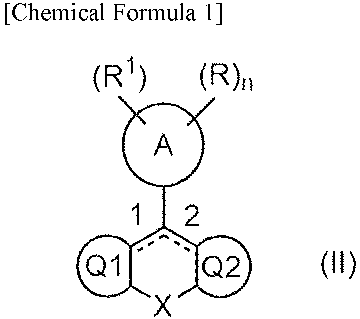
wherein ring A is an aromatic ring which is phenyl or 5- or 6-membered heteroaryl;
ring Q1 is a ring structure of Formula (i) or (ii) below, 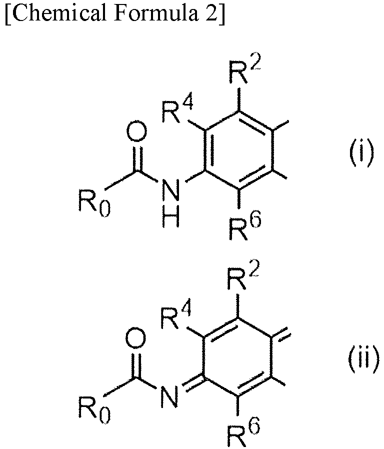
wherein R², R⁴, R⁶ and R₀ are as defined below;
ring Q2 is a ring structure of Formula (iii), (iv), (v), or (vi) below, 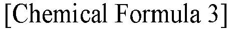 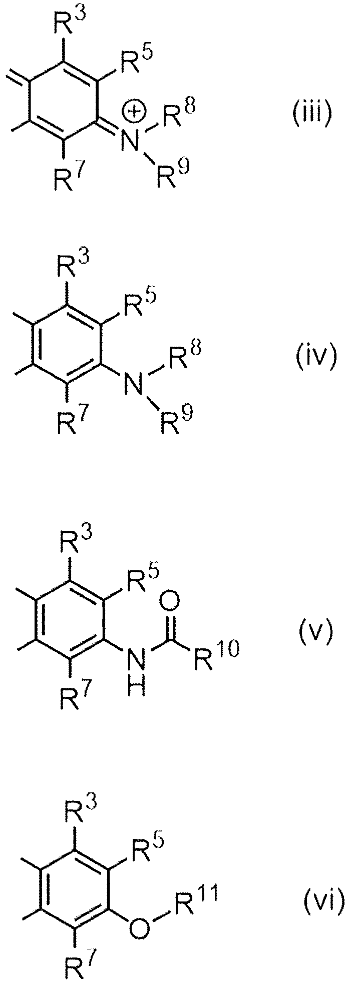
wherein R³, R⁵, R⁷, R⁸, R⁹, R¹⁰, and R¹¹ are as defined below;
a bond - - - represented as 1 and a bond - - - represented as 2 in an X atom-containing ring structure each represent a single bond or a double bond, provided that both of the bonds do not represent a double bond at the same time;
R is selected from the group consisting of -CO₂H, -PO₃H₂, -SO₃H, -L-CO₂H, -L-PO₃H₂, and -L-SO₃H, wherein L is a linker;
n is an integer of 0 to 5;
R¹ represents a monovalent substituent optionally present on the aromatic ring, wherein the substituents may be the same or different when two or more monovalent substituents are present,
wherein, R¹, together with a carbon atom on the ring A to which R¹ is bonded, a carbon atom on the X atom-containing ring structure to which the ring A is bonded, and a part of the ring A, may form a 5- or 6-membered carbocyclic or heterocyclic ring;
R² and R³ are each independently a hydrogen atom, a substituted or unsubstituted alkyl group having 1 to 6 carbon atoms, a halogen atom, the water-soluble substituent, or an alkyl group having the water-soluble substituent and having 1 to 6 carbon atoms;
R⁴ and R⁵ are each independently a hydrogen atom, a substituted or unsubstituted alkyl group having 1 to 6 carbon atoms, a halogen atom, the water-soluble substituent, or an alkyl group having the water-soluble substituent and having 1 to 6 carbon atoms;
R⁶ and R⁷ are each independently a hydrogen atom, a substituted or unsubstituted alkyl group having 1 to 6 carbon atoms, a halogen atom, the water-soluble substituent, or an alkyl group having water-soluble substituent and having 1 to 6 carbon atoms;
R⁸ and R⁹ are each independently a hydrogen atom, a substituted or unsubstituted alkyl group having 1 to 6 carbon atoms, a substituted or unsubstituted alkenyl group having 1 to 6 carbon atoms, an alkyl group having the water-soluble substituent and having 1 to 10 carbon atoms, preferably 1 to 6 carbon atoms (this alkyl group may be substituted with an aryl group, and the aryl group may be substituted with the water-soluble substituent), or an alkenyl group having the water-soluble substituent and having 1 to 10 carbon atoms, preferably 1 to 6 carbon atoms (this alkenyl group may be substituted with an aryl group, and the aryl group may be substituted with the water-soluble substituent),
wherein R⁸ and R⁹ may together form a 4- to 7-membered heterocyclyl that may contain a nitrogen atom to which R⁸ and R⁹ are attached and may have a substituent,
R⁸ or R⁹, or both of R⁸ and R⁹, together with R⁵ or R⁷, each may form a 5- to 7-membered heterocyclyl or heteroaryl containing a nitrogen atom to which R⁸ or R⁹ is attached, and further, the heterocyclyl or heteroaryl may have a substituent selected from the group consisting of alkyl having 1 to 6 carbon atoms, alkenyl having 2 to 6 carbon atoms, alkynyl having 2 to 6 carbon atoms, an aralkyl group having 6 to 10 carbon atoms, and an alkyl-substituted alkenyl group having 6 to 10 carbon atoms,
the water-soluble substituent may be bonded to these heterocyclyl and heteroaryl directly or through the substituent;
R¹¹ is a hydrogen atom, a substituted or unsubstituted alkyl group having 1 to 6 carbon atoms, a substituted or unsubstituted alkenyl group having 1 to 6 carbon atoms, an alkyl group having the water-soluble substituent and having 1 to 10 carbon atoms, preferably 1 to 6 carbon atoms (this alkyl group may be substituted with an aryl group, and the aryl group may be substituted with the water-soluble substituent), or an alkenyl group having the water-soluble substituent and having 1 to 10 carbon atoms, preferably 1 to 6 carbon atoms (this alkenyl group may be substituted with an aryl group, and the aryl group may be substituted with the water-soluble substituent);
X is selected from an oxygen atom, Si(R^{a})(R^{b}), C(R^{a})(R^{b}), P(=O)R^{c}, S(R^{d})₂, Ge(R^{a})(R^{b}), a tellurium atom, or a bismuth atom,
wherein, R^{a} and R^{b} are each independently a substituted or unsubstituted alkyl group having 1 to 6 carbon atoms, or a substituted or unsubstituted aryl group,
R^{c} is a substituted or unsubstituted alkyl group having 1 to 6 carbon atoms, or a substituted or unsubstituted phenyl group,
R^{d} is a substituted or unsubstituted aryl group, which is the same or different, the alkyl group, the aryl group, and the phenyl group of R^{a}, R^{b}, R^{c} and R^{d} may be substituted with the water-soluble substituent; and
R₀ and R¹⁰ are each independently selected from a partial structure of an amino acid, a partial structure of a carboxylic acid, T-O-, or a saturated or unsaturated 5- or 6-membered carbocyclic or heterocyclic group,
wherein, the partial structure of an amino acid, together with C=O to which R₀ or R¹⁰ is bonded, constitutes an amino acid, an amino acid residue, a peptide, or a part of an amino acid,
the partial structure of a carboxylic acid, together with C=O to which R₀ or R¹⁰ is bonded, constitutes a part of a carboxylic acid,
T is a saccharide, a partial structure of a saccharide, or a saccharide having a self-cleaving linker, and
the partial structure of a saccharide of T, together with O to which T is bonded, constitutes a saccharide or a part of a saccharide.

2. The fluorescent probe according to claim 1, wherein Q2 represents a ring structure of Formula (iii).

3. The fluorescent probe according to claim 1, wherein Q2 represents a ring structure of Formula (iv), (v), or (vi).

4. The fluorescent probe according to claim 1 or 2, wherein n is 1 to 3.

5. The fluorescent probe according to claim 1 or 2, wherein at least one of R² to R⁹ and R^{a}, R^{b}, R^{c} and R^{d} is the water-soluble substituent, an alkyl group having the water-soluble substituent and having 1 to 6 carbon atoms, an aryl group having the water-soluble substituent, and a phenyl group having the water-soluble substituent.

6. The fluorescent probe according to claim 1 or 2, wherein X is an oxygen atom or Si(R^{a})(R^{b}).

7. The fluorescent probe according to claim 4, wherein R is -SO₃H or -L-SO₃H.

8. The fluorescent probe according to claim 1 or 2, wherein the hydrolase is a peptide bond hydrolase selected from an aminopeptidase, a peptidase, a protease, or an amidase.

9. The fluorescent probe according to claim 1 or 2, wherein a MiLogP value of the compound or a salt thereof is 2.56 or less.

10. The fluorescent probe according to claim 1 or 2, wherein a MiLogP value of a fluorescent mother nucleus of the compound or a salt thereof is less than 2.47.

11. The fluorescent probe according to claim 1 or 2, wherein a MiLogP value of the compound or a salt thereof is 2.56 or less, and a MiLogP value of a fluorescent mother nucleus of the compound or a salt thereof is less than 2.47.

12. The fluorescent probe according to claim 1 or 9, which is a fluorescent probe for a microdevice.

13. The fluorescent probe according to claim 1 or 2, wherein the compound represented by General Formula (II) or a salt thereof is a compound represented by any one of General Formulas (Ia) to (Ie) below or a salt thereof: 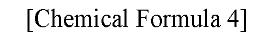 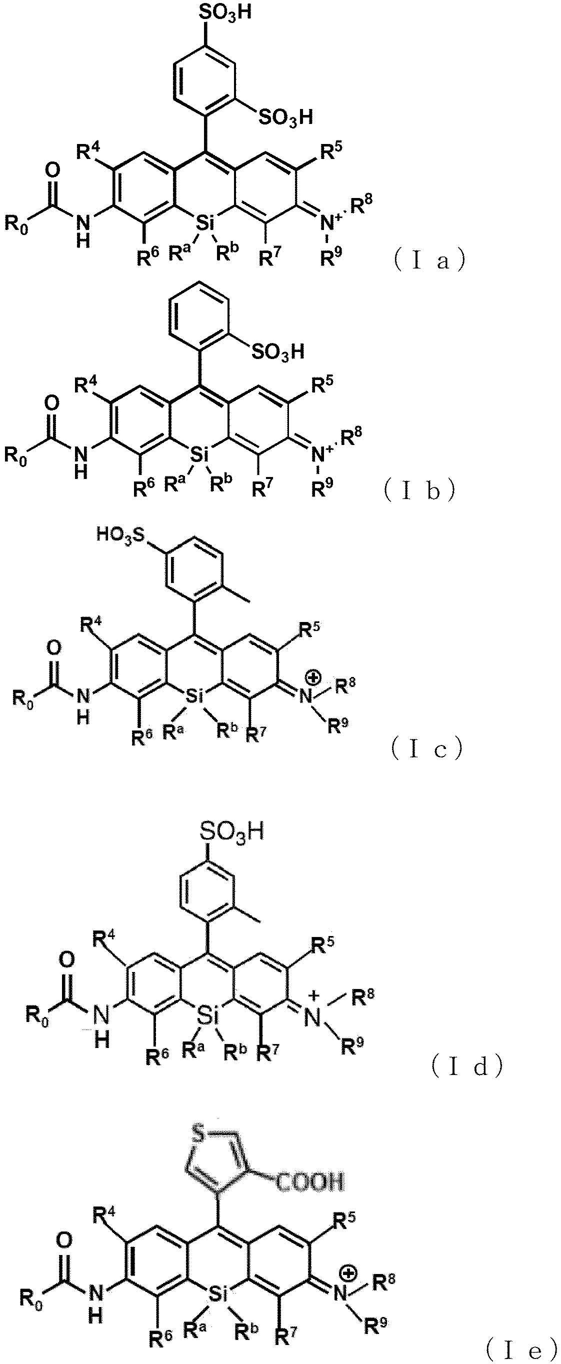 wherein R⁴ to R⁹, R^{a}, R^{b}, and R₀ are as defined in General Formula (II).

14. The fluorescent probe according to claim 1 or 2, wherein the compound represented by General Formula (II) or a salt thereof is a compound represented by General Formula (If) below or a salt thereof: 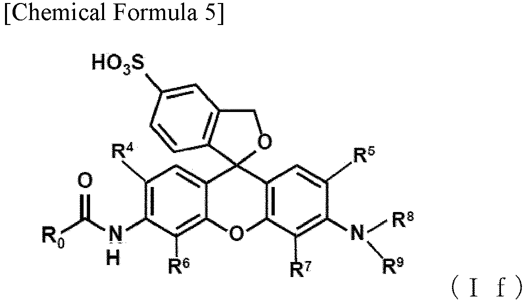 wherein R⁴ to R⁹ and R₀ are as defined in General Formula (II).

15. The fluorescent probe according to claim 1 or 2, wherein the compound or a salt thereof is a compound selected from the following compounds, or a salt thereof.

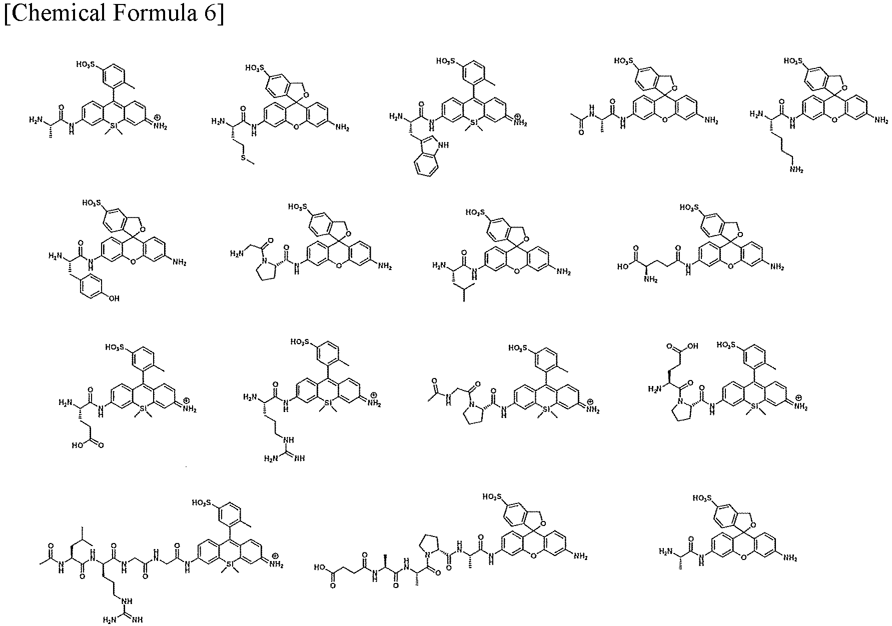

16. A method for diagnosing pancreatic cancer or a method for predicting a possibility that a test subject from which a biological sample is derived has pancreatic cancer, the method comprising detecting, by a microdevice, single-molecule enzyme activity of CD13 in a biological sample obtained from a subject, using the fluorescent probe according to claim 1 or 2, in which -CO-R₀ and optionally -CO-R¹⁰ is an alanine, lysine, arginine, or methionine residue in General Formula (II).

17. A method for diagnosing pancreatic cancer or a method for predicting a possibility that a test subject from which a biological sample is derived has pancreatic cancer, the method comprising detecting, by a microdevice, single-molecule enzyme activity of DPP4 in a biological sample obtained from a subject, using the fluorescent probe according to claim 1 or 2, in which -CO-R₀ and optionally -CO-R¹⁰ is -Pro-Xaa (Pro represents a proline residue and Xaa represents an amino acid residue such as glycine, serine, or glutamic acid) in General Formula (II).

18. A method for diagnosing pancreatic cancer or a method for predicting a possibility that a test subject from which a biological sample is derived has pancreatic cancer, the method comprising:
detecting, by a microdevice, single-molecule enzyme activity of CD13 in a biological sample obtained from a subject, using the fluorescent probe according to claim 1 or 2, in which -CO-R₀ and optionally -CO-R¹⁰ is an alanine, lysine, arginine, or methionine residue in General Formula (II); and
detecting, by a microdevice, single-molecule enzyme activity of DPP4 in the biological sample obtained from the subject, using the fluorescent probe according to claim 1 or 2, in which -CO-R₀ and optionally -CO-R¹⁰ is -Pro-Xaa (Pro represents a proline residue and Xaa represents an amino acid residue such as glycine, serine, or glutamine) in General Formula (II).

19. The method according to claim 16 or 17, wherein the biological sample is a biological sample of a patient with pancreatic cancer, a patient suspected of having pancreatic cancer, or a healthy subject.

20. A compound represented by any one of Formulas (1) to (5) below or a salt thereof: 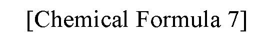 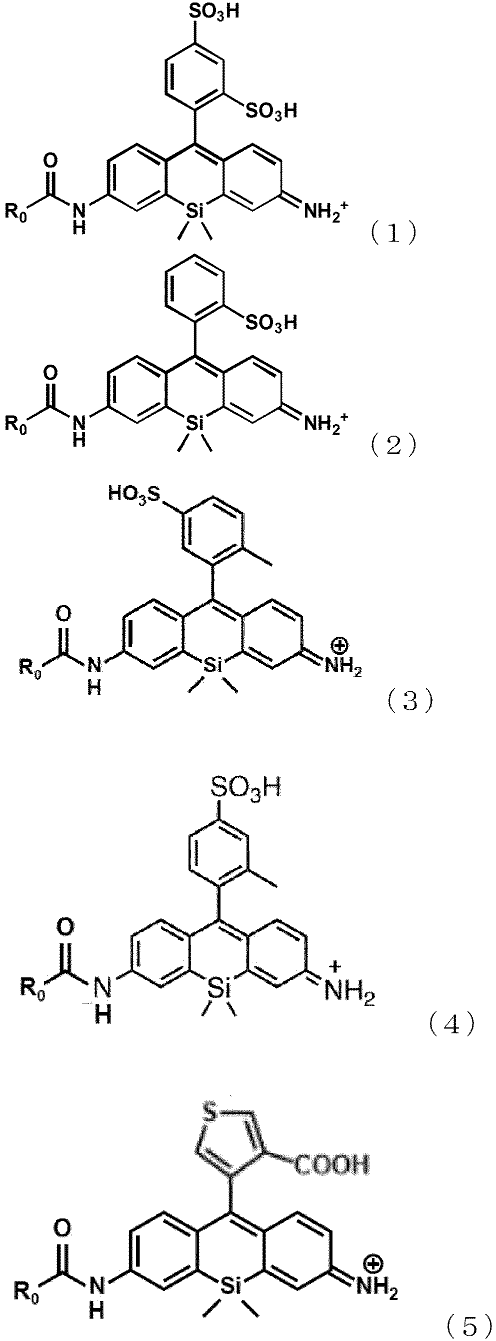 wherein
R₀ is selected from a partial structure of an amino acid, a partial structure of a carboxylic acid, or T-O-,
wherein the partial structure of an amino acid, together with C=O to which R₀ is bonded, constitutes an amino acid, an amino acid residue, a peptide, or a part of an amino acid,
the partial structure of a carboxylic acid, together with C=O to which R₀ is bonded, constitutes a part of a carboxylic acid,
T is a saccharide, a partial structure of a saccharide, or a saccharide having a self-cleaving linker, and
the partial structure of a saccharide of T, together with O to which T is bonded, constitutes a saccharide or a part of a saccharide.

21. A compound represented by Formula (6) below or a salt thereof: 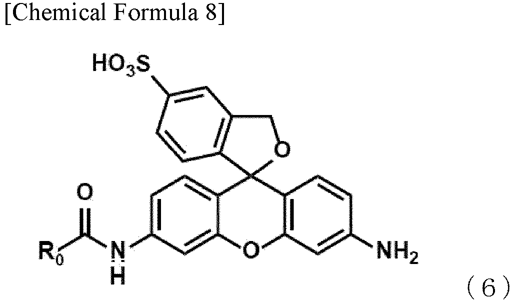 wherein
R₀ is selected from a partial structure of an amino acid, a partial structure of a carboxylic acid, or T-O-,
wherein the partial structure of an amino acid, together with C=O to which R₀ is bonded, constitutes an amino acid, an amino acid residue, a peptide, or a part of an amino acid,
the partial structure of a carboxylic acid, together with C=O to which R₀ is bonded, constitutes a part of a carboxylic acid,
T is a saccharide, a partial structure of a saccharide, or a saccharide having a self-cleaving linker, and
the partial structure of a saccharide of T, together with O to which T is bonded, constitutes a saccharide or a part of a saccharide.

22. Use of a compound represented by General Formula (III) below or a salt thereof, for the production of the compound according to claim 1 or 2 or a salt thereof: 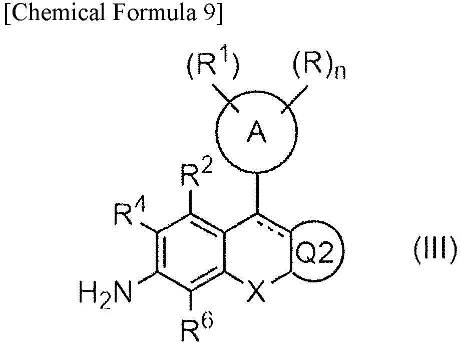 wherein ring A, ring Q2, X, n, R, R¹, R², R⁴, and R⁶ are as defined in General Formula (II), and a bond - - - represents a single bond or a double bond.

23. Use of a compound represented by General Formula (IV) below or a salt thereof, for the production of the compound according to claim 1 or 2 or a salt thereof: 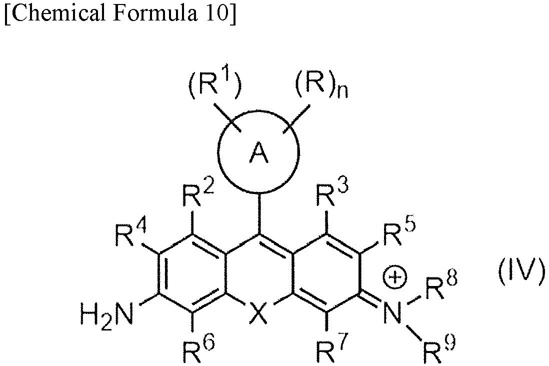 wherein ring A, X, n, R, and R¹ to R⁹ are as defined in General Formula (II).

24. A compound represented by General Formula (V) below or a salt thereof: [Chemical Formula 11] 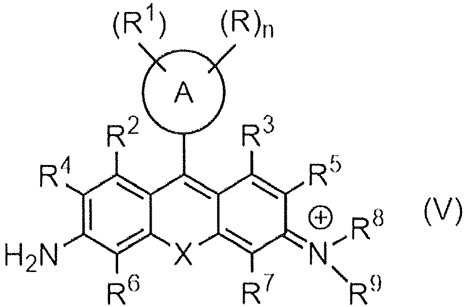 wherein
ring A, R, R⁸, and R⁹ are as defined in General Formula (II),
n is 1 or 2,
R¹ is a hydrogen atom, an unsubstituted alkyl group having 1 to 4 carbon atoms, or an alkyl group substituted with a hydroxyl group and having 1 to 4 carbon atoms,
R² to R⁷ are a hydrogen atom,
R⁸ and R⁹ are each independently a hydrogen atom or a substituted or unsubstituted alkyl group having 1 to 4 carbon atoms, and
X is an oxygen atom or Si(R^{a})(R^{b}) (R^{a} and R^{b} are as defined in Formula (II)).

25. The compound according to claim 24 or a salt thereof, which is a compound selected from the following compounds, or a salt thereof.

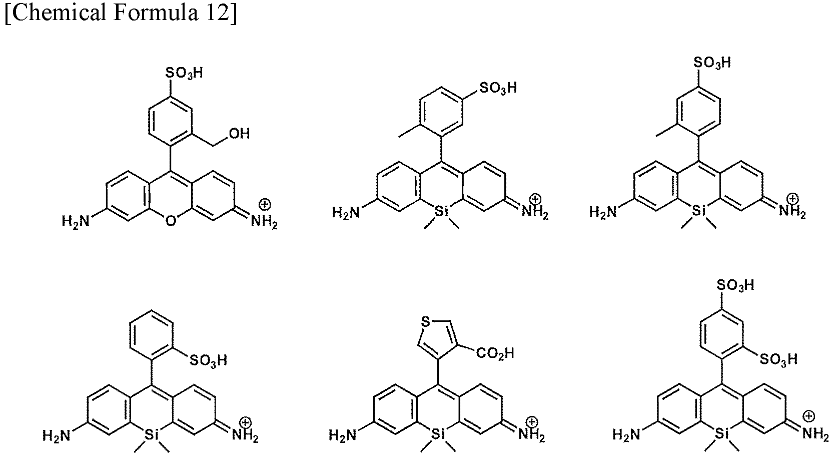

26. Use of the compound according to claim 24 or 25 or a salt thereof, for the production of the compound according to claim 1 or 2 or a salt thereof.

27. A method of selecting a fluorescent probe, the method comprising:
preparing the fluorescent probe according to claim 1 or 2; and
selecting a fluorescent probe satisfying that (A) a solubility of the fluorescent probe and an enzymolysate thereof in hexadecane in a solubility measurement test (25°C) in sealing oil is 0.3 µM or less, (B) a ratio of a fluorescence intensity of a well edge region to a fluorescence intensity of a well center region of the enzymolysate of the fluorescent probe in a fluorescence intensity measurement test in a microdevice is 0.6 or less, and (C) a ratio of a signal/noise ratio (first ratio) of the enzymolysate in a microdevice to a signal/noise (second ratio) of the enzymolysate in a cuvette in an enzymolysis test of the fluorescent probe in the microdevice and the cuvette is 0.4 or more.
